# EUROPEAN PATENT APPLICATION

(11) **EP 4 454 660 A1**
(43) Date of publication of application: **30.10.2024**
(21) Application number: 24172271.9
(22) Date of filing: 24.04.2024
(51) Int. Cl.: A61K 39/12, A61P 31/14

(54) **RIBONUCLEOPROTEIN NANOCOMPLEX VACCINES AND USES THEREOF**

(30) Priority: 24.04.2023 US 202363497895 P
(71) Applicant: Centre For Virology, Vaccinology and Therapeutics Limited, Pak Shek Kok, N.T. (HK)
(72) Inventor: KOK, Kin Hang, Hong Kong (HK); LAM, Joy Yan, Hong Kong (HK); YUEN, Kwok Yung, Hong Kong (HK)
(74) Representative: CMS Cameron McKenna Nabarro Olswang LLP

(57) **Abstract**

The present invention relates to vaccine compositions that specifically contain complexes comprising an adaptor-antigen polypeptide and viral RNA scaffold. The present invention further relates to uses of the vaccines for the preparation of pharmaceutical compositions, methods of treating or preventing viral infections, and kits comprising the vaccines.

## Description

### CROSS-REFERENCE TO RELATED APPLICATIONS

This application claims the priority benefit of U.S. Provisional Patent Application No. 63/497,895, filed April 24, 2023, the entire contents of which are incorporated herein by reference in their entirety.

### REFERENCE TO AN ELECTRONIC SEQUENCE LISTING

The contents of the electronic sequence listing (253322000341seqlist.xml; Size: 75,643 bytes; and Date of Creation: April 19, 2024) is herein incorporated by reference in its entirety.

### FIELD OF THE INVENTION

The present invention relates to vaccine compositions that comprise complexes comprising an adaptor-antigen polypeptide and a nucleic acid scaffold (e.g., viral RNA). Further provided herein are pharmaceutical compositions comprising the vaccines, methods of preventing or ameliorating viral infection using the vaccines, methods of making thereof, and kits comprising the vaccines.

### BACKGROUND OF THE INVENTION

Vaccines act to stimulate a protective immune response within an individual against specific pathogens, such as a human, such that, if later infected with that pathogen, the immune system can quickly prevent the infection from spreading within the body and causing disease. In this way, vaccines mimic natural infection without causing illness. Conventionally, vaccines are administered by intramuscular injection. However, this method of administration fails to prime the local, tissue resident-specific immune cells found within the tissues or organs affected by a viral infection (*e*.*g*., resident lung immune cells to prevent respiratory viral infection such as SARS-CoV-2, influenza, respiratory syncytial virus (RSV), *etc*.).

Administering vaccines, such as recombinant protein vaccines, via nasal administration (e.g., nasal inhalation) has not yet proven particularly effective and safe in humans, except when combined with adjuvants. However, traditional adjuvants, such as alum, generally are inadequate for mucosal vaccination within the human respiratory tract due to adverse side effects, including an irritative inflammatory immune response, that are caused by exogenous adjuvants. There is needed a safe and effective vaccine that can be administered to relevant tissues (e.g., intranasal, oral, mucosal administration) to prime tissue-resident immune cells against a viral target without causing side effects such as a systemic inflammatory innate immune response in the patient.

### BRIEF SUMMARY OF THE INVENTION

In one aspect there is provided a vaccine composition comprising nano-complexes comprising a i) scaffold molecule comprising a viral RNA and ii) an adaptor-antigen polypeptide comprising a viral antigen fused to a viral nucleoprotein capable of binding to the viral RNA.

In some embodiments, the molar ratio of the adaptor-antigen polypeptide to the scaffold molecule is more than about 5:1, such as more than any of 6:1, 7:1, 8:1, 9:1, or 10:1.

In some embodiments according to any of the vaccine compositions described above, the viral antigen is an antigen polypeptide. In some embodiments, the viral antigen is an immunogenic protein.

In some embodiments according to any of the vaccine compositions described above, the viral antigen is fused to the C-terminus of the viral nucleoprotein. In some embodiments, the viral antigen is fused to the N-terminus of the viral nucleoprotein.

In some embodiments according to any of the vaccine compositions described above, the viral RNA comprises an untranslated region of a viral genome, such as a 5' untranslated region or a 3' untranslated region of a viral gene of the viral genome.

In some embodiments according to any of the vaccine compositions described above, the viral RNA is about 10 to about 500 (e.g., about 10 to about 300) nucleotides long, such as about 200 to about 500 (e.g., about 200 to about 300) nucleotides long. In some embodiments, the viral RNA is at least about 200 nucleotides long.

In some embodiments according to any of the vaccine compositions described above, the viral RNA is derived from SARS-CoV-2. In some embodiments, the viral RNA comprises nucleotides 1-266 of a viral gene of the SARS-CoV-2 genome set forth in SEQ ID NO:25.

In some embodiments according to any of the vaccine compositions described above, the viral RNA is derived from an influenza A virus (IAV). In some embodiments, the viral RNA is derived from an IAV subtype selected from the group consisting of H1N1, H5N1, H3N2, and H7N9. In some embodiments, the viral RNA comprises nucleotides 1-326 of a gene of the IAV genome set forth in SEQ ID NO:30.

In some embodiments according to any of the vaccine compositions described above, the viral nucleoprotein is derived from SARS-CoV-2. In some embodiments, the viral nucleoprotein derived from SARS-CoV-2 comprises the amino acid sequence of SEQ ID NO: 12.

In some embodiments according to any of the vaccine compositions described above, the viral nucleoprotein is derived from an IAV. In some embodiments, the viral nucleoprotein is derived from an IAV subtype selected from the group consisting of H1N1, H5N1, H3N2, and H7N9. In some embodiments, the viral nucleoprotein derived from IAV H5N1 comprises the amino acid sequence of SEQ ID NO: 13.

In some embodiments according to any of the vaccine compositions described above, the viral nucleoprotein and the viral antigen are derived from the same virus. In some embodiments, the viral nucleoprotein and the viral antigen are derived from different viruses.

In some embodiments according to any of the vaccine compositions described above, the viral antigen is a SARS-Cov-2 protein or a fragment or variant thereof, such as a SARS-CoV-2 spike protein or a fragment or variant thereof.

In some embodiments according to any of the vaccine compositions described above, the viral antigen comprises a receptor binding domain (RBD). In some embodiments, the SARS-CoV-2 viral antigen comprises the amino acid sequence of SEQ ID NO: 14 or 15.

In some embodiments according to any of the vaccine compositions described above, the viral antigen is an influenza virus protein or a fragment or variant thereof, such as an influenza HA protein or a fragment or variant thereof. In some embodiments, the influenza virus protein or fragment or variant thereof is derived from influenza A virus (IAV) or influenza B virus (IBV). In some embodiments, the influenza virus protein or fragment or variant thereof is derived from an IAV subtype selected from the group consisting of H1N1, H5N1, H3N2, and H7N9. In some embodiments, the IAV viral antigen comprises an amino acid sequence selected from the group consisting of SEQ ID NOs: 16-18 and 31. In some embodiments, the influenza virus protein or a fragment or variant thereof is derived from an IBV subtype of Yamagata or Victoria. In some embodiments, the IBV viral antigen comprises the amino acid sequence of SEQ ID NO: 19 or 20.

In some embodiments according to any of the vaccine compositions described above, the viral antigen is a respiratory syncytial virus ("RSV") protein, such as an RSV F protein or a fragment or variant thereof. In some embodiments, the RSV F protein or fragment or variant thereof is derived from an RSV subtype selected from the group consisting of RSV A subtype and RSV B subtype. In some embodiments, the RSV viral antigen comprises the amino acid sequence of SEQ ID NO:21 or 22.

In some embodiments according to any of the vaccine compositions described above, the viral antigen is a hepatitis C virus ("HCV") protein or a fragment or variant thereof, such as an HCV E2 protein or a fragment or variant thereof. In some embodiments, the HCV E2 protein or fragment or variant thereof is derived from an HCV genotype of HCV genotype 1a or HCV genotype 2a. In some embodiments, the HCV viral antigen comprises the amino acid sequence of SEQ ID NO:23 or 24.

In some embodiments according to any of the vaccine compositions described above, the adaptor-antigen polypeptide comprises an amino acid sequence selected from the group consisting of SEQ ID NOs: 1-11 and 26-29.

In some embodiments according to any of the vaccine compositions described above, the nano-complexes in the vaccine composition have an average particle size of about 100nm to about 130nm.

In some embodiments according to any of the vaccine compositions described above, the nano-complexes in the vaccine composition have a zeta-potential of about -10mV to about - 20mV.

In some embodiments according to any of the vaccine compositions described above, the vaccine composition provides heterosubtypic protection against two or more virus subtypes that are from the same virus species (e.g., IAV, for example two or more of H1N1, H3N2, H5N1, and H7N9).

In another aspect, there is provided a method of preventing viral infection in an individual *(e.g.,* a human), comprising administering to the individual an effective amount of any of the vaccine composition described above. In some embodiments, the vaccine composition is administered by intranasal or intradermal administration. In some embodiments, the vaccine composition is administered with an initial dose, followed by one or two booster doses. In some embodiments, the initial dose and the one or two booster doses are administered from about 7 days to about 21 days apart. In some embodiments, the initial dose and the one or two booster doses are administered about 14 days apart. In some embodiments, the initial dose and a first booster dose, and the first booster dose and a second booster dose are administered about 7 to about 21 days apart, optionally about 14 days apart.

In some embodiments according to any of the methods described above, the method prevents viral infection by two or more viruses from different virus genera. In some embodiments, the method prevents viral infection by SARS-CoV-2 and IAV. In some embodiments, the vaccine composition provides heterosubtypic protection against two or more virus species that are part of the same virus family, optionally wherein the two or more virus species are part of the same virus genus. In some embodiments, i) the two or more virus species are from the same virus family; or ii) the two or more virus species are from different virus families. In some embodiments, the method prevents viral infection by three or more virus species, wherein: i) the three or more virus species are from the same virus family; ii) the three or more virus species are from different virus families; or iii) the three or more virus species comprise: (a) two or more virus subtypes or subspecies that are from the same virus species, and (b) one or more virus species that is from a different virus family than the viruses of (a).

### BRIEF DESCRIPTION OF THE DRAWINGS

The drawings illustrate certain features and advantages of this disclosure. These embodiments are not intended to limit the scope of the appended claims in any manner.

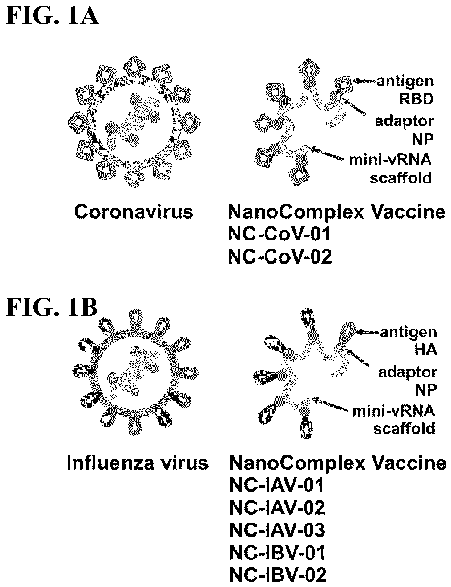

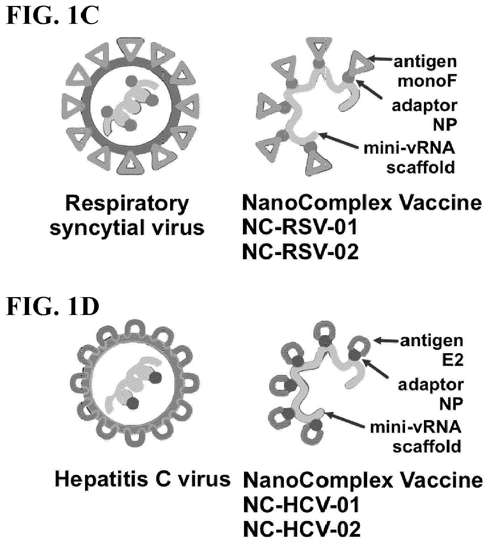

**FIGs. 1A-1D** depict the NanoComplex vaccines against various viral antigens. **FIG. 1A** depicts the vaccines targeting the coronavirus (*i.e*., SARS-CoV-2), including NC-CoV-01 and NC-CoV-02. **FIG. 1B** depicts the vaccines targeting influenza virus, including NC-IAV-01, NC-IAV-02, NC-IAV-03, NC-IBV-01, and NC-IBV-02. **FIG. 1C** depicts the vaccines targeting RSV, including NC-RSV-01 and NC-RSV-02. **FIG. 1D** depicts the vaccines targeting HCV, including NC-HCV-01 and NC-HCV-02. RBD, receptor binding domain; HA, hemagglutinin; monoF, RSV F monomer; E2, envelope glycoprotein E2; NP, nucleoprotein; mini-vRNA, mini-viral RNA.

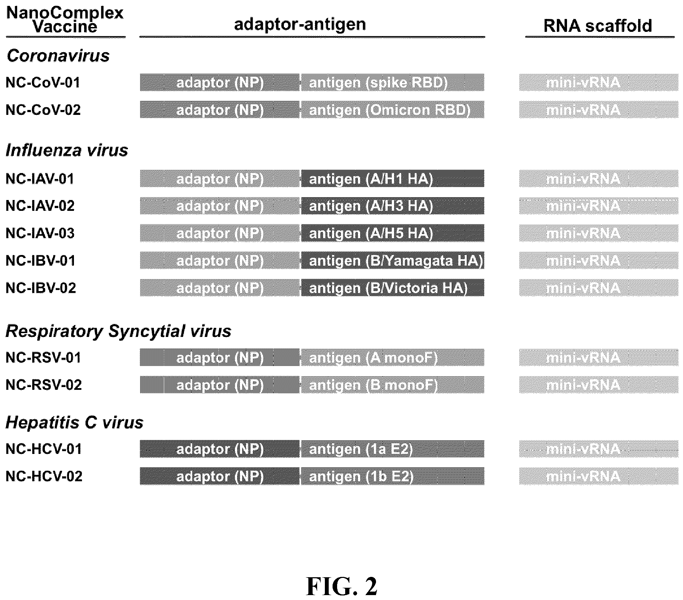

**FIG. 2** depicts a schematic overview of the adaptor-antigen polypeptide and RNA scaffold components in the exemplary NanoComplex vaccines.

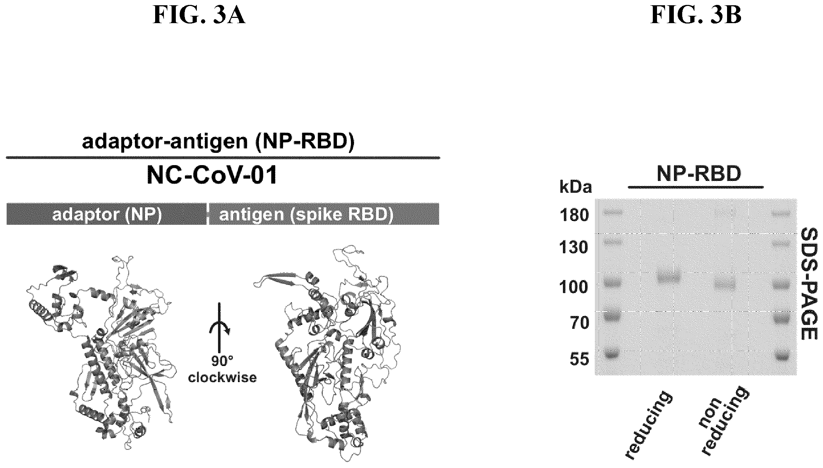

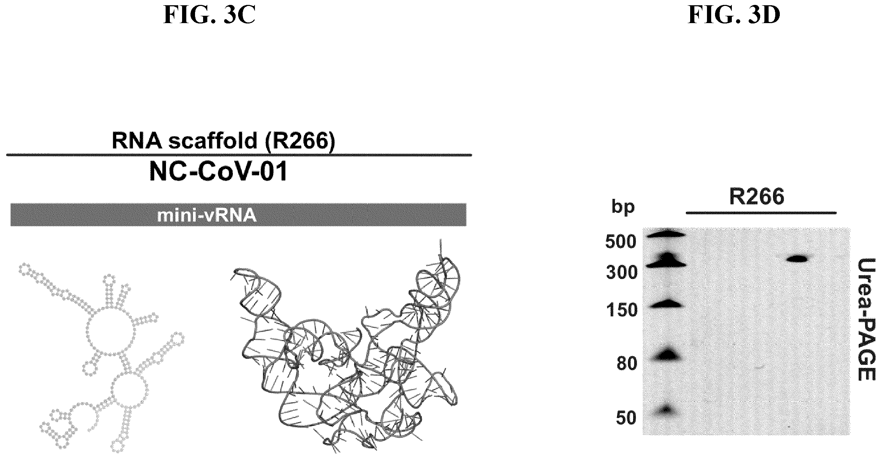

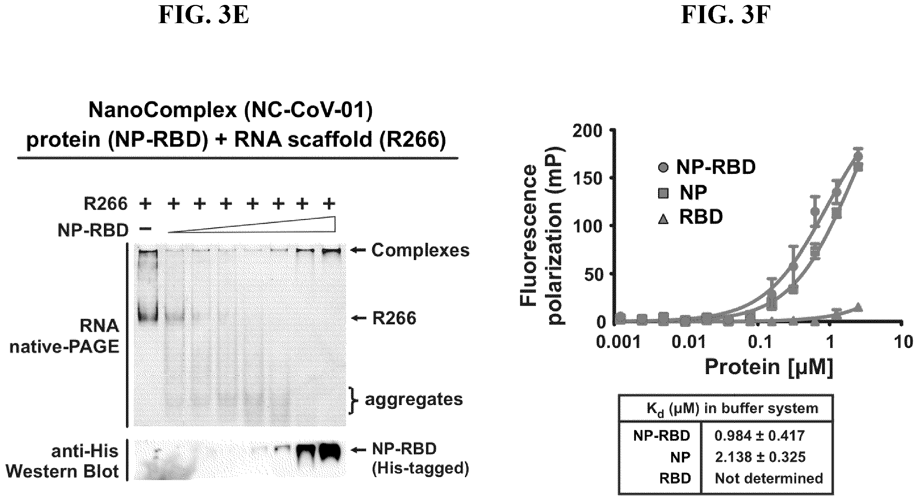

**FIGs. 3A-3F** depict the results from testing the NanoComplex formation of NC-CoV-01 vaccine. **FIG. 3A** depicts the structure prediction of nucleocapsid (NP)-RBD, the adaptor-antigen component. **FIG. 3B** provides the SDS-PAGE results for the tested NP-RBD protein. **FIG. 3C** left panel shows the RNA secondary structure prediction of R266 (exemplary RNA scaffold), and the right panel shows the 3D structure prediction of R266. **FIG. 3D** shows that R266 was visualized using Urea-PAGE. **FIG. 3E** shows that the association of adaptor-antigen and R266 was confirmed by gel-shift assay. R266 was mixed with an increasing amount of NP-RBD and analyzed by Urea-PAGE (upper panel) and Western blot (bottom panel). **FIG. 3F** shows that the association of adaptor-antigen and R266 was further confirmed by Fluorescence polarization. Briefly, HEX-labelled RNA oligo was mixed with increasing concentration of NP-RBD, full-length NP, and spike RBD protein, and the fluorescence polarization was measured. Kd in the specific buffer system was calculated.

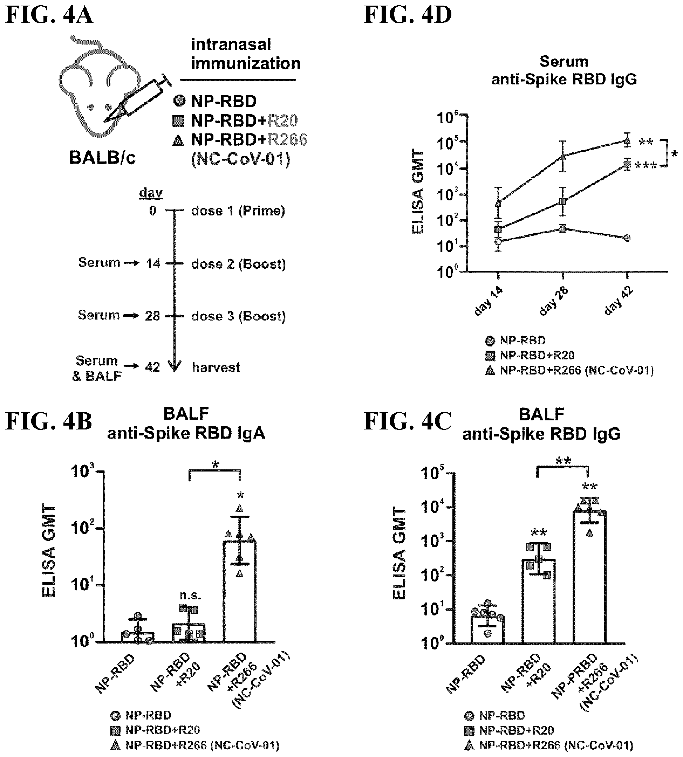

**FIGs. 4A-4C** demonstrate that NC-CoV-01 elicited mucosal immunity *in vivo.* **FIG. 4A** provides a schematic diagram illustrating the immunization of NC-CoV-01 in the animal. BALB/c mice were intranasally immunized with 3 doses of NP-RBD, NP-RBD+R20 (20 nt viral RNA) control, or NP-RBD+R266 (NC-CoV-01). Sera and bronchoalveolar fluid (BALF) were harvested and tested with ELISA for antigen-specific antibodies. **FIG. 4B** shows the anti-Spike RBD IgA response in BALF. **FIG. 4C** shows that the anti-Spike RBD IgG response in BALF. **FIG. 4D** shows the anti-Spike RBD IgG response in serum across days 14, 28, and 42 of the experiment. All ELISA results were presented as geometric mean titers with a 95% confidence interval. *: p<0.05, **: p<0.005, ***: p<0.0005, n.s.: not significant.

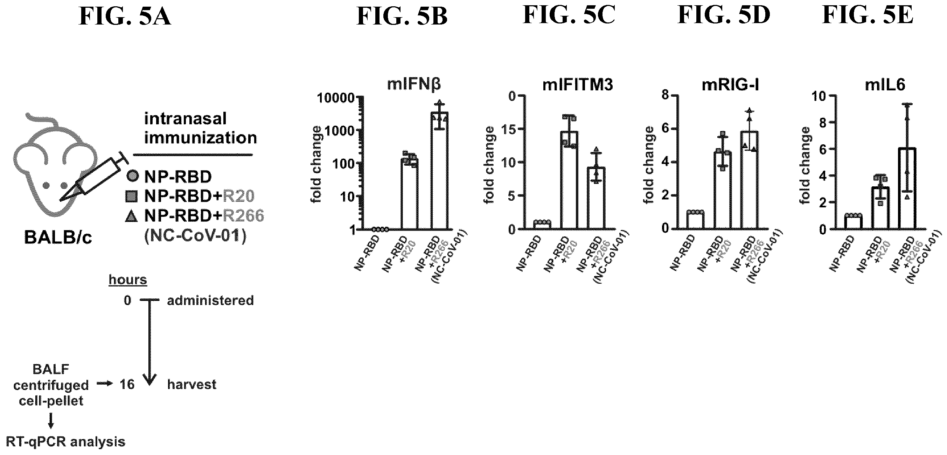

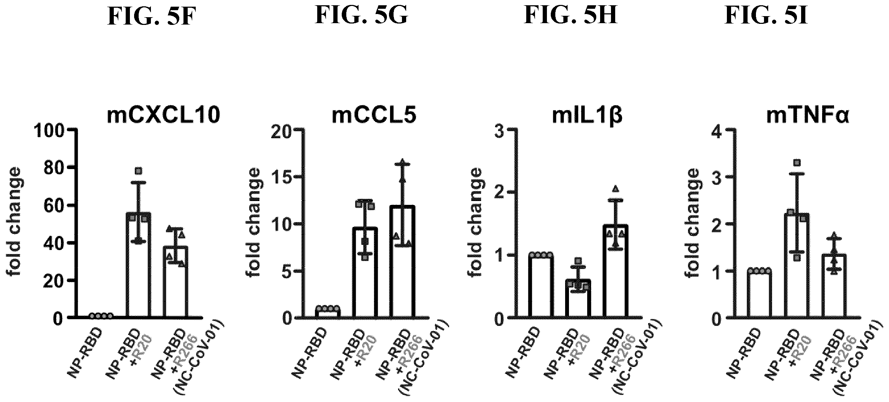

**FIGs. 5A-5I** depict the gene expression results of various pro-inflammatory cytokines and chemokines *in vivo* after intranasal immunization against SARS-CoV-2 with the NC-CoV-01 vaccine. **FIG. 5A** shows a schematic overview of the murine intranasal immunization model: NP-RBD was mixed with either R20 or R266 and administered nasally into BALB/c mice. BALF was collected at 16 hours post-administration. RNA was extracted from the cell pellet of these BALF and was analyzed by RT-qPCR. **FIGs. 5B-5I** show the induction of interferon-β (IFN-β; **FIG. 5B**), interferon-stimulated genes (IFITM3, **FIG. 5C**; and RIG-I, **FIG. 5D**), chemokines (CXCL10, **FIG. 5F**; and CCL5, **FIG. 5G**) and pro-inflammatory cytokines (IL-6, **FIG. 5E**; IL-1β, **FIG. 5H**; and TNFα, **FIG. 5I**), all of which were presented as fold ± S.D.

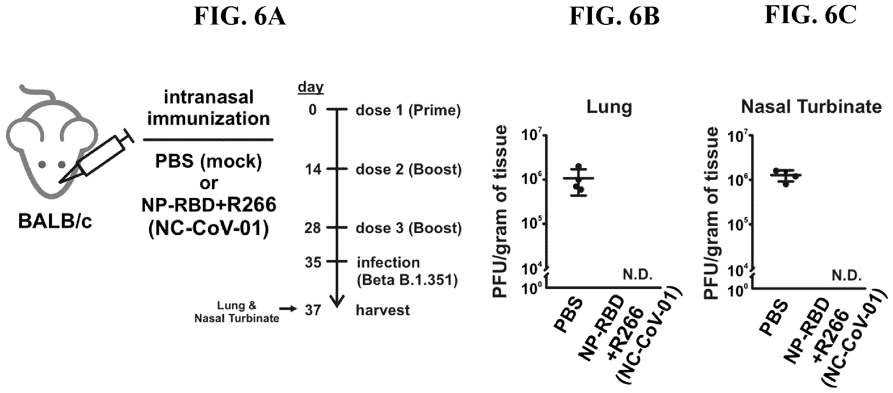

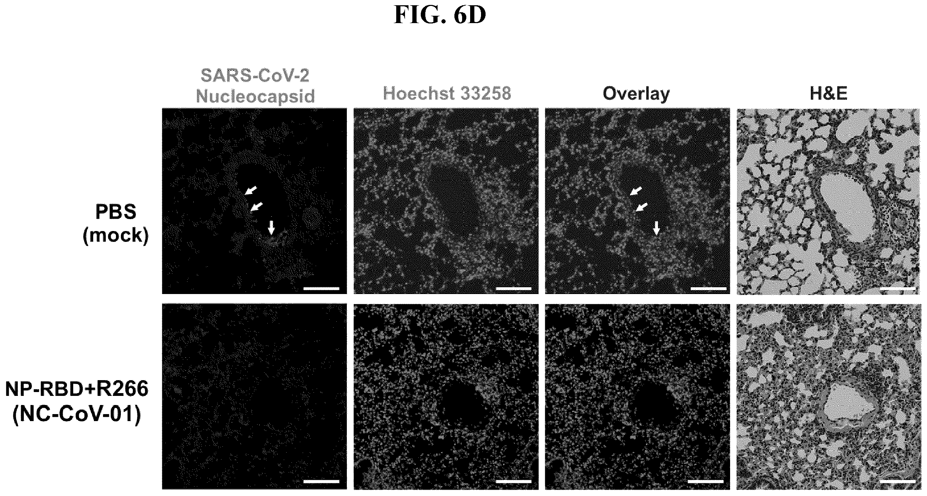

**FIGs. 6A-6D** show that the NC-CoV-01 vaccine protects mice against developing SARS-CoV-2 infection. **FIG. 6A** provides a schematic overview of the murine intranasal immunization model. **FIGs. 6B- 6C** show the virus infectious titer levels in lung (**FIG. 6B**) and nasal turbinate (**FIG. 6C**) homogenates, as analyzed by plaque assays. Data were presented as PFU per gram of tissues. **FIG. 6D** shows the immunohistology and H&E staining of mouse lung, wherein SARS-CoV-2 nucleocapsid-positive stained cells are indicated by the arrows. N.D., not detected; H&E, hematoxylin and eosin.

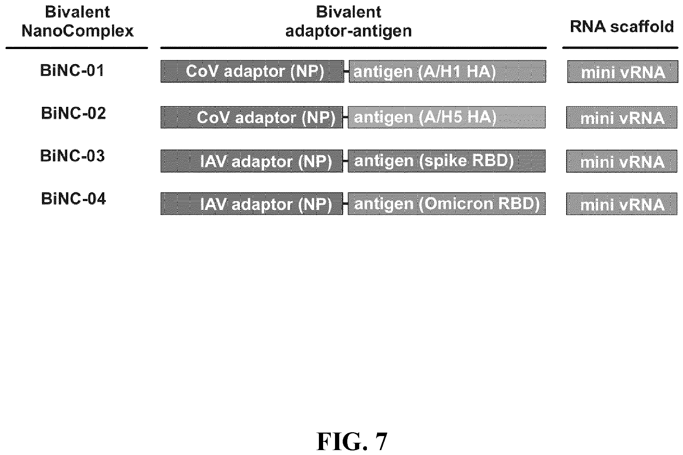

**FIG. 7** depicts the SARS-CoV-2/FluA bivalent NanoComplex vaccines against SARS-CoV-2 and influenza A viral antigens. In the exemplary bivalent NanoComplex vaccine formats provided herein, a SARS-CoV-2 adaptor is fused with an influenza A virus (IAV) antigen, and then complexed with the SARS-CoV-2 mini vRNA scaffold, R266, to generate the bivalent NanoComplex (BiNC) vaccine (BiNC-01 and BiNC-02). Alternatively, an IAV adaptor is fused with a SARS-CoV-2 antigen and then complexed with a mini vRNA scaffold (e.g., the IAV mini-vRNA, R326; or the SARS-CoV-2 mini-vRNA, R266) to generate the BiNC vaccine (BiNC-03, BiNC-04).

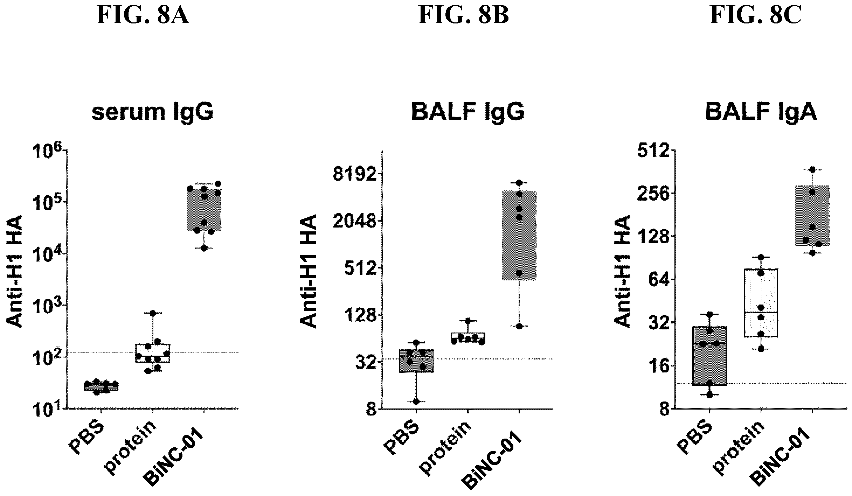

**FIGs. 8A-8C** show the murine antibody responses that were specific to H1N1 that arose after intranasal vaccination with the exemplary BiNC-01 vaccine. **FIG. 8A** shows the serum IgG responses. **FIG. 8B** shows the BALF IgG responses. **FIG. 8C** shows the BALF IgA responses.

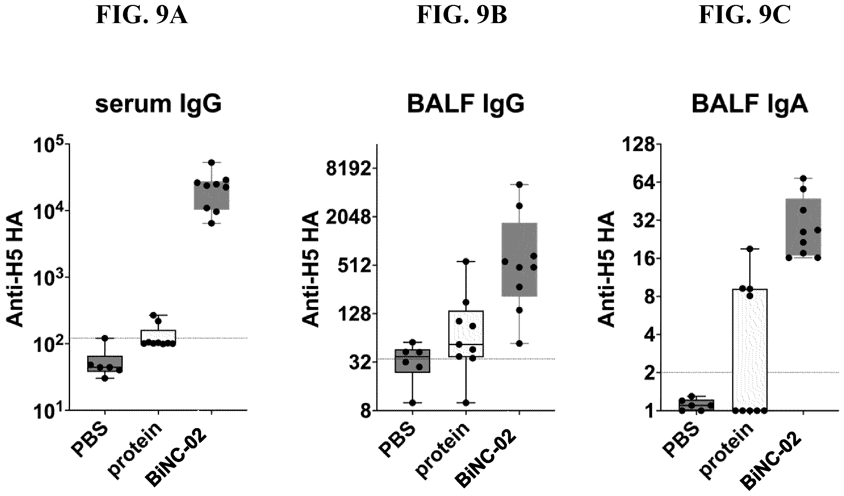

**FIGs. 9A-9C** show the murine antibody responses that were specific to H5N1 that arose after intranasal vaccination with the exemplary BiNC-02 vaccine. **FIG. 9A** shows the serum IgG responses. **FIG. 9B** shows the BALF IgG responses. **FIG. 9C** shows the BALF IgA responses.

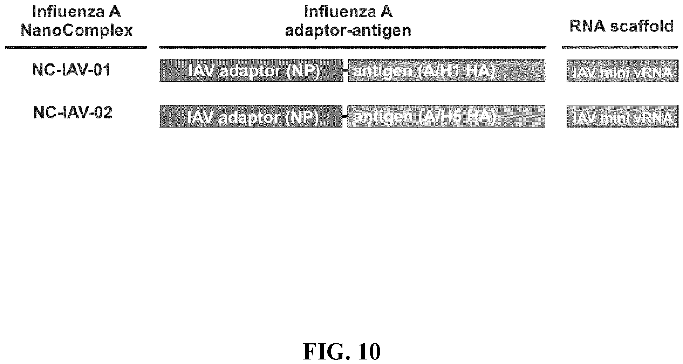

**FIG. 10** depicts two exemplary influenza A-specific NanoComplex vaccines that were constructed using adaptor-antigen method: (1) H1N1-specific (NC-IAV-01), and (2) H5N1-specific (NC-IAV-02) vaccines.

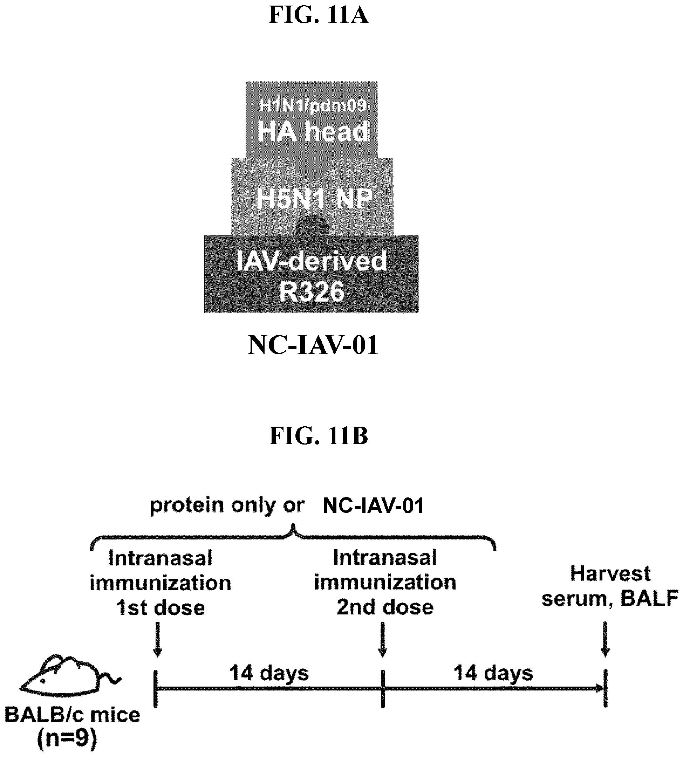

**FIGs. 11A-11B** provide schematic overviews of the viral complex (**FIG. 11A**) and *in vivo* vaccination experimental design (**FIG. 11B**). **FIG. 11A** provides a ribbon diagram illustrating the protein structure of the NC-IAV-01 NanoComplex vaccine. **FIG. 11B** provides a schematic diagram illustrating the immunization of the animal with 2 doses of NC-IAV-01 administered intranasally. R326, exemplary mini-vRNA scaffold derived from IAV genome.

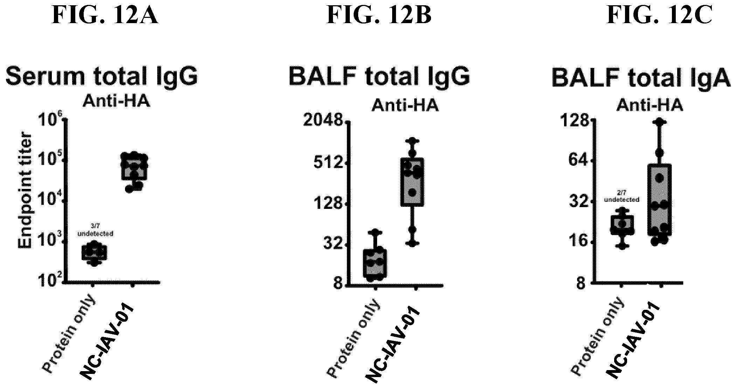

**FIGs. 12A-12C** show the murine antibody responses that were specific to H1N1 that arose after intranasal vaccination with the exemplary NC-IAV-01 NanoComplex vaccine. **FIG. 12A** shows the serum IgG responses. **FIG. 12B** shows the BALF IgG responses. **FIG. 12C** shows the BALF IgA responses. R326, exemplary mini-vRNA scaffold derived from IAV genome.

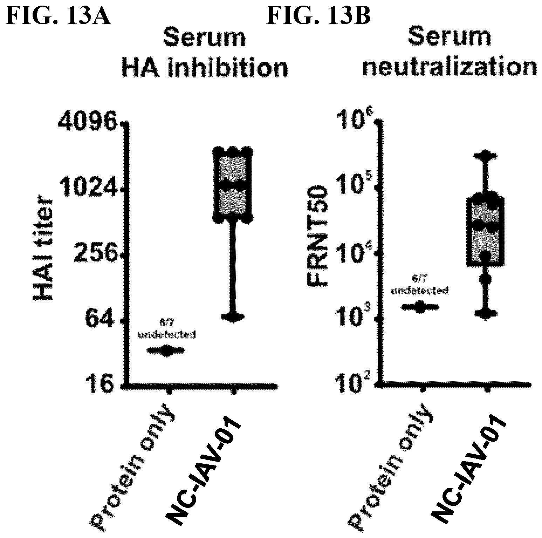

**FIGs. 13A-13B** show the neutralization of H1N1 by the murine immune system after intranasal vaccination of BALB/c mice with the exemplary NC-IAV-01 NanoComplex vaccine. FIG. 13A shows the serum hemagglutination inhibition response *in vivo.* **FIG. 13B** shows the serum FRNT50 neutralization response *in vivo.* R326, exemplary mini-vRNA scaffold derived from IAV genome.

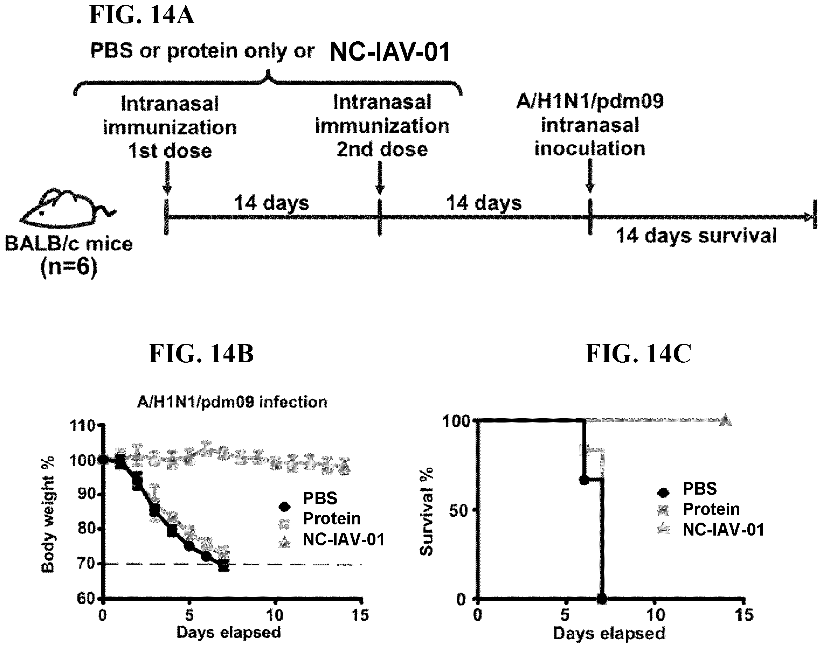

**FIGs. 14A-14C** depict the experimental design and accompanying results of the live H1N1 virus challenge in mice that were intranasally vaccinated with 2 doses of the exemplary NC-IAV-01 NanoComplex vaccine. **FIG. 14A** provides a schematic overview of the experimental design, wherein BALB/c mice were intranasally vaccinated with 2 doses of NC-IAV-01 and then challenged by live H1N1 virus. **FIG. 14B** shows the results from monitoring murine body weight over 14 days post-infection. **FIG. 14C** shows the survival curve of infected mice up to 14 days post-infection. R326, exemplary mini-vRNA scaffold derived from IAV genome.

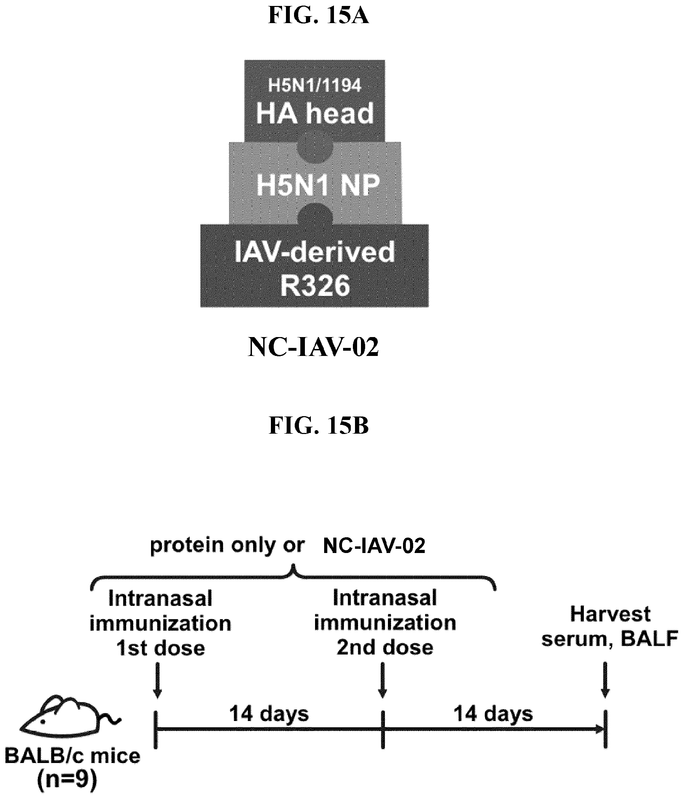

**FIGs. 15A-15B** provide schematic overviews of the NC-IAV-02 viral complex (**FIG.** 15A) and *in vivo* vaccination experimental design (**FIG. 15B**). **FIG. 11A** provides a ribbon diagram illustrating the protein structure of the NC-IAV-02 NanoComplex vaccine. **FIG. 11B** provides a schematic diagram illustrating the immunization of the animal with 2 doses of NC-IAV-02 administered intranasally. R326, exemplary mini-vRNA scaffold derived from IAV genome.

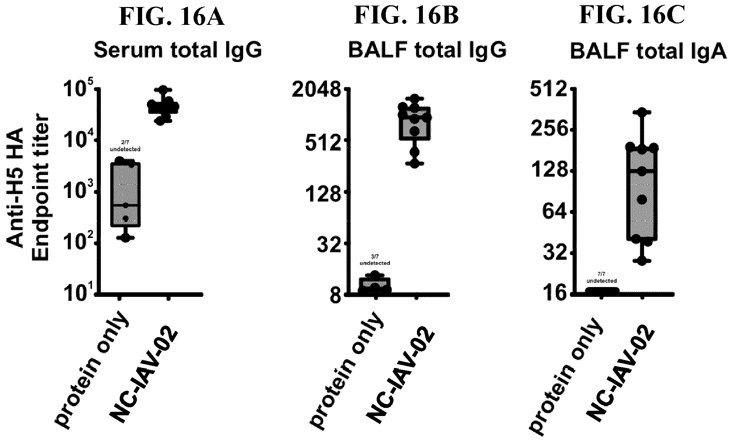

**FIGs. 16A-16C** show the murine antibody responses that were specific to H5N1 that arose after intranasal vaccination with the exemplary NC-IAV-02 NanoComplex vaccine. **FIG. 16A** shows the serum IgG responses. **FIG. 16B** shows the BALF IgG responses. **FIG. 16C** shows the BALF IgA responses. R326, exemplary mini-vRNA scaffold derived from IAV genome.

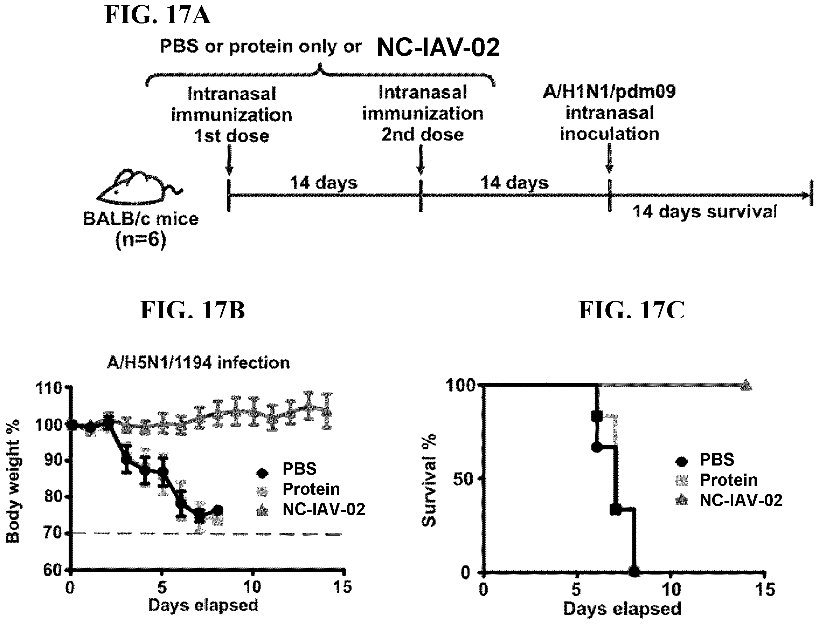

**FIGs. 17A-17C** depict the experimental design and accompanying results of the live H5N1 virus challenge in mice that were intranasally vaccinated with 2 doses of the exemplary NC-IAV-02 NanoComplex vaccine. **FIG. 17A** provides a schematic overview of the experimental design, wherein BALB/c mice were intranasally vaccinated with 2 doses of NC-IAV-02 and then challenged by live H5N1 virus. **FIG. 17B** shows the results from monitoring murine body weight over 14 days post-infection. **FIG. 17C** shows the survival curve of infected mice up to 14 days post-infection. R326, exemplary mini-vRNA scaffold derived from IAV genome.

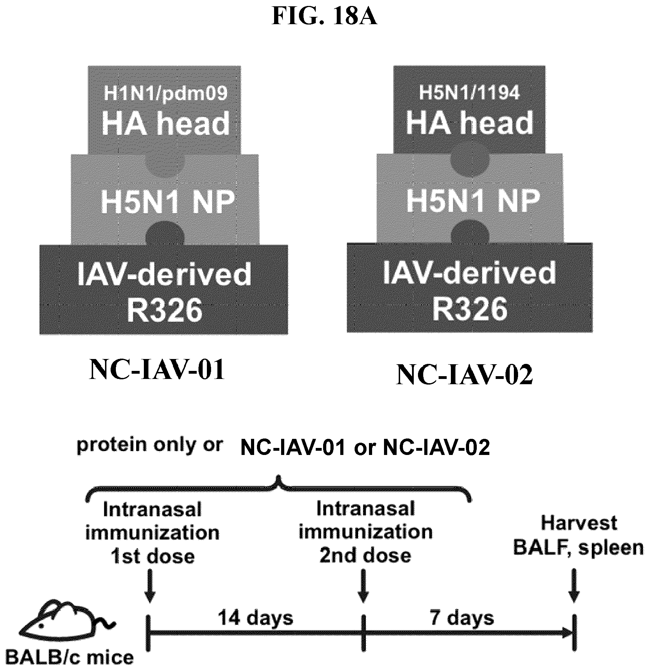

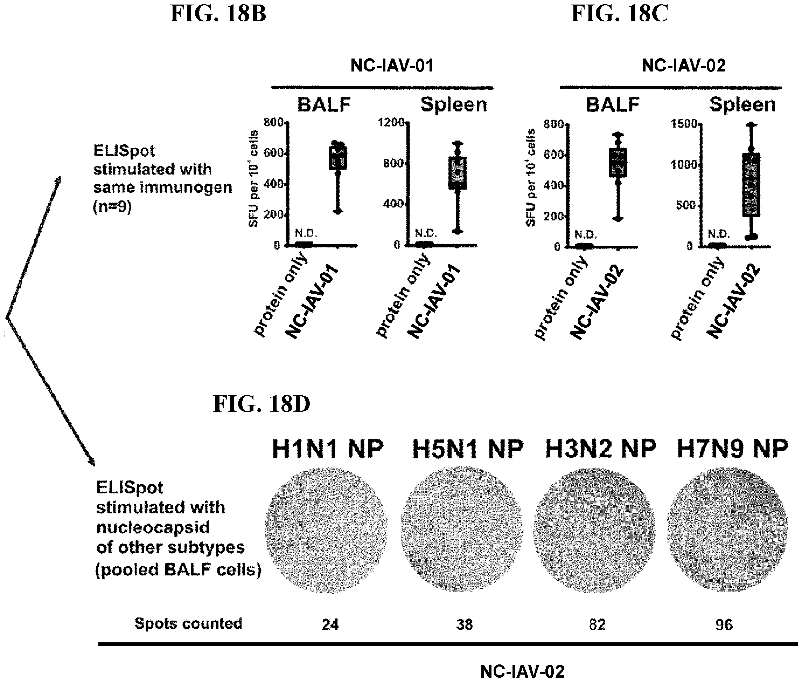

FIGs. 18A-18D show the cross-reactivity of IAV adaptor protein to different IAV subtypes. **FIG. 18A** provides a schematic overview of the experimental design, wherein mice were intranasally immunized with either the exemplary NC-IAV-01 or the exemplary NC-IAV-02 NanoComplex vaccines. BALF cells and spleen cells were harvested 7 days after administration of the second dose and assayed by ELISpot, using the original immunogen or NP from different subtypes. **FIGs. 18B-18C** shows the results of the ELISpot assay using cells that were stimulated by the same immunogen as the vaccine (**FIG. 18B**: NC-IAV-01; and **FIG. 18C**: NC-IAV-02). FIG. 18D shows the results of the ELISpot assay using cells that were stimulated by viral nucleocapsids of different influenza A subtypes, i.e., H1N1, H5N1, H3N2, or H7N9, and wherein the vaccine was NC-IAV-02. NP, nucleocapsid; R326, exemplary mini-vRNA scaffold derived from IAV genome.

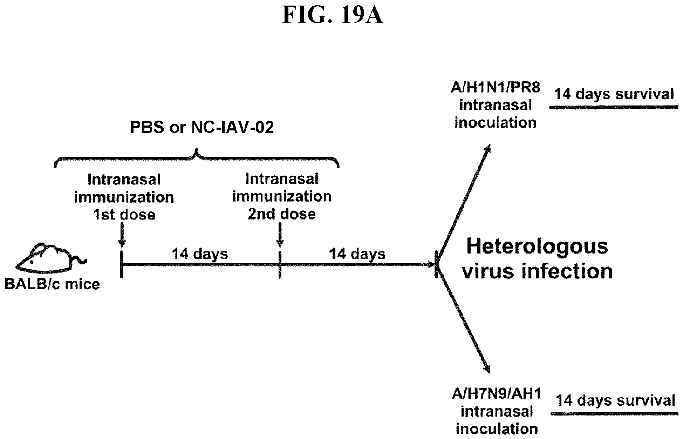

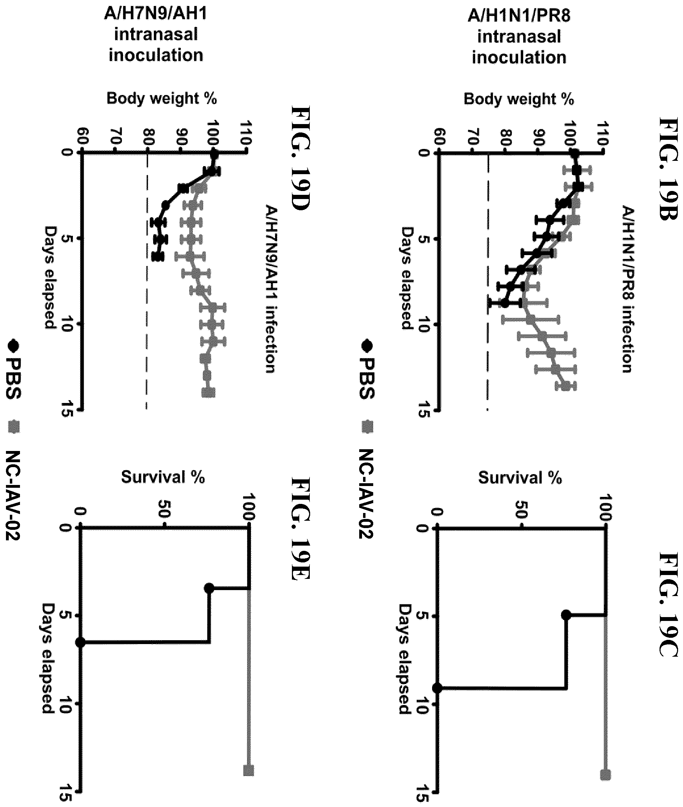

**FIGs. 19A-19E** show the cross-protection against different IAV subtypes resulting from vaccination with the exemplary NC-IAV-02 NanoComplex vaccine. **FIG. 19A** provides a schematic overview of the experimental design, wherein mice were intranasally immunized with 2 doses of NC-IAV-02, and then challenged by two distinct live IAV subtypes (H1N1/PR8 and H7N9/AH1). **FIGs. 19B-19C** show the change in body weight over 14 days (**FIG. 19B**) and the survival curve up to 14 days (**FIG. 19C**) for mice that were challenged with H1N1/PR8 live virus. **FIGs. 19D-19E** show the change in body weight over 14 days (**FIG. 19D**) and the survival curve up to 14 days (**FIG. 19E**) for mice that were challenged with H7N9/AH1 live virus. R326, exemplary mini-vRNA scaffold derived from IAV genome.

### DETAILED DESCRIPTION OF THE INVENTION

The present application provides novel vaccine compositions and methods for vaccinating or treating an individual (*e*.*g*., human) against a target disease, disorder, or infection, such as a viral infection. Specifically, the inventors have designed and tested a novel vaccine nano-complex platform that comprises two parts: (i) a nucleoprotein that is fused with a target antigen (*e.g.*, a viral antigen), which is also known as the adaptor-antigen polypeptide, and (ii) a nucleic acid scaffold, for example a viral RNA comprising a mini-viral RNA (mini-vRNA). The nucleic acid scaffold (e.g., viral RNA) scaffold allows for the binding of multiple adaptor-antigen polypeptides, as the nucleoprotein has a high affinity for nucleic acid scaffold (e.g., viral RNA). This vaccine nano-complex platform demonstrates high vaccination efficacy that is significantly improved by both the formation of the adaptor-antigen/RNA nano-complexes, which increases the size of the immunogenic complex relative to traditional vaccines, and the presence of the nucleic acid scaffold (e.g., viral RNA) as an adjuvant to activate host innate immunity. Without being bound by theory, it is believed that use of this nano-complex vaccine platform has a number of advantages: 1) The vaccination efficacy via mucosal administration is safe and highly effective; 2) The nano-complex vaccine produces no or low (*e*.*g*., lower than traditional vaccines, mild or undistinguishable symptoms, or nearly no) side effects compared to traditional vaccines and therefore presents with an improved safety profile; 3) Nasal spray of protein vaccine is comparably less invasive than intramuscular injection and could potentially improve induction of mucosal immunity to combat infections of the mucosa; 4) Nasal spray of recombinant protein vaccine does not involve handling of infectious materials and thus does not involve the safety concerns associated with infectious materials; 5) A mix-and-match approach to the construction of the adaptor-antigen polypeptide can provide for effective bivalent vaccination from a single vaccine molecule; and 6) The nano-complex vaccine is capable of providing heterosubtypic protection against multiple virus subtypes (for example, but not limited to a nano-complex vaccine targeting influenza A virus subtype H5N1 also protecting against IAV subtypes H1N1, N3N2, and/or H7N9).

Accordingly, the present application in one aspect provides a vaccine composition comprising nano-complexes comprising i) a scaffold molecule comprising a nucleic acid (*e.g.*, a viral RNA) and ii) an adaptor-antigen polypeptide comprising a target antigen (*e*.*g*., a viral antigen) fused to a viral nucleoprotein capable of binding to the nucleic acid (*e.g.*, viral RNA).

In another aspect, there is provided a method of preventing or treating a disease, disorder, or infection (*e.g.*, a viral infection) in an individual (*e.g.*, human), comprising administering to the individual an effective amount of the vaccine composition. In some embodiments, the vaccine composition is administered by intranasal or intradermal administration.

All publications, comprising patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### I. DEFINITIONS

It will be understood by one of ordinary skill in the art that uracil and thymine can both be represented by 't', instead of 'u' for uracil and '1' for thymine; in the context of a ribonucleic acid, it will be understood that 't' is used to represent uracil unless otherwise indicated.

The term "genomic RNA" as used herein refers to the heritable genetic information of an RNA virus. However, in the context of the present invention the term "genome" typically also refers to the genome of an RNA virus and hence an RNA genome having a ribonucleic acid sequence. The person skilled in the art will understand that the genome of an RNA virus may also be provided as a DNA sequence in a vector, such as a plasmid. The RNA genome is then generated in a host cell via transcription following transfection of the host cell. Hence it will be understood that when referring to nucleic acid sequences of a positive-sense RNA virus, sequences in the "Sequence Listing" section can refer to RNA sequence (replacing "T" with "U") or DNA sequence.

The term "gene" as used herein refers to a DNA or RNA locus of heritable genomic sequence which affects an organism's (e.g., RNA virus) traits by being expressed as a functional product or by regulation of gene expression. Genes and polynucleotides may include introns and exons as in genomic sequence, or just the coding sequences as in cDNAs, such as an open reading frame (ORF), comprising a start codon (methionine codon) and a translation stop codon. Genes and polynucleotides can also include regions that regulate their expression, such as transcription initiation, translation, and transcription termination. Thus, also included are regulatory elements such as a promoter.

The terms "nucleic acid", "nucleotide", and "polynucleotide" as used herein are used interchangeably and refer to a single or double- stranded polymer of deoxyribonucleotide bases or ribonucleotide bases read from the 5' to the 3' end and include double stranded DNA (dsDNA), single stranded DNA (ssDNA), single stranded RNA (ssRNA, negative-sense and positive-sense), double stranded RNA (dsRNA), genomic DNA, cDNA, cRNA, recombinant DNA, or recombinant RNA and derivatives thereof, such as those containing modified backbones.

The term "ribonucleic acid", "RNA" or "RNA oligonucleotide" as used herein describes a molecule consisting of a sequence of nucleotides, which are built of a nucleobase a ribose sugar, and a phosphate group. RNAs are usually single stranded molecules and can exert various functions.

The terms "upstream" and "downstream" refer to a relative position in DNA or RNA. Each strand of DNA or RNA possesses a 5' end and a 3' end, relating to the terminal carbon position of the deoxyribose or ribose units. By convention, "upstream" means towards the 5' end of a polynucleotide, whereas "downstream" means towards the 3' end of a polynucleotide. In the case of double stranded DNA, e.g., genomic DNA, the term "upstream" means towards the 5' end of the coding strand, whereas "downstream" means towards the 3' end of the coding strand.

The term "coding strand" or "positive-sense strand" refers to an RNA strand encoding for proteins.

The term "non-coding strand" "anti-sense strand" or "negative-sense strand" or "negative- strand" refers to an RNA strand that needs to be transcribed by an RNA-dependent RNA polymerase into a positive strand RNA prior to translation.

The term "encodes" and "codes for" refers broadly to any process whereby the information in a polymeric macromolecule is used to direct the production of a second molecule that is different from the first. The second molecule may have a chemical structure that is different from the chemical nature of the first molecule. For example, the term "encode" describes the process of semiconservative DNA replication, where one strand of a double-stranded DNA molecule is used as a template to encode a newly synthesized complementary sister strand by a DNA-dependent DNA polymerase. Further, a DNA molecule can encode an RNA molecule (e.g., by use of a DNA-dependent RNA polymerase) or an RNA molecule (negative stranded) can encode an RNA molecule (positive-stranded) (*e.g.*, by use of an RNA-dependent RNA polymerase). Also, an RNA molecule (positive-stranded) can encode a polypeptide, as in the process of translation. When used to describe the process of translation, the term "encode" also extends to the triplet codon that encodes an amino acid. An RNA molecule can also encode a DNA molecule, e.g., by the process of reverse transcription using an RNA-dependent DNA polymerase. When referring to a DNA molecule encoding a polypeptide, a process of transcription and translation is referred to.

The term "expression" as used herein refers to transcription and/or translation of a heterologous nucleic acid sequence within a host cell. The level of expression of a gene product of interest in a host cell may be determined on the basis of either the amount of the corresponding mRNA (or positive-stranded RNA) that is present in the cell, or the amount of the polypeptide encoded by the selected sequence. For example, RNA transcribed from a selected sequence can be quantified by Northern blot hybridization, ribonuclease RNA protection, in situ hybridization to cellular RNA, or by PCR, such as qPCR. Proteins encoded by a selected sequence can be quantitated by various methods, e.g., by ELISA, by Western blotting, by radioimmunoassay, by immunoprecipitation, by assaying for the biological activity of the protein, by immunostaining of the protein followed by FACS analysis or by homogeneous time-resolved fluorescence (HTRF) assays. The level of expression of a non-coding RNA, such as a miRNA or shRNA may be quantified by PCR, such as qPCR.

A "reference sequence" of a virus is a sequence that does not comprise any human made mutations (*i.e.*, a wild-type viral sequence) and is the viral sequence on which all other versions thereof are compared. For example, the reference SARS-CoV-2 virus is the originally isolated strain described by NIH GenBank Locus NC_045512 and as the hCoV-19 reference sequence by the Global Initiative on Sharing Avian Influenza Data (GISAID).

As used herein, "treatment" or "treating" is an approach for obtaining beneficial or desired results including clinical results. For purposes of this application, beneficial or desired clinical results include, but are not limited to, one or more of the following: preventing onset of the disease, strengthening immunity against the disease, decreasing one more symptoms resulting from the disease, diminishing the extent of the disease, stabilizing the disease (e.g., preventing or delaying the worsening of the disease), preventing or delaying the spread or transmission of the disease, preventing or delaying the occurrence or recurrence (i.e., infection or reinfection) of the disease, delay or slowing the progression of the disease, ameliorating the disease state, decreasing the dose of one or more other medications required to treat the disease, delaying the progression of the disease, increasing the quality of life, and/or prolonging survival. Also encompassed by "treatment" is a reduction of pathological consequence of the disease. The methods of the present application contemplate any one or more of these aspects of treatment.

"Preventing," as used herein, includes providing prophylaxis with respect to the occurrence or recurrence of a disease in a subject that may be predisposed or at risk to the disease but has not yet contracted or been diagnosed with the disease.

The term "vaccine" is used in the broadest sense and specifically covers any biological preparation that provides active acquired immunity to a particular infectious disease.

As used herein, the term "vaccinate" refers to clinical intervention designed to administer a therapeutically effective amount of a vaccine to an individual in need thereof in order to prevent infection, prophylactically immunize, prevent transmission, reduce severity, elicit an immune response, or treat an infection in an individual who has been or may be exposed to a pathogen such as viruses. For example, an individual who is effectively vaccinated may not contract or may contract only mild illness caused by an infection compared to an individual who is not vaccinated.

As used herein, "heterosubtypic immunity" refers to the ability to induce immunity in a subject across multiple virus species, subtypes, or subspecies. In this regard, a vaccine that is designed to target one virus subtype (e.g., influenza A virus subtype H5N1) would simultaneously provide cross-protection against other virus subtypes that were not specifically targeted by the vaccine, for example due to genetic similarities (e.g., IAV H1N1, H3N2, and/or H7N9). Heterosubtypic immunity may further extend to some viruses of different species of the same genus that are closely genetically related.

As used herein, the term "multivalent" as used in conjunction with a vaccine composition refers to the vaccine composition being capable of inducing immunity against two, three, or more, different virus species using one vaccine molecule. This includes virus species that are from the same virus genus or virus family, and/or that are from different virus families or virus genera. For example, the two or more viruses can be genetically distinct such that the likelihood of generating heterosubtypic immunity is low.

As used herein, an "effective amount" refers to an amount of an agent or drug effective to vaccinate or treat a virus infection in a subject. The "therapeutically effective amount" can vary depending on the compound or the cell, the disease and its severity and the age, weight, *etc.*, of the subject to be treated.

The terms "individual," "subject" and "patient" are used interchangeably herein to describe a mammal, including humans. In some embodiments, the individual is human. In some embodiments, the individual is a non-human. In some embodiments, an individual suffers from a respiratory infection. In some embodiments, the individual is in need of treatment.

The terms "polypeptide" and "protein" are used interchangeably to refer to a polymer of amino acid residues and are not limited to a minimum length. Such polymers of amino acid residues may contain natural or non-natural amino acid residues, and include, but are not limited to, peptides, oligopeptides, dimers, trimers, and multimers of amino acid residues. Both full-length proteins and fragments thereof are encompassed by the definition. The terms also include post-expression modifications of the polypeptide, for example, glycosylation, sialylation, acetylation, phosphorylation, and the like. Furthermore, for purposes of the present application, a "polypeptide" refers to a protein which includes modifications, such as deletions, additions, and substitutions (generally conservative in nature), to the native sequence, as long as the protein maintains the desired activity. These modifications may be deliberate, as through site-directed mutagenesis, or may be accidental, such as through mutations of hosts which produce the proteins or errors due to PCR amplification.

The term "substantially similar" or "substantially the same," as used herein, denotes a sufficiently high degree of similarity between two or more numeric values such that one of skill in the art would consider the difference between the two or more values to be of little or no biological and/or statistical significance within the context of the biological characteristic measured by said value. In some embodiments the two or more substantially similar values differ by no more than about any one of 5%, 10%, 15%, 20%, 25%, or 50%.

A polypeptide "variant" means a biologically active polypeptide having at least about 80% amino acid sequence identity and no more than 100% identity with the native sequence polypeptide after aligning the sequences and introducing gaps, if necessary, to achieve the maximum percent sequence identity. Such variants include, for instance, polypeptides wherein one or more amino acid residues are added, or deleted, at the N- or C-terminus of the polypeptide. In some embodiments, a variant has at least about 80% amino acid sequence identity. In some embodiments, a variant has at least about 90% amino acid sequence identity. In some embodiments, a variant has at least about 95% amino acid sequence identity with the native sequence polypeptide.

As used herein, "Percent (%) amino acid sequence identity" with respect to a peptide, polypeptide or antibody sequence are defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or MEGALIGN^{™} (DNASTAR) software. Those skilled in the art can determine appropriate parameters for measuring alignment, including any algorithms needed to achieve maximal alignment over the full length of the sequences being compared.

As used herein, "percent (%) nucleic acid sequence identity" and "homology" with respect to a nucleic acid, DNA, or RNA sequence are defined as the percentage of nucleic acid nucleotides in a candidate sequence that are identical with the nucleic acid nucleotides in the specific DNA or RNA sequence, after aligning the sequences and introducing gaps, if necessary, to achieve the maximum percent sequence identity, and not considering any conservative substitutions as part of the sequence identity. Alignment for purposes of determining percent nucleic acid sequence identity can be achieved in various ways that are within the skill in the art, for instance, using publicly available computer software such as BLAST, BLAST-2, ALIGN, or MEGALIGN^{™} (DNASTAR) software. Those skilled in the art can determine appropriate parameters for measuring alignment, including any algorithms needed to achieve maximal alignment over the full length of the sequences being compared.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some versions contain an intron(s).

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

The term "vector" is used to describe a polynucleotide that may be engineered to contain a cloned polynucleotide or polynucleotides that may be propagated in a host cell. A vector may include one or more of the following elements: an origin of replication, one or more regulatory sequences (such as, for example, promoters and/or enhancers) that regulate the expression of the polypeptide of interest, and/or one or more selectable marker genes (such as, for example, antibiotic resistance genes and genes that may be used in colorimetric assays, e.g., β-galactosidase). The term "expression vector" refers to a vector that is used to express a polypeptide of interest in a host cell.

The term "cell" includes the primary subject cell and its progeny. A "host cell" refers to a cell that may be or has been a recipient of a vector or isolated polynucleotide. Host cells may be prokaryotic cells or eukaryotic cells. Exemplary eukaryotic cells include mammalian cells, such as primate or non-primate animal cells; fungal cells, such as yeast; plant cells; and insect cells. Nonlimiting exemplary mammalian cells include, but are not limited to, NSO cells, PER.C6^{®} cells (Crucell), and 293 and CHO cells, and their derivatives, such as 293-6E and DG44 cells, respectively.

As used herein, by "pharmaceutically acceptable" or "pharmacologically compatible" is meant a material that is not biologically or otherwise undesirable, e.g., the material may be incorporated into a pharmaceutical composition administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug administration.

It is understood that embodiments of the invention described herein include "consisting of" and/or "consisting essentially of" embodiments.

Reference to "about" a value or parameter herein includes (and describes) variations that are directed to that value or parameter per se. For example, description referring to "about X" includes description of "X".

The term "about X-Y" used herein has the same meaning as "about X to about Y."

As used herein, reference to "not" a value or parameter generally means and describes "other than" a value or parameter. For example, the method is not used to treat cancer of type X means the method is used to treat cancer of types other than X.

As used herein and in the appended claims, the singular forms "a," "an," and "the" include plural referents unless the context clearly dictates otherwise.

### II. NANOCOMPLEX VACCINE COMPOSITION

Provided herein is a vaccine composition comprising one or more nano-complexes comprising i) a scaffold molecule comprising a nucleic acid and ii) an adaptor-antigen polypeptide comprising a target antigen fused to a viral nucleoprotein capable of binding to the nucleic acid. The scaffold molecule can comprise an RNA molecule or a DNA molecule. In some embodiments, the scaffold molecule comprises a viral RNA. In some embodiments, the target antigen is a viral antigen. In some embodiments, the viral RNA, the viral nucleoprotein, and/or the viral antigen are derived from different viruses. In some embodiments, the viral RNA, the viral nucleoprotein, and the viral antigen are derived from the same virus. In some embodiments, the viral RNA and the viral nucleoprotein are derived from the same virus. In some embodiments, the viral nucleoprotein and the viral antigen are derived from the same virus. In some embodiments, the viral nucleoprotein and the viral antigen are derived from different viruses. In some embodiments, the viral RNA and the viral nucleoprotein are derived from the same virus, and the viral antigen is derived from a different virus. In some embodiments, the viral RNA and the viral antigen are derived from the same virus, and the viral nucleoprotein is derived from a different virus.

In some embodiments, the molar ratio of the adaptor-antigen polypeptide to the scaffold molecule (e.g., viral RNA) is at least about 5:1. In some embodiments, the molar ratio of the adaptor-antigen polypeptide to the scaffold molecule is about 6:1 to about 12:1, such as any of about 6:1 to about 10:1, about 8:1 to about 12:1, or about 6:1 to about 8:1. For example, in some embodiments, the molar ratio of the adaptor-antigen polypeptide to the scaffold molecule is about any of 5:1, 6:1, 7:1, 8:1, 9:1, 10:1, 11:1, or 12:1, or ranges in between any of the ratios.

In some embodiments, there is provided a vaccine composition comprising nano-complexes, wherein the nano-complexes comprise: i) a scaffold molecule comprising a viral RNA and ii) an adaptor-antigen polypeptide comprising a viral antigen fused to a viral nucleoprotein capable of binding to the viral RNA. In some embodiments, the viral antigen is a SARS-CoV-2 protein or a fragment or variant thereof, such as a SARS-CoV-2 spike protein or a fragment or variant thereof, for example comprising an RBD peptide. In some embodiments, the SARS-CoV-2 viral antigen comprises the amino acid sequence of SEQ ID NO: 14 or 15, or a variant thereof having at least about 80% (*e.g.*, at least about any of 80%, 85%, 90%, 95%, 96%, 97%, 98%, 99%, or 100%) sequence identity to SEQ ID NO: 14 or 15. In some embodiments, the viral antigen is an influenza virus protein or a fragment or variant thereof, for example an influenza HA protein or a fragment or variant thereof. In some embodiments, the influenza viral antigen comprises an influenza A virus (IAV) antigen, such as comprising an amino acid sequence selected from the group consisting of SEQ ID NOs: 16-18 and 31, or a variant thereof having at least about 80% (*e.g.*, at least about any of 80%, 85%, 90%, 95%, 96%, 97%, 98%, 99%, or 100%) sequence identity to an amino acid sequence selected from the group consisting of SEQ ID NOs: 16-18 and 31. In some embodiments, the influenza viral antigen comprises an influenza B virus (IBV) antigen, e.g., an IBV viral antigen comprising the amino acid sequence of SEQ ID NO: 19 or 20, or a variant thereof having at least about 80% (*e.g.*, at least about any of 80%, 85%, 90%, 95%, 96%, 97%, 98%, 99%, or 100%) sequence identity to the amino acid sequence of SEQ ID NO: 19 or 20. In some embodiments, the viral antigen is an RSV protein or a fragment or variant thereof, for example an RSV F protein or a fragment or variant thereof. In some embodiments, the RSV viral antigen comprises the amino acid sequence of SEQ ID NO: 21 or 22, or a variant thereof having at least about 80% *(e.g.,* at least about any of 80%, 85%, 90%, 95%, 96%, 97%, 98%, 99%, or 100%) sequence identity to SEQ ID NO: 21 or 22. In some embodiments, the viral antigen is an HCV protein or a fragment or variant thereof, for example an HCV E2 protein or a fragment or variant thereof. In some embodiments, the HCV viral antigen comprises the amino acid sequence of SEQ ID NO: 23 or 24, or a variant thereof having at least about 80% *(e.g.,* at least about any of 80%, 85%, 90%, 95%, 96%, 97%, 98%, 99%, or 100%) sequence identity to SEQ ID NO: 23 or 24. In some embodiments, the viral RNA, the viral nucleoprotein, and/or the viral antigen are derived from different viruses, such as different virus families, genus, species, or subtypes. In some embodiments, the viral RNA, the viral nucleoprotein, and the viral antigen are derived from the same virus. In some embodiments, the viral RNA and the viral nucleoprotein are derived from the same virus. In some embodiments, the viral nucleoprotein and the viral antigen are derived from the same virus. In some embodiments, the viral nucleoprotein and the viral antigen are derived from different viruses. In some embodiments, the viral RNA and the viral nucleoprotein are derived from the same virus, and the viral antigen is derived from a different virus. In some embodiments, the viral RNA and the viral antigen are derived from the same virus, and the viral nucleoprotein is derived from a different virus. In some embodiments, the nano-complexes in the vaccine composition have an average particle size of about 100nm to about 130nm. In some embodiments, the nano-complexes in the vaccine composition have a zeta-potential of about -10mV to about -20mV. In some embodiments, the molar ratio of the adaptor-antigen polypeptide to the scaffold molecule is at least about 5:1 (e.g., about 6:1 to about 12:1). In some embodiments, the viral RNA is about 10 to about 500 *(e.g.,* about 200 to about 500) nucleotides long. In some embodiments, the viral antigen is fused to the C-terminus of the viral nucleoprotein.

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, and wherein the viral nucleoprotein is derived from SARS-CoV-2. In some embodiments, the viral nucleoprotein comprises the amino acid sequence of SEQ ID NO: 12, or a variant thereof having at least about 80% *(e.g.,* at least about any of 80%, 85%, 90%, 95%, 96%, 97%, 98%, 99%, or 100%) sequence identity to SEQ ID NO: 12. In some embodiments, the viral nucleoprotein and the viral antigen are derived from the same virus. In some embodiments, the viral RNA, the viral nucleoprotein, and/or the viral antigen are derived from different viruses, such as different virus families, genera, species, or subtypes. In some embodiments, the viral RNA, the viral nucleoprotein, and the viral antigen are derived from the same virus. In some embodiments, the viral RNA and the viral nucleoprotein are derived from the same virus. In some embodiments, the viral nucleoprotein and the viral antigen are derived from the same virus. In some embodiments, the viral nucleoprotein and the viral antigen are derived from different viruses. In some embodiments, the viral RNA and the viral nucleoprotein are derived from the same virus, and the viral antigen is derived from a different virus. In some embodiments, the viral RNA and the viral antigen are derived from the same virus, and the viral nucleoprotein is derived from a different virus. In some embodiments, the nano-complexes in the vaccine composition have an average particle size of about 100nm to about 130nm. In some embodiments, the nano-complexes in the vaccine composition have a zeta-potential of about -10mV to about -20mV. In some embodiments, the molar ratio of the adaptor-antigen polypeptide to the scaffold molecule is at least about 5:1 (*e.g.*, about 6:1 to about 12:1). In some embodiments, the viral RNA is about 10 to about 500 (*e.g.*, about 200 to about 500) nucleotides long. In some embodiments, the viral RNA is derived from SARS-CoV-2, such as comprising nucleotides 1-266 of a viral gene of the SARS-CoV-2 genome set forth in SEQ ID NO:25. In some embodiments, the viral antigen is fused to the C-terminus of the viral nucleoprotein via an optional linker (e.g., GS linker).

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, and wherein the viral nucleoprotein is derived from IAV (e.g., H1N1, H3N2, H5N1, or H7N9). In some embodiments, the viral nucleoprotein comprises the amino acid sequence of SEQ ID NO: 13, or a variant thereof having at least about 80% *(e.g.,* at least about any of 80%, 85%, 90%, 95%, 96%, 97%, 98%, 99%, or 100%) sequence identity to SEQ ID NO: 13. In some embodiments, the viral nucleoprotein and the viral antigen are derived from the same virus. In some embodiments, the viral RNA, the viral nucleoprotein, and/or the viral antigen are derived from different viruses, such as different virus families, genera, species, or subtypes. In some embodiments, the viral RNA, the viral nucleoprotein, and the viral antigen are derived from the same virus. In some embodiments, the viral RNA and the viral nucleoprotein are derived from the same virus. In some embodiments, the viral nucleoprotein and the viral antigen are derived from the same virus. In some embodiments, the viral nucleoprotein and the viral antigen are derived from different viruses. In some embodiments, the viral RNA and the viral nucleoprotein are derived from the same virus, and the viral antigen is derived from a different virus. In some embodiments, the viral RNA and the viral antigen are derived from the same virus, and the viral nucleoprotein is derived from a different virus. In some embodiments, the nano-complexes in the vaccine composition have an average particle size of about 100nm to about 130nm. In some embodiments, the nano-complexes in the vaccine composition have a zeta-potential of about -10mV to about -20mV. In some embodiments, the molar ratio of the adaptor-antigen polypeptide to the scaffold molecule is at least about 5:1 (e.g., about 6:1 to about 12:1). In some embodiments, the viral RNA is about 10 to about 500 (*e.g.*, about 200 to about 500) nucleotides long. In some embodiments, the viral antigen is fused to the C-terminus of the viral nucleoprotein. In some embodiments, the viral antigen is fused to the C-terminus of the viral nucleoprotein via an optional linker (e.g., GS linker).

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, and wherein the viral RNA comprises (or consists essentially of, or consists of) an untranslated region of a viral genome (*e.g.*, a 5' untranslated region or a 3' untranslated region of a viral gene of the viral genome). In some embodiments, the viral RNA comprises an untranslated region (*e.g.*, a 5' untranslated region or a 3' untranslated region) of a viral gene of the genome of SARS-CoV-2. In some embodiments, the viral RNA comprises nucleotides 1-266 of a viral gene of the genome of SARS-CoV-2 (e.g., as set forth in SEQ ID NO:25). In some embodiments, the viral RNA and the viral nucleoprotein are derived from the same virus. In some embodiments, the viral nucleoprotein is derived from SARS-CoV-2. In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, and wherein the viral RNA comprises nucleotides 1-266 of a viral gene of the SARS-CoV-2 genome set forth in SEQ ID NO:25. In some embodiments, the viral nucleoprotein is derived from IAV. In some embodiments, the viral RNA comprises an untranslated region (*e.g.*, a 5' untranslated region or a 3' untranslated region) of the genome of IAV. In some embodiments, the viral RNA comprises nucleotides 1-326 of a viral gene of the genome of IAV (e.g., as set forth in SEQ ID NO:30). Hence in some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, and wherein the viral RNA comprises nucleotides 1-326 of a viral gene of the IAV genome set forth in SEQ ID NO:30. In some embodiments, the viral RNA, the viral nucleoprotein, and/or the viral antigen are derived from different viruses, such as different virus families, genera, species, or subtypes. In some embodiments, the viral RNA, the viral nucleoprotein, and the viral antigen are derived from the same virus. In some embodiments, the viral RNA and the viral nucleoprotein are derived from the same virus. In some embodiments, the viral nucleoprotein and the viral antigen are derived from the same virus. In some embodiments, the viral nucleoprotein and the viral antigen are derived from different viruses. In some embodiments, the viral RNA and the viral nucleoprotein are derived from the same virus, and the viral antigen is derived from a different virus. In some embodiments, the viral RNA and the viral antigen are derived from the same virus, and the viral nucleoprotein is derived from a different virus. In some embodiments, the viral RNA is about 10 to about 500 (*e*.*g*., about 200 to about 500, or about 200 to about 300) nucleotides long. In some embodiments, the nano-complexes in the vaccine composition have an average particle size of about 100nm to about 130nm. In some embodiments, the nano-complexes in the vaccine composition have a zeta-potential of about -10mV to about -20mV. In some embodiments, the molar ratio of the adaptor-antigen polypeptide to the scaffold molecule is at least about 5:1 (*e*.*g*., about 6:1 to about 12:1). In some embodiments, the viral antigen is fused to the C-terminus of the viral nucleoprotein via an optional linker (e.g., GS linker).

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, wherein the viral nucleoprotein is derived from SARS-CoV-2, and wherein the viral RNA comprises a 5' untranslated region or a 3' untranslated region of a viral gene of a viral genome (such as 5' or 3' UTR of a SARS-CoV-2 gene or an IAV gene). In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, wherein the viral nucleoprotein is derived from SARS-CoV-2, and wherein the viral RNA comprises (a) a 5' untranslated region (e.g., nucleotides 1-266) of a viral gene of the genome of SARS-CoV-2 (SARS-CoV-2 reference sequence: SEQ ID NO: 25) or (b) a 5' untranslated region (*e*.*g*., nucleotides 1-326) of a viral gene of the genome of IAV (IAV H7N9 reference sequence: SEQ ID NO:30). In some embodiments, the viral nucleoprotein comprises the amino acid sequence of SEQ ID NO: 12, or a variant thereof having at least about 80% (*e.g.*, at least about any of 80%, 85%, 90%, 95%, 96%, 97%, 98%, 99%, or 100%) sequence identity to SEQ ID NO: 12. In some embodiments, the viral antigen is a SARS-Cov-2 protein or a fragment or a variant thereof, such as a SARS-CoV-2 spike protein or a fragment or variant thereof, for example comprising an RBD peptide. In some embodiments, the viral antigen is an influenza virus protein or a fragment or variant thereof, for example an influenza HA protein or a fragment or variant thereof. In some embodiments, the viral antigen is an RSV protein or a fragment or variant thereof, for example an RSV F protein or a fragment or variant thereof. In some embodiments, the viral antigen is an HCV protein or a fragment or variant thereof, for example an HCV E2 protein or a fragment or variant thereof. In some embodiments, the viral RNA, the viral nucleoprotein, and/or the viral antigen are derived from different viruses, such as different virus families, genera, species, or subtypes. In some embodiments, the viral RNA, the viral nucleoprotein, and the viral antigen are derived from the same virus. In some embodiments, the viral RNA and the viral nucleoprotein are derived from the same virus. In some embodiments, the viral nucleoprotein and the viral antigen are derived from the same virus. In some embodiments, the viral nucleoprotein and the viral antigen are derived from different viruses. In some embodiments, the viral RNA and the viral nucleoprotein are derived from the same virus, and the viral antigen is derived from a different virus. In some embodiments, the viral RNA and the viral antigen are derived from the same virus, and the viral nucleoprotein is derived from a different virus. In some embodiments, the nano-complexes in the vaccine composition have an average particle size of about 100nm to about 130nm. In some embodiments, the nano-complexes in the vaccine composition have a zeta-potential of about -10mV to about -20mV. In some embodiments, the molar ratio of the adaptor-antigen polypeptide to the scaffold molecule is at least about 5:1 (*e.g.*, about 6:1 to about 12:1). In some embodiments, the viral RNA is about 10 to about 500 (*e.g.*, about 200 to about 300) nucleotides long. In some embodiments, the viral antigen is fused to the C-terminus of the viral nucleoprotein via an optional linker (e.g., GS linker).

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, wherein the viral nucleoprotein is derived from IAV (e.g., H1N1, H3N2, H5N1, or H7N9), and wherein the viral RNA comprises a 5' untranslated region or a 3' untranslated region of a viral gene of a viral genome (such as 5' or 3' UTR of a SARS-CoV-2 gene or an IAV gene). In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, wherein the viral nucleoprotein is derived from IAV, and wherein the viral RNA comprises (a) a 5' untranslated region (*e*.*g*., nucleotides 1-266) of a viral gene of the genome of SARS-CoV-2 (SARS-CoV-2 reference sequence: SEQ ID NO: 25) or (b) a 5' untranslated region (*e*.*g*., nucleotides 1-326) of a viral gene of the genome of IAV (IAV H7N9 reference sequence: SEQ ID NO: 30). In some embodiments, the viral nucleoprotein comprises the amino acid sequence of SEQ ID NO: 13, or a variant thereof having at least about 80% (*e.g.*, at least about any of 80%, 85%, 90%, 95%, 96%, 97%, 98%, 99%, or 100%) sequence identity to SEQ ID NO: 13. In some embodiments, the viral antigen is a SARS-Cov-2 protein or a fragment or variant thereof, such as a SARS-CoV-2 spike protein or a fragment or variant thereof, for example comprising an RBD peptide. In some embodiments, the viral antigen is an influenza virus protein or a fragment or variant thereof, for example an influenza HA protein or a fragment or variant thereof. In some embodiments, the viral antigen is an RSV protein or a fragment or variant thereof, for example an RSV F protein or a fragment or variant thereof. In some embodiments, the viral antigen is an HCV protein or a fragment or variant thereof, for example an HCV E2 protein or a fragment or variant thereof. In some embodiments, the viral RNA, the viral nucleoprotein, and/or the viral antigen are derived from different viruses, such as different virus families, genera, species, or subtypes. In some embodiments, the viral RNA, the viral nucleoprotein, and the viral antigen are derived from the same virus. In some embodiments, the viral RNA and the viral nucleoprotein are derived from the same virus. In some embodiments, the viral nucleoprotein and the viral antigen are derived from the same virus. In some embodiments, the viral nucleoprotein and the viral antigen are derived from different viruses. In some embodiments, the viral RNA and the viral nucleoprotein are derived from the same virus, and the viral antigen is derived from a different virus. In some embodiments, the viral RNA and the viral antigen are derived from the same virus, and the viral nucleoprotein is derived from a different virus. In some embodiments, the nano-complexes in the vaccine composition have an average particle size of about 100nm to about 130nm. In some embodiments, the nano-complexes in the vaccine composition have a zeta-potential of about -10mV to about -20mV. In some embodiments, the molar ratio of the adaptor-antigen polypeptide to the scaffold molecule is at least about 5:1 (*e*.*g*., about 6:1 to about 12:1). In some embodiments, the viral RNA is about 10 to about 500 (*e.g.*, about 200 to about 500) nucleotides long. In some embodiments, the viral antigen is fused to the C-terminus of the viral nucleoprotein. In some embodiments, the viral antigen is fused to the C-terminus of the viral nucleoprotein via an optional linker (e.g., GS linker).

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, wherein the viral nucleoprotein is derived from SARS-CoV-2 (e.g., comprising SEQ ID NO: 12), wherein the viral RNA comprises 5' untranslated region of a gene of the SARS-CoV-2 genome (e.g., nucleotides 1-266 of a viral gene of the SARS-CoV-2 genome sequence of SEQ ID NO: 25), and wherein the viral antigen is a SARS-Cov-2 protein or a fragment or variant thereof (*e.g.*, a SARS-CoV-2 spike protein or a fragment or variant thereof, such as comprising an RBD peptide). In some embodiments, the SARS-CoV-2 viral antigen comprises the amino acid sequence of SEQ ID NO: 14 or 15, or a variant thereof having at least about 80% (*e.g.*, at least about any of 80%, 85%, 90%, 95%, 96%, 97%, 98%, 99%, or 100%) sequence identity to SEQ ID NO: 14 or 15. In some embodiments, the viral antigen is fused to the C-terminus of the viral nucleoprotein via an optional linker (e.g., GS linker). In some embodiments, the viral RNA, the viral nucleoprotein, and/or the viral antigen are derived from different virus strains, such as different virus subspecies, variants, subvariants, or subtypes (e.g., SARS-CoV-2 variants such as B.1.1.7, B.1.351, B.1.526, B1.526.1, B1.617, B.1.617.1, B.1.617.2, B1.617.3, P.2, P.1 or B.1.1.28.1, A.23.1, CAL.20C, B.1.427, B.1.429, B.1.525, BA.2, BA.5, BA.2.75.2, BQ.1, BQ.1.1, XBB, and/or P.1.351). In some embodiments, the viral RNA, the viral nucleoprotein, and the viral antigen are derived from the same virus strain. In some embodiments, the viral RNA and the viral nucleoprotein are derived from the same virus strain. In some embodiments, the viral nucleoprotein and the viral antigen are derived from the same virus strain. In some embodiments, the viral nucleoprotein and the viral antigen are derived from different virus strains. In some embodiments, the viral RNA and the viral nucleoprotein are derived from the same virus strain, and the viral antigen is derived from a different virus strain. In some embodiments, the viral RNA and the viral antigen are derived from the same virus strain, and the viral nucleoprotein is derived from a different virus strain. In some embodiments, the adaptor-antigen polypeptide comprises the amino acid sequence of SEQ ID NO: 1 or 2. In some embodiments, the nano-complexes in the vaccine composition have an average particle size of about 100nm to about 130nm. In some embodiments, the nano-complexes in the vaccine composition have a zeta-potential of about - 10mV to about -20mV. In some embodiments, the molar ratio of the adaptor-antigen polypeptide to the scaffold molecule is at least about 5:1 (*e.g.*, about 6:1 to about 12:1). In some embodiments, the viral RNA is about 10 to about 300 (*e.g.*, about 200 to about 300) nucleotides long.

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, wherein the viral nucleoprotein is derived from SARS-CoV-2 (e.g., comprising SEQ ID NO: 12), wherein the viral RNA comprises the 5' untranslated region of a gene of the SARS-CoV-2 genome (*e*.*g*., nucleotides 1-266 of a viral gene of the SARS-CoV-2 genome sequence of SEQ ID NO: 25), and wherein the viral antigen is an influenza virus protein or a fragment or variant thereof (*e.g.*, an influenza HA protein or a fragment or variant thereof). In some embodiments, the influenza virus protein or fragment or variant thereof is derived from IAV or IBV. In some embodiments, the viral antigen is fused to the C-terminus of the viral nucleoprotein via an optional linker (e.g., GS linker). In some embodiments, the influenza viral antigen is an IAV antigen comprising an amino acid sequence selected from the group consisting of SEQ ID NOs: 16-18 and 31, or a variant thereof having at least about 80% (*e.g*., at least about any of 80%, 85%, 90%, 95%, 96%, 97%, 98%, 99%, or 100%) sequence identity to the amino acid sequence selected from the group consisting of SEQ ID NOs: 16-18 and 31. In some embodiments, the adaptor-antigen polypeptide comprises the amino acid sequence of SEQ ID NO: 26 or 27. In some embodiments, the influenza viral antigen is an IBV antigen comprising the amino acid sequence of SEQ ID NO: 19 or 20, or a variant thereof having at least about 80% (*e.g.*, at least about any of 80%, 85%, 90%, 95%, 96%, 97%, 98%, 99%, or 100%) sequence identity to the amino acid sequence of SEQ ID NO: 19 or 20. In some embodiments, the viral RNA, the viral nucleoprotein, and/or the viral antigen are derived from different viruses, such as different virus families, genera, species, and/or subtypes. In some embodiments, the viral RNA and the viral nucleoprotein are derived from the same virus, and the viral antigen is derived from a different virus. In some embodiments, the viral RNA and the viral nucleoprotein are derived from different virus strains (e.g., virus subtypes or virus subspecies). In some embodiments, the viral RNA and the viral nucleoprotein are derived from the same virus strain. In some embodiments, the nano-complexes in the vaccine composition have an average particle size of about 100nm to about 130nm. In some embodiments, the nano-complexes in the vaccine composition have a zeta-potential of about -10mV to about -20mV. In some embodiments, the molar ratio of the adaptor-antigen polypeptide to the scaffold molecule is at least about 5:1 (*e*.*g*., about 6:1 to about 12:1). In some embodiments, the viral RNA is about 10 to about 300 (*e.g.*, about 200 to about 300) nucleotides long.

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, wherein the viral nucleoprotein is derived from SARS-CoV-2 (e.g., comprising SEQ ID NO: 12), wherein the viral RNA comprises 5' untranslated region of a gene of the SARS-CoV-2 genome (*e*.*g*., nucleotides 1-266 of a viral gene of the SARS-CoV-2 genome sequence of SEQ ID NO: 25), and wherein the viral antigen is an RSV virus protein or a fragment or variant thereof (*e.g.*, an RSV F protein or a fragment or variant thereof). In some embodiments, the RSV viral antigen comprises the amino acid sequence of SEQ ID NO: 21 or 22, or a variant thereof having at least about 80% *(e.g.,* at least about any of 80%, 85%, 90%, 95%, 96%, 97%, 98%, 99%, or 100%) sequence identity to SEQ ID NO: 21 or 22. In some embodiments, the viral antigen is fused to the C-terminus of the viral nucleoprotein via an optional linker (e.g., GS linker). In some embodiments, the viral RNA, the viral nucleoprotein, and/or the viral antigen are derived from different viruses, such as different virus families, genera, species, and/or subtypes. In some embodiments, the viral RNA and the viral nucleoprotein are derived from the same virus, and the viral antigen is derived from a different virus. In some embodiments, the viral RNA and the viral nucleoprotein are derived from different virus strains (e.g., virus subtypes or virus subspecies). In some embodiments, the viral RNA and the viral nucleoprotein are derived from the same virus strain. In some embodiments, the adaptor-antigen polypeptide comprises the amino acid sequence of SEQ ID NO: 8 or 9. In some embodiments, the nano-complexes in the vaccine composition have an average particle size of about 100nm to about 130nm. In some embodiments, the nano-complexes in the vaccine composition have a zeta-potential of about -10mV to about -20mV. In some embodiments, the molar ratio of the adaptor-antigen polypeptide to the scaffold molecule is at least about 5:1 (*e*.*g*., about 6:1 to about 12:1). In some embodiments, the viral RNA is about 10 to about 500 (*e.g.*, about 200 to about 300) nucleotides long.

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, wherein the viral nucleoprotein is derived from SARS-CoV-2 (e.g., comprising SEQ ID NO: 12), wherein the viral RNA comprises 5' untranslated region of a gene of the SARS-CoV-2 genome (*e*.*g*., nucleotides 1-266 of a viral gene of the SARS-CoV-2 genome sequence of SEQ ID NO: 25), and wherein the viral antigen is an HCV virus protein or a fragment or variant thereof (*e*.*g*., an HCV E2 protein or a fragment or variant thereof). In some embodiments, the HCV viral antigen comprises the amino acid sequence of SEQ ID NO: 23 or 24, or a variant thereof having at least about 80% (*e.g.*, at least about any of 80%, 85%, 90%, 95%, 96%, 97%, 98%, 99%, or 100%) sequence identity to SEQ ID NO: 23 or 24. In some embodiments, the viral antigen is fused to the C-terminus of the viral nucleoprotein via an optional linker (e.g., GS linker). In some embodiments, the viral RNA, the viral nucleoprotein, and/or the viral antigen are derived from different viruses, such as different virus families, genera, species, and/or subtypes. In some embodiments, the viral RNA and the viral nucleoprotein are derived from the same virus, and the viral antigen is derived from a different virus. In some embodiments, the viral RNA and the viral nucleoprotein are derived from different virus strains (e.g., virus subtypes or virus subspecies). In some embodiments, the viral RNA and the viral nucleoprotein are derived from the same virus strain. In some embodiments, the adaptor-antigen polypeptide comprises the amino acid sequence of SEQ ID NO: 10 or 11. In some embodiments, the nano-complexes in the vaccine composition have an average particle size of about 100nm to about 130nm. In some embodiments, the nano-complexes in the vaccine composition have a zeta-potential of about -10mV to about -20mV. In some embodiments, the molar ratio of the adaptor-antigen polypeptide to the scaffold molecule is at least about 5:1 (*e*.*g*., about 6:1 to about 12:1). In some embodiments, the viral RNA is about 10 to about 500 (*e.g.*, about 200 to about 300) nucleotides long.

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, wherein the viral nucleoprotein is derived from IAV (e.g., comprising SEQ ID NO: 13), wherein the viral RNA comprises the 5' untranslated region of a gene of the IAV genome (e.g., nucleotides 1-326 of a viral gene of the IAV genome sequence of SEQ ID NO: 30), and wherein the viral antigen is an influenza virus protein or a fragment or variant thereof *(e.g.,* an influenza HA protein or a fragment or variant thereof), and wherein the viral antigen is a SARS-Cov-2 protein or a fragment or variant thereof (*e.g.*, a SARS-CoV-2 spike protein or a fragment or variant thereof, such as comprising an RBD peptide). In some embodiments, the SARS-CoV-2 viral antigen comprises the amino acid sequence of SEQ ID NO: 14 or 15, or a variant thereof having at least about 80% *(e.g.,* at least about any of 80%, 85%, 90%, 95%, 96%, 97%, 98%, 99%, or 100%) sequence identity to SEQ ID NO: 14 or 15. In some embodiments, the viral antigen is fused to the C-terminus of the viral nucleoprotein via an optional linker (e.g., GS linker In some embodiments, the viral RNA, the viral nucleoprotein, and/or the viral antigen are derived from different viruses, such as different virus families, genera, species, and/or subtypes. In some embodiments, the viral RNA and the viral nucleoprotein are derived from the same virus, and the viral antigen is derived from a different virus. In some embodiments, the viral RNA and the viral nucleoprotein are derived from different virus strains (e.g., virus subtypes or virus subspecies). In some embodiments, the viral RNA and the viral nucleoprotein are derived from the same virus strain. In some embodiments, the adaptor-antigen polypeptide comprises the amino acid sequence of SEQ ID NO: 28 or 29. In some embodiments, the nano-complexes in the vaccine composition have an average particle size of about 100nm to about 130nm. In some embodiments, the nano-complexes in the vaccine composition have a zeta-potential of about -10mV to about -20mV. In some embodiments, the molar ratio of the adaptor-antigen polypeptide to the scaffold molecule is at least about 5:1 (*e*.*g*., about 6:1 to about 12:1). In some embodiments, the viral RNA is about 10 to about 500 (*e.g.*, about 200 to about 500) nucleotides long.

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, wherein the viral nucleoprotein is derived from IAV (e.g., comprising SEQ ID NO: 13), wherein the viral RNA comprises the 5' untranslated region of a gene of the IAV genome (e.g., nucleotides 1-326 of a viral gene of the IAV genome sequence of SEQ ID NO: 30), and wherein the viral antigen is an influenza virus protein or a fragment or variant thereof (*e.g.*, an influenza HA protein or a fragment or variant thereof). In some embodiments, the influenza virus protein or fragment or variant thereof is derived from IAV or IBV. In some embodiments, the viral antigen is fused to the C-terminus of the viral nucleoprotein via an optional linker (e.g., GS linker). In some embodiments, the influenza viral antigen is an IAV antigen comprising an amino acid sequence selected from the group consisting of SEQ ID NOs: 16-18 and 31, or a variant thereof having at least about 80% (*e.g.*, at least about any of 80%, 85%, 90%, 95%, 96%, 97%, 98%, 99%, or 100%) sequence identity to the amino acid sequence selected from the group consisting of SEQ ID NOs: 16-18 and 31. In some embodiments, the adaptor-antigen polypeptide comprises the amino acid sequence selected from the group consisting of SEQ ID NOs: 3-5. In some embodiments, the influenza viral antigen is an IBV antigen comprising the amino acid sequence of SEQ ID NO: 19 or 20, or a variant thereof having at least about 80% (*e.g.*, at least about any of 80%, 85%, 90%, 95%, 96%, 97%, 98%, 99%, or 100%) sequence identity to the amino acid sequence of SEQ ID NO: 19 or 20. In some embodiments, the adaptor-antigen polypeptide comprises the amino acid sequence of SEQ ID NO: 6 or 7. In some embodiments, the viral RNA, the viral nucleoprotein, and the viral antigen are derived from the same virus strain (e.g., virus subspecies or virus subtype). In some embodiments, the viral RNA and the viral nucleoprotein are derived from the same virus strain. In some embodiments, the viral nucleoprotein and the viral antigen are derived from the same virus strain. In some embodiments, the viral nucleoprotein and the viral antigen are derived from different virus strains. In some embodiments, the viral RNA and the viral nucleoprotein are derived from the same virus strain, and the viral antigen is derived from a different virus strain. In some embodiments, the viral RNA and the viral antigen are derived from the same virus strain, and the viral nucleoprotein is derived from a different virus strain. In some embodiments, the nano-complexes in the vaccine composition have an average particle size of about 100nm to about 130nm. In some embodiments, the nano-complexes in the vaccine composition have a zeta-potential of about -10mV to about -20mV. In some embodiments, the molar ratio of the adaptor-antigen polypeptide to the scaffold molecule is at least about 5:1 (*e.g.*, about 6:1 to about 12:1). In some embodiments, the viral RNA is about 10 to about 500 (*e.g.*, about 200 to about 500) nucleotides long.

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, wherein the viral nucleoprotein is derived from IAV (e.g., comprising SEQ ID NO: 13), wherein the viral RNA comprises the 5' untranslated region of a gene of the IAV genome (e.g., nucleotides 1-326 of a viral gene of the IAV genome sequence of SEQ ID NO: 30), and wherein the viral antigen is an influenza virus protein or a fragment or variant thereof (*e.g.*, an influenza HA protein or a fragment or variant thereof), and wherein the viral antigen is an RSV virus protein or a fragment or variant thereof (*e.g*., an RSV F protein or a fragment or variant thereof). In some embodiments, the RSV viral antigen comprises the amino acid sequence of SEQ ID NO: 21 or 22, or a variant thereof having at least about 80% (*e.g.*, at least about any of 80%, 85%, 90%, 95%, 96%, 97%, 98%, 99%, or 100%) sequence identity to SEQ ID NO: 21 or 22. In some embodiments, the viral antigen is fused to the C-terminus of the viral nucleoprotein via an optional linker (e.g., GS linker). In some embodiments, the viral RNA, the viral nucleoprotein, and/or the viral antigen are derived from different viruses, such as different virus families, genera, species, and/or subtypes. In some embodiments, the viral RNA and the viral nucleoprotein are derived from the same virus, and the viral antigen is derived from a different virus. In some embodiments, the viral RNA and the viral nucleoprotein are derived from different virus strains (e.g., virus subtypes or virus subspecies). In some embodiments, the viral RNA and the viral nucleoprotein are derived from the same virus strain. In some embodiments, the nano-complexes in the vaccine composition have an average particle size of about 100nm to about 130nm. In some embodiments, the nano-complexes in the vaccine composition have a zeta-potential of about - 10mV to about -20mV. In some embodiments, the molar ratio of the adaptor-antigen polypeptide to the scaffold molecule is at least about 5:1 (*e*.*g*., about 6:1 to about 12:1). In some embodiments, the viral RNA is about 10 to about 500 (*e.g.*, about 200 to about 500) nucleotides long.

In some embodiments, there is provided a vaccine composition comprising nano-complexes, wherein the nano-complexes comprise: i) a scaffold molecule comprising a viral RNA, and ii) an adaptor-antigen polypeptide comprising a viral antigen fused to a viral nucleoprotein capable of binding to the viral RNA, wherein the viral nucleoprotein is derived from IAV (e.g., comprising SEQ ID NO: 13), wherein the viral RNA comprises the 5' untranslated region of a gene of the IAV genome (e.g., nucleotides 1-326 of a viral gene of the IAV genome sequence of SEQ ID NO: 30), and wherein the viral antigen is an HCV virus protein or a fragment or variant thereof (e.g., an HCV E2 protein or a fragment or variant thereof). In some embodiments, the HCV viral antigen comprises the amino acid sequence of SEQ ID NO: 23 or 24, or a variant thereof having at least about 80% (*e.g.*, at least about any of 80%, 85%, 90%, 95%, 96%, 97%, 98%, 99%, or 100%) sequence identity to SEQ ID NO: 23 or 24. In some embodiments, the viral antigen is fused to the C-terminus of the viral nucleoprotein via an optional linker (e.g., GS linker). In some embodiments, the viral RNA, the viral nucleoprotein, and/or the viral antigen are derived from different viruses, such as different virus families, genera, species, and/or subtypes. In some embodiments, the viral RNA and the viral nucleoprotein are derived from the same virus, and the viral antigen is derived from a different virus. In some embodiments, the viral RNA and the viral nucleoprotein are derived from different virus strains (e.g., virus subtypes or virus subspecies). In some embodiments, the viral RNA and the viral nucleoprotein are derived from the same virus strain. In some embodiments, the nano-complexes in the vaccine composition have an average particle size of about 100nm to about 130nm. In some embodiments, the nano-complexes in the vaccine composition have a zeta-potential of about -10mV to about -20mV. In some embodiments, the molar ratio of the adaptor-antigen polypeptide to the scaffold molecule is at least about 5:1 (*e.g.*, about 6:1 to about 12:1). In some embodiments, the viral RNA is about 10 to about 500 (*e.g.*, about 200 to about 500) nucleotides long.

### Adaptor-antigen polypeptides

In some embodiments, an adaptor-antigen polypeptide comprises a target antigen fused to a nucleoprotein (e.g., viral nucleoprotein) that is capable of binding to a nucleic acid (*e.g.*, an RNA or DNA). In some embodiments, the target antigen is a viral antigen. In some embodiments, the RNA is a viral RNA, *e.g.*, a mini-viral RNA. In some embodiments, the RNA is a non-viral RNA. In some embodiments, the RNA is a host RNA. In some embodiments, the target antigen is fused to the N-terminus of the nucleoprotein (*e*.*g*., viral nucleoprotein). In some embodiments, the target antigen is fused to the C-terminus of the nucleoprotein (e.g., viral nucleoprotein). In some embodiments, there is a linker (*e.g.*, peptide linker) connecting the target antigen and the nucleoprotein. Any linker that can confer desired spacing between the adaptor-antigen polypeptide components and/or flexibility of the adaptor-antigen polypeptide can be used herein. In some embodiments, the linker comprises the amino acid sequence of GS.

### Nucleoproteins

In some embodiments, the nucleoprotein (*e*.*g*., viral nucleoprotein) comprises domains and/or subdomains capable of binding to a nucleic acid, for example single-stranded or double-stranded DNA or RNA. In some embodiments, the nucleoprotein comprises an RNA-binding domain. In some embodiments, the viral nucleoprotein is capable of binding to an RNA, for example a viral RNA (*e.g.*, a mini-viral RNA), a non-viral RNA (e.g., a host RNA). In some embodiments, the nucleic acid (*e.g.*, viral RNA) acts as a scaffold molecule.

In some embodiments, the nucleoprotein, e.g., the viral nucleoprotein naturally associates with a viral RNA. In some embodiments, multiple copies of the adaptor-antigen polypeptide (*e.g.*, 2, 3, 4, 5, 6, 7, 9, 10, 20, 30, 50, 60, 70, 80, 90, 100, or more copies of the adaptor-antigen polypeptide) are bound to a single viral RNA scaffold.

In some embodiments, the RNA scaffold (e.g., viral RNA) also acts as an adjuvant, such that the attached adaptor-antigen polypeptides encase the viral RNA and thereby mask the adjuvant to prevent an irritative response such as the inflammatory caused by traditional exogenous adjuvants upon mucosal administration of a traditional vaccine. In some embodiments, the attached adaptor-antigen polypeptides prevent the induction of an innate immune response (e.g., excessive innate immune response), including production (e.g., excessive production) of inflammatory cytokines, upon mucosal administration of the vaccine composition to an individual (*e.g.*, a human individual). Accordingly, in some embodiments, the vaccine composition provided herein induces fewer (or no) side effects upon mucosal administration to an individual (*e*.*g*., a human individual) than vaccines comprising traditional adjuvants as currently known in the art.

In some embodiments, the vaccine composition is phagocytosed directly by antigen presenting cells (APCs). In some embodiments, the RNA scaffold (e.g., viral RNA) activates the APC upon phagocytosis of the vaccine composition. In some embodiments, the attached adaptor-antigen polypeptides prevent the induction of an innate immune response prior to APC phagocytosis.

In some embodiments, the nucleoprotein is derived from a virus, such as any virus known or to be discovered, including any exemplary viruses described herein. In some embodiments, the nucleoprotein (*e*.*g*., viral nucleoprotein) is derived from SARS-CoV-2. In some embodiments, the nucleoprotein comprises (or consists essentially of, or consists of) a portion of a viral protein, for example the SARS-CoV-2 RBD. In some embodiments, the viral nucleoprotein comprises an amino acid sequence of SEQ ID NO: 12. In some embodiments, the nucleoprotein (*e*.*g*., viral nucleoprotein) is derived from IAV (e.g., IAV H7N9, for example IAV H7N9/ZJ). In some embodiments, the nucleoprotein comprises (or consists essentially of, or consists of) a portion of an IAV viral protein. In some embodiments, the viral nucleoprotein comprises an amino acid sequence of SEQ ID NO: 13. In some embodiments, the viral nucleoprotein is fused to a target antigen peptide, *e.g.*, a viral antigen peptide, such as any of the target antigen peptides described below. In some embodiments, the nucleoprotein and the target antigen are derived from the same source, *e.g.,* the same virus. In some embodiments, the nucleoprotein and the target antigen are derived from different sources, e.g., different viruses, such as different subtypes of a virus, different species of a virus, or different virus families. In some embodiments, the nucleoprotein and the scaffold molecule (e.g., nucleic acid, such as viral RNA) are derived from the same source, *e.g.,* the same virus. In some embodiments, the nucleoprotein and the scaffold molecule (e.g., nucleic acid, such as viral RNA) are derived from different sources, *e*.*g*., different viruses, such as different subtypes of a virus, different species of a virus, or different virus families.

### Target antigens

In some embodiments, the target antigen is a viral antigen. In some embodiments, the viral antigen is an antigen polypeptide (*e*.*g*., an immunogenic polypeptide). The viral antigen of the present disclosure can include any viral antigen known in the art or to be discovered. The viral antigen can be a wildtype protein, or a mutant or variant form (e.g., with increased or reduced immunogenic activity).

In some embodiments, the viral antigen is a peptide encoded by the viral genome. The viral antigen peptide can have a length of about 10 to about 500 amino acids (aa), e.g., about 100 to about 300 aa, about 200 to about 500 aa, about 10 to about 300 aa, about 100 to about 200 aa, about 10 to about 100 aa, about 200 to about 450 aa, about 300 to about 500 aa, or about 10 to about 80 aa. For example, the viral antigen peptide can have a length of about any of 10, 20, 30, 40, 50, 60, 70, 80, 90, 100, 120, 140, 160, 180, 200, 220, 240, 260, 280, 300, 350, 400, 450, or 500 amino acids, or a range in between any of the aa lengths. The major viral antigens that induce a protective antibody response are the surface glycoproteins of viruses that contain lipid envelopes, or the proteins present on the surface of icosahedral viruses. IgA antibodies recognize the same viral proteins as IgG antibodies. For instance, polyclonal IgA antibodies, like polyclonal IgG antibodies, recognize the hemagglutinin of influenza virus (see, e.g., Clements et al., J Clin Microbiol. 1986, 24(1): 157-160; and Murphy et al., Infect Immun. 1982, 36(3):1108-1108); the gp70 (fusion) and gp90 (attachment glycoprotein) of respiratory syncytial virus (see, *e*.*g*., Murphy et al., J Clin Microbiol. 1986, 23(6): 1009-1014); the gp340 of Epstein-Barr virus (see, *e.g.*, Yao et al., Int J Cancer 1991, 48(1):45-50); the viral protein (VP)1, VP2, and VP3 of polioviruses (see, *e.g.,* Zhaori et al., J Infect Dis. 1989, 159(6): 1018-1024); and the VP4 or VP7 of rotaviruses (see, *e.g.,* Conner et al., J Virol. 1991, 65(5):2562-2571; Richardson and Bishop, J Clin Microbiol. 1990, 28(9):1891-1897; and Shaw et al., J Virol. 1991, 65(6):3052-3059).

Exemplary target antigens derived from viral proteins as described below include virion surface or membrane proteins, for example SARS-CoV-2 spike (S; including receptor binding domain) protein, influenza hemagglutinin (HA) protein, respiratory syncytial virus (RSV) fusion (F) protein, or hepatitis C virus (HCV) envelope glycoprotein E2. For example, target antigens including viral envelope, viral membrane, or affiliated proteins may be beneficial targets for preventing viral entry into host cells. Target antigens including polymerases or viral nucleic acids may be beneficial for preventing the replication of viruses. The vaccine composition described herein can be designed to target antigens of interest, such as any of the viral antigens described herein. Viral antigens derived from exemplary target viruses may include any protein encoded by the exemplary target viruses such that the proteins can induce an immunological response within an individual (*e.g.*, a human individual).

In some embodiments, the viral antigen is a viral protein or a fragment or variant thereof. In some embodiments, the viral antigen is a viral surface glycoprotein or a fragment or variant thereof. The viral antigen peptide can have a length of about of about 10 to about 500 amino acids (aa), e.g., about 100 to about 300 aa, about 200 to about 500 aa, about 10 to about 300 aa, about 100 to about 200 aa, about 10 to about 100 aa, about 200 to about 450 aa, about 300 to about 500 aa, or about 10 to about 80 aa. For example, the viral antigen peptide can have a length of about any of 10, 20, 30, 40, 50, 60, 70, 80, 90, 100, 120, 140, 160, 180, 200, 220, 240, 260, 280, 300, 350, 400, 450, or 500 amino acids, or a range in between any of the aa lengths. In some embodiments, the viral antigen comprises (or consists essentially of, or consists of) an amino acid sequence selected from the group consisting of SEQ ID NOs: 14-24 and 31, or a variant thereof having at least about 80% (*e.g.*, at least about any of 80%, 85%, 90%, 95%, 96%, 97%, 98%, 99%, or 100%) sequence identity to an amino acid sequence selected from the group consisting of SEQ ID NOs: 14-24 and 31. In some embodiments, the viral nucleoprotein and the viral antigen are derived from the same virus. In some embodiments, the viral nucleoprotein and the viral antigen are derived from different viruses, such as different subtypes of a virus, different species of a virus, or different virus families. In some embodiments, the viral antigen and the viral RNA are derived from the same virus. In some embodiments, the viral antigen and the viral RNA are derived from the different viruses, such as different subtypes of a virus, different species of a virus, or different virus families.

In some embodiments, the viral antigen is a SARS-Cov-2 protein or a fragment or variant thereof. In some embodiments, the viral antigen is a SARS-CoV-2 spike protein or a fragment or variant thereof. In some embodiments, the viral antigen comprises an receptor-binding domain (RBD) peptide. In some embodiments, the viral antigen comprises an amino acid sequence of SEQ ID NO: 14 or 15, or a variant thereof having at least about 80% *(e.g.,* at least about any of 80%, 85%, 90%, 95%, 96%, 97%, 98%, 99%, or 100%) sequence identity to SEQ ID NO: 14 or 15.

In some embodiments, the viral antigen is an influenza virus protein or a fragment or variant thereof, such as derived from IAV or IBV. In some embodiments, the viral antigen is an influenza (*e*.*g*., IAV or IBV) HA protein or a fragment or variant thereof. In some embodiments, the influenza virus protein or fragment or variant thereof is derived from an IAV subtype selected from the group consisting of H1N1, H5N1, H3N2, and H7N9. In some embodiments, the IAV viral antigen comprises an amino acid sequence selected from the group consisting of SEQ ID NOs: 16-18 and 31, or a variant thereof having at least about 80% (*e.g.*, at least about any of 80%, 85%, 90%, 95%, 96%, 97%, 98%, 99%, or 100%) sequence identity to an amino acid sequence selected from the group consisting of SEQ ID NOs: 16-18 and 31. In some embodiments, the influenza virus protein or a fragment or variant thereof is derived from an IBV subtype of Yamagata or Victoria. In some embodiments, the IBV viral antigen comprises the amino acid sequence of SEQ ID NO: 19 or 20, or a variant thereof having at least about 80% (*e*.*g*., at least about any of 80%, 85%, 90%, 95%, 96%, 97%, 98%, 99%, or 100%) sequence identity SEQ ID NO: 19 or 20.

In some embodiments, the viral antigen is a respiratory syncytial virus (RSV) protein or a fragment or variant thereof, e.g., derived from RSV A subtype or RSV B subtype. In some embodiments, the viral antigen is an RSV (*e*.*g*., subtype A or subtype B) F protein or a fragment or variant thereof. In some embodiments, the viral antigen comprises an amino acid sequence of SEQ ID NO: 21 or 22, or a variant thereof having at least about 80% (*e.g.*, at least about any of 80%, 85%, 90%, 95%, 96%, 97%, 98%, 99%, or 100%) sequence identity to SEQ ID NO: 21 or 22.

In some embodiments, the viral antigen is a hepatitis C virus (HCV) protein or a fragment or variant thereof, such as derived from HCV genotype 1a or HCV genotype 2a. In some embodiments, the viral antigen is an HCV (*e*.*g*., genotype 1a or 2a) E2 protein or a fragment or variant thereof. In some embodiments, the viral antigen comprises an amino acid sequence of SEQ ID NO: 23 or 24, or a variant thereof having at least about 80% (*e.g.*, at least about any of 80%, 85%, 90%, 95%, 96%, 97%, 98%, 99%, or 100%) sequence identity to SEQ ID NO: 23 or 24.

### Exemplary viruses

The vaccine composition comprising nano-complexes can be designed to target (e.g., inducing immune response or building immunity against) any virus, e.g., any sequenced virus. The targeted virus can be a DNA virus or RNA virus. For example, the vaccine composition described herein can be designed to target a virus selected from any one of the *Orthomyxoviridae*, *Filoviridae*, *Flaviviridae*, *Coronaviridae*, or *Poxviridae* families, or any combinations thereof, including identified and unidentified genera, species, subtypes, strains, and reassortants thereof, such as Influenza virus A *(e.g.,* H1N1, H5N1, H3N2, H7N9), Influenza virus B, Influenza virus C, Influenza virus D, Ebola virus, Marburg virus, Yellow Fever virus, West Nile virus, *Dengue* virus, *Zika* virus, *Hepacivirus B*, *Hepacivirus C*, *Pegivirus A*, *Pegivirus B*, *Pegivirus C*, *Pestivirus A*, *Pestivirus C*, alpha coronaviruses 229E (HCoV-229E), New Haven coronavirus NL63 (HCoV-NL63), beta coronaviruses OC43 (HCoV-OC43), coronavirus HKU1 (HCoV-HKU1), Severe Acute Respiratory Syndrome coronavirus (SARS-CoV), Middle East Respiratory Syndrome coronavirus (MERS-CoV), Severe Acute Respiratory Syndrome Coronavirus 2 (SARS-CoV-2), smallpox virus (variola), vaccinia virus, cowpox virus, monkeypox virus, orf virus, pseudocowpox, bovine papular stomatitis virus, tanapox virus yaba monkey tumor virus, molluscum contagiosum virus (MCV), chikungunya, norovirus, enterovirus, respiratory syncytial virus (RSV), Junin virus, Lassa fever virus, Nipah virus, or Hendra virus. By way of example, four viruses *(i.e.,* sarbecovirus *(e.g.,* SARS-CoV-2 virus), influenza virus, respiratory syncytial virus, and hepatitis C virus) are described herein, including viral strains, variants, subspecies, or related species. In some embodiments, the nano-complexes described herein target any of the viruses described herein. In some embodiments, the viral nucleoprotein, viral antigen, and/or viral RNA or DNA are derived from any of the viruses described herein.

### i. Sarbecovirus

Sarbecovirus is a subgenus of the Betacoronavirus genus within the Coronaviridae family of viruses. Examples of sarbecoviruses include, but are not limited to, SARS-CoV/SARS-CoV-1, SARS-CoV-2, SC2r-CoV, SC2r-CoV GX-P5L, Bat CoV BtKY72, Bat CoV BM48-31, 16BO133, JTMC15, Bat SARS CoV Rf1, BtCoV HKU3, LYRa11, Bat SARS-CoV/Rp3, Bat SL-CoV YNLF_31C, Bat SL-CoV YNLF_34C, SHC014-CoV, WIV1, WIV16, Civet SARS-CoV, (Bat) Rc-o319, Bat SL-ZXC21, Bat SL-ZC45, Pangolin SARSr-CoV-GX, Pangolin SARSr-CoV-GD, Rs7327, Rs4231, Rs4084, Rf4092, JL2012, 273-2005, HeB2013, HuB2013, Rs4247, Longquan-140, HKU3-1, GX2013, Shaanxi2011, 279-2005, As6526, Yunnan2011, Rs4237, Rs4081, Bat RshSTT182, Bat RshSTT200, (Bat) RacCS203, (Bat) RmYN02, (Bat) RpYN06, YN2013, (Bat) RaTG13, (Bat) BANAL-52, and SARS-CoV-2 combined variants of concern (VOC) (for example, see Nature (2022) 603:913-918, hereby incorporated by reference in its entirety). A SARS-CoV-2 variant is defined as being a "variant of concern" (VOC) upon demonstration of the following: (i) an increase in transmissibility or other detrimental change in epidemiology, (ii) an increase in virulence or change in clinical disease presentation, (iii) escape from immunity derived from natural infection, and/or (iv) a decrease in effectiveness of public health or clinical counter-measures, such as vaccination, treatment in current clinical use, testing if the impact is such that it is not easily mitigated by standard, and laboratory quality and regulatory measures. Sarbecoviruses are generally understood to be enveloped, positive-sense single-stranded RNA viruses that enter host cells by binding to the angiotensin-converting enzyme 2 (ACE2) receptor. For example, SARS-CoV-2 binds both ACE2 and TMPRSS2, both of which are expressed by epithelial cells that can be found in various tissues, such as prostate, testis, ovary, uterus, breast, lung, oral, cardiac, nasal passageway, ileum, intestine, colon, stomach, thyroid, liver, gallbladder, pancreas, kidney, bladder, cornea, neural, placental, *etc.* Receptor expression levels are increased in various inflammatory disease states, including but not limited to, in hypertension, COPD, asthma, cardiovascular disease, diabetes, Crohn's disease (CD), inflammatory bowel disease (IBD), *etc.* As a result, individuals with any of these or similar diseases or with immunodeficiency diseases are at an increased risk for sarbecovirus infection and complications thereof.

In some embodiments, the sarbecovirus is selected from the group consisting of SARS-CoV, SARS-CoV-2, SC2r-CoV, RaTG13, SC2r-CoV GX-P5L, and SARS-CoV combined VOC. In some embodiments, the sarbecovirus is SARS-CoV-2.

A variant further can be termed a variant of interest, a variant of concern, or a variant of high consequence. In some embodiments, the sarbecovirus is a SARS-CoV-2 variant selected from the group consisting of a B.1.1.7 variant, a B.1.351 variant, a B.1.526 variant, a B1. 526.1 variant, a B.1.1.529 variant, a B1.617 variant, a B.1.617.1 variant, a B.1.617.2 variant, a B1.617.3 variant, a P.2 variant, a P.1 (also known as B.1.1.28.1) variant, an A.23.1 variant, a CAL.20C variant, a B.1.427 variant, a B.1.429 variant, a B.1.525 variant, a BA.2, a BA.5 variant, a BA.2.75.2 variant, a BQ.1 variant, a BQ.1.1 variant, an XBB variant, and a P.1.351 variant. In some embodiments, the SARS-CoV-2 virus comprises a variant spike gene, including but not limited to any one of BA.2, BA.5, BA.2.75.2, BQ.1, BQ.1.1, or XBB. Other variants of SARS-CoV-2 are known in the art. For example, see Gomez et al., Vaccines 9(3): 243, 2021 and Tang et al., Journal of Infection 82: e27-e28, 2021, which are incorporated herein by reference in their entirety.

A "SARS-CoV-2 genome" refers to the 29,903 nucleotides long sequence described by NIH GenBank Locus NC_045512, or the hCoV-19 reference sequence described by the Global Initiative on Sharing Avian Influenza Data (GISAID) (SEQ ID NO: 25). A DNA sequence for the SARS-CoV-2 genome, with coding regions, is available as accession number NC_045512.2 from the NCBI website. Viral genomes comprise subunits known as open reading frames (ORFs) that may encode for viral proteins. Within the SARS-CoV-2 genome, these subunits may include *ORF1ab*, RNA-dependent RNA polymerase, helicase, gene *S*, *nsp3*, *nsp4B*, *nsp9, ORF3a,* gene A, gene *M*/*OPF5*, *ORF6*, *ORF7a*, *ORF7b*, *ORF8*, gene *N*/*ORF9*, and *ORF10.*

*ORF1ab* encodes a large polyprotein that encompasses multiple of the proteins encoded by other ORFs described herein, as indicated in **Table 1** below. *Nsp3* encodes a protein that includes a transmembrane domain 1 (TM1), which has NCBI accession no. YP_009725299.1. The nsp3 protein has additional conserved domains including an N-terminal acidic (Ac), a predicted phosphoesterase, a papain-like proteinase, Y-domain, transmembrane domain 1 (TM1), and an adenosine diphosphate-ribose 1"-phosphatase (ADRP). *Nsp4b* encodes a protein that includes transmembrane domain 2 (TM2), which has NCBI accession no. YP_009725300. *Nsp9* encodes a ssRNA-binding protein with NCBI accession number YP_009725305.1. Genes *E*, *M*, *S*, and *N* encode the envelope, membrane, spike, and nucleocapsid proteins, respectively. The viral envelope protein is a membrane protein that is involved in viral assembly, budding, and envelope formation. Viral membrane proteins attach the virus to the host cell and promote fusion between viral and host cell membranes for viral entry. The viral spike protein binds to specific receptors on the host cell to engage in viral entry of host cells and initiation of host cell infection. The viral nucleocapsid protein binds to and encapsulates the viral RNA. Together, these four proteins make up the integral structural proteins found in sarbecoviruses, e.g., in SARS-CoV-2.

**Table 1. Locations of nucleotide sequences that encode SARS-CoV-2 viral proteins and ORFs within the full SARS-CoV-2 genome sequence (SEQ ID NO: 25).**

| **SARS-CoV-2 Subunit/Open Reading Frame** | **Nucleotide Location within hCoV-19 reference sequence** |
|---|---|
| Leader sequence/5' UTR | Position 1 - 265 |
| 3' UTR | Position 29,675 - 29,870 |
| Poly-A tail | Position 29,871 - 29,903 |
| *ORF1ab* | Position 266 - 21,552 |
| *nsp3* | Position 2,720 - 8,554 |
| *nsp4B* | Position 8,555 - 10,054 |
| *nsp9* | Position 12,686 - 13,024 |
| RNA-dependent RNA polymerase | Positions 13,442 - 13,468 and 13,468 - 16,236 |
| Helicase | Position 16,237 - 18,039 |
| Spike (*S*) | Position 21,563 - 25,384 |
| *ORF3a* | Position 25,393 - 26,220 |
| Envelope (*E*)/*ORF4* | Position 26,245 - 26,472 |
| Membrane (*M*)/*ORF5* | Position 26,523 - 27,191 |
| *ORF6* | Position 27,202 - 27,387 |
| *ORF7a* | Position 27,394 - 27,759 |
| *ORF7b* | Position 27,756 - 27,887 |
| *ORF8* | Position 27,894 - 28,259 |
| Nucleocapsid (*N*)/*ORF9* | Position 28,274 - 29,553 |
| *ORF10* | Position 29,558 - 29,674 |

In some embodiments, the viral antigen is a SARS-Cov-2 protein or a fragment or variant thereof. In some embodiments, the viral antigen is a SARS-CoV-2 spike protein or a fragment or variant thereof. In some embodiments, the viral antigen comprises an RBD peptide. In some embodiments, the viral nucleoprotein and the viral antigen are derived from the same virus. In some embodiments, the viral nucleoprotein and the viral antigen are derived from different viruses, such as different viral variants, or different viral families, genera, species, or subspecies. In some embodiments, the viral nucleoprotein is derived from SARS-CoV-2, e.g., comprising an RNA-binding domain derived from SARS-CoV-2. In some embodiments, the viral nucleoprotein comprises an amino acid sequence of SEQ ID NO: 12, or a variant thereof having at least about 80% *(e.g.,* at least about any of 80%, 85%, 90%, 95%, 96%, 97%, 98%, 99%, or 100%) sequence identity to SEQ ID NO: 12. In some embodiments, the viral RNA is derived from SARS-CoV-2. In some embodiments, the viral RNA is about 10 to about 500 (e.g., about 10 to about 300) nucleotides long. For example, in some embodiments, the viral RNA is about any of 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 nucleotides long, or a range in between any of the nucleotide lengths. In some embodiments, the viral RNA is about 10-50, about 30-60, about 40-70, about 50-80, about 60-90, about 70-100, about 10-100, about 80-110, about 90-120, about 100-130, about 110-140, about 120-150, about 130-160, about 140-170, about 150-180, about 160-190, about 170-200, about 180-210, about 190-220, about 200-230, about 210-240, about 220-250, about 230-260, about 240-270, about 250-280, about 260-290, about 270-300, about 280-310, about 290-320, about 300-330, about 310-340, about 320-350, about 330-360, about 340-370, about 350-380, about 360-390, about 370-400, about 380-410, about 390-420, about 400-430, about 410-440, about 420-450, about 430-460, about 440-470, about 450-480, about 460-490, or about 470-500 nucleotides long. In some embodiments, the viral RNA is about 200 to about 500 (e.g., about 200 to about 300) nucleotides long. For example, the viral RNA is at least about any of 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 nucleotides long. In some embodiments, the viral RNA comprises nucleotides 1-266 of a viral gene of the SARS-CoV-2 genome (e.g., SEQ ID NO:25) or a modified genome thereof.

### ii. Influenza

Influenza, or more commonly called the flu, is a contagious viral infection that can cause mild to severe symptoms and life-threatening complications, including death, even in healthy children and adults. Influenza viruses are members of the *Orthomyxoviridae* family of viruses. Examples of influenza virus include the genus *Alphainfluenzavirus* (Influenza A virus, or IAV), the genus *Betainfluenzavirus* (Influenza B virus, or IBV), the genus *Gammainfluenzavirus* (Influenza C virus, or ICV), and the genus *Deltainfluenzavirus* (Influenza D virus, or IDV). Each of the four species is the sole member of its own genus. IAV and IBV circulate in humans and cause seasonal epidemics, and ICV causes a mild infection, primarily in children. IDV can infect humans but is not known to cause illness.

Influenza A viruses are the only influenza viruses known to cause flu pandemics. IAVs are divided into subtypes based on two proteins on the surface of the virus: hemagglutinin (H) and neuraminidase (N). There are 18 different hemagglutinin subtypes and 11 different neuraminidase subtypes (H1 through H18, and N1 through N11, respectively). While more than 130 IAV subtype combinations have been identified in nature, primarily from wild birds, there are potentially many more IAV subtype combinations given the propensity for virus reassortment, whereby influenza viruses swap gene segments, e.g., when two influenza viruses infect a host at the same time and swap genetic information. For example, very recently, the H17N10 and H18N11 IAVs were identified in fruit bats from Central and South America, demonstrating that previously unidentified strains very likely are already in circulation in yet to be determined animal reservoirs. Current subtypes of IAVs that routinely circulate in people include H1N1 and H3N2, and further examples that have been monitored or otherwise a cause for concern in humans include, but may not be limited to, H1N1, H1N2, H2N2, H2N3, H3N2, H5N1, H7N7, and H7N9. IAV subtypes can be further broken down into different genetic clades and sub-clades (e.g., H1N1: clade 6B.1, sub-clade 6B1.A; or H3N2: clades 3C.2a or 3C.3a, sub-clades 3C.2a1, 3C.2a2, 3C.2a3, or 3C.2a4). In some embodiments, the IAV is selected from the group consisting of H1N1/pdm09, H5N1/1194, H3N2/4801, H7N9/AH1, and H7N9/ZJ.

Influenza B viruses can be separated into two distinct lineages: Yamagata and Victoria. IBV is the most prominent circulating strain of influenza every four to five years. Furthermore, IBV infections carry higher risks of hospitalizations compared to IAV, *e.g.,* in HIV patients, and IBV has demonstrated significantly higher mortality rates compared to IAV strains. IBV has shown limited antigenic drift compared to IAV, as well as a lack of animal reservoirs (see, *e.g.,* Sharma et al., Eur Respir J. 2019, 54(2): 1901325). IBV subtypes can be further broken down into different genetic clades and sub-clades, *e.g.,* B/Yamagata *(e.g.,* clades Y1, Y2, Y3), B/Victoria (e.g., subclades V1A.1, V1A.2, V1A.3). Influenza C virus infections generally cause mild illness and are not thought to cause human epidemics. Influenza D viruses (*i.e*., *Thogotovirus*) primarily affect cattle with spillover to other animals but are not known to infect humans or cause illness. See, *e.g.,* the CDC website titled Types of Influenza Viruses (www.cdc.gov/flu/about/viruses/types.)

Influenza viruses have a negative-sense, single-stranded segmented RNA genome. IAV and IBV have eight genome segments that encode 10 major proteins: PB1 polymerase, PB2 polymerase, PA polymerase, hemagglutinin (HA), nucleoprotein (NP), neuraminidase (NA), matrix 1 (M1), matrix 2 (M2), nonstructural 1 (NS1), and nonstructural 2 (NS2) proteins. ICV and IDV have seven genome segments that encode nine major proteins, wherein instead of HA and NA proteins, ICV and IDV genome encodes a hemagglutinin-esterase fusion (HEF) protein that merges the functions of HA and NA. Three segments encode three subunits of an RNA-dependent RNA polymerase (RdRp) complex: PB1, a transcriptase, PB2, which recognizes 5' caps, and PA (P3 for ICV and IDV), which is an endonuclease. The matrix protein (M1) and membrane protein (M2) share a segment, as do the non-structural protein (NS1) and the nuclear export protein (NEP). For IAV and IBV, hemagglutinin (HA) and neuraminidase (NA) are encoded on one segment each, whereas ICV and IDV encode a hemagglutinin-esterase fusion (HEF) protein on one segment. The final genome segment encodes the viral nucleoprotein (NP). Influenza viruses also encode several accessory proteins, such as PB1-F2 and PA-X, that can be expressed via alternative open reading frames, and which are important in host defense suppression, virulence, and pathogenicity.

The virion consists of the genome bound to nucleoproteins in separate ribonucleoprotein (RNP) complexes for each segment, which are surrounded by a lipid bilayer membrane (*i.e.*, the viral envelope). A copy of the RdRp with all subunits incorporated is bound to each RNP. The envelope is reinforced structurally by matrix proteins, M1 and M2, on the interior that enclose the RNPs, and the envelope contains HA and NA (or HEF in the case if ICV or IDV) proteins extending outward from the exterior surface of the envelope. M2 proteins form proton ion channels through the viral envelope that are required for viral entry and exit, including the acidification of the virion interior during viral uncoating and control of the pH within the cell's Golgi during HA synthesis. See, *e.g.*, Webster et al., Microbiol Rev. 1992, 56(1): 152-179; and Wille and Holmes, Cold Spring Harb Perspect Med. 2020, 10(7):a038489.

In some embodiments, the viral antigen is an influenza protein or a fragment or variant thereof, such as derived from IAV or IBV. In some embodiments, the influenza virus protein or a fragment or variant thereof is derived from an IAV subtype selected from the group consisting of H1N1, H5N1, H3N2, and H7N9. In some embodiments, the influenza virus protein or a fragment or variant thereof is derived from an IBV subtype of Yamagata or Victoria. In some embodiments, the viral antigen is an influenza HA protein or a fragment or variant thereof. For example, in some embodiments, the viral antigen is an IAV or IBV HA protein or a fragment or variant thereof. In some embodiments, the viral antigen is selected from the group consisting of an IAV H1N1 pdm09 HA head peptide, an IAV H5N1 1194 HA head peptide, IAV H3N2 4801 HA head peptide, an IAV H7N9/AH1 HA head peptide, an IAV H7N9/ZJ HA head peptide, an IBV Yamagata HA head, and an IBV Victoria HA head peptide. In some embodiments, the IAV viral antigen comprises an amino acid sequence selected from the group consisting of SEQ ID NOs:16-18 and 31. In some embodiments, the IBV viral antigen comprises the amino acid sequence of SEQ ID NO:19 or 20. In some embodiments, the adaptor-antigen polypeptide comprises an amino acid sequence selected from the group consisting of SEQ ID NOs: 3-7, 26, and 27, or a variant thereof having at least about 80% *(e.g.,* at least about any of 80%, 85%, 90%, 95%, 96%, 97%, 98%, 99%, or 100%) sequence identity to an amino acid sequence selected from the group consisting of SEQ ID NOs:3-7, 26, and 27. In some embodiments, the viral nucleoprotein and the viral antigen are derived from the same virus. In some embodiments, the viral nucleoprotein is derived from IAV (e.g., IAV H5N1). In some embodiments, the viral nucleoprotein comprises the amino acid sequence of SEQ ID NO: 13, or a variant thereof having at least about 80% (*e.g.*, at least about any of 80%, 85%, 90%, 95%, 96%, 97%, 98%, 99%, or 100%) sequence identity to SEQ ID NO:13. In some embodiments, the viral nucleoprotein and the viral antigen are derived from different viruses, such as different viral variants, or different viral families, genera, species, or subspecies. In some embodiments, the viral RNA is derived from influenza (e.g., IAV, for example IAV H7N9, such as H7N9/ZJ). In some embodiments, the viral RNA is about 10 to about 500 (e.g., about 10 to about 400) nucleotides long. For example, in some embodiments, the viral RNA is about any of 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, or 400, 410, 420, 430, 440, 450, 460, 470, 480, 490, or 500 nucleotides long, or a range in between any of the nucleotide lengths. In some embodiments, the viral RNA is about 10-50, about 30-60, about 40-70, about 50-80, about 60-90, about 70-100, about 10-100, about 80-110, about 90-120, about 100-130, about 110-140, about 120-150, about 130-160, about 140-170, about 150-180, about 160-190, about 170-200, about 180-210, about 190-220, about 200-230, about 210-240, about 220-250, about 230-260, about 240-270, about 250-280, about 260-290, about 270-300, about 280-310, about 290-320, about 300-330, about 310-340, about 320-350, about 330-360, about 340-370, about 350-380, about 360-390, about 370-400, about 380-410, about 390-420, about 400-430, about 410-440, about 420-450, about 430-460, about 440-470, about 450-480, about 460-490, or about 470-500 nucleotides long. In some embodiments, the viral RNA is about 200 to about 500 nucleotides long. For example, the viral RNA is at least about any of 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 nucleotides long. In some embodiments, the viral RNA comprises nucleotides 1-326 of a viral gene (e.g., PB2 gene) of an influenza A genome (e.g., H7N9, such as H7N9/ZJ; e.g., as set forth in SEQ ID NO:30) or modified genome thereof.

### iii. Respiratory Syncytial virus

Respiratory syncytial virus (RSV) belongs to the genus *Orthopneumovirus* within the family *Pneumoviridae* and order *Mononegavirales.* Members of this genus include human RSV, bovine RSV, and murine pneumonia virus. There are two major antigenic subtypes of human RSV (A and B) determined largely by antigenic drift and duplications in RSV-G sequences, but accompanied by genome-wide sequence divergence, including within RSV-F sequences.

In addition to the pathological burden on the pediatric population (e.g., children 5 years of age and younger, in particular infants 6 months of age and younger), RSV is increasingly being recognized as an important pathogen in older adults. RSV infection leads to an increase in hospitalization rates among those aged 65 years and over, and to an increased mortality rate among this population that approaches the rates seen with influenza.

Respiratory syncytial virus is an enveloped virus with a negative sense, single-stranded, non-segmented RNA genome of approximately 15,000 nucleotides, containing 10 genes that encode 11 proteins, and in order, these genes are nonstructural 1 (Ns1), nonstructural 2 (Ns2), nucleoprotein (N), phosphoprotein (P), matrix (M), small hydrophobic protein (SH), glycoprotein (G), fusion (F), M2-1 cofactor, M2-2 cofactor, and RNA-dependent RNA polymerase (L). The attachment glycoprotein (G) and the fusion glycoprotein (F) embedded in virion envelope mediate virus attachment and entry, respectively.

RSV G enhances pathogenicity *in vivo* and facilitates attachment to host cells but is not absolutely required for infection. RSV G protein is produced in two forms: a full-length membrane-bound form containing about 298 amino acids (without insertions), and a secreted form (sG) translated through internal initiation at the second AUG codon (Met48). The full-length G protein is a type II membrane protein and contains an N-terminal cytosolic domain (amino acids (aa) 1-36), a transmembrane domain (aa 37-67), and an ectodomain (aa 68-298). The ectodomain consists of two hypervariable mucin-like domains flanking a conserved central domain (CCD) and a heparin-binding domain (HBD). The two mucin-like domains contain 30 to 40 O-linked glycans and 3 to 5 N-linked glycans and are associated with the antigenicity of RSV. The CCD is a target of protective RSV neutralizing antibodies and contains three amino acids that constitute a CX3C motif that interacts with the human chemokine receptor CX3CR1, which is a critical step for RSV infection. The HBD facilitates binding to potential attachment receptors of RSV, including heparan sulfate and other glycosaminoglycans (GAGs). See Zhao et al., Front Microbiol. 2022, 13:966235.

RSV F is a class I fusion protein that mediates fusion of the viral membrane with a host-cell membrane, a process that poses an otherwise insurmountable energetic barrier to viral entry. Initially, the F protein exists in a metastable prefusion conformation that is anchored in the viral membrane by a transmembrane domain. In this conformation the hydrophobic fusion peptide is buried within the central cavity of the protein. An unknown event causes prefusion F to undergo a dramatic conformational change, resulting in refolding of five distinct structural elements adjacent to the fusion peptide into a single alpha helix and insertion of the fusion peptide into the host-cell membrane. In this state, called the pre-hairpin intermediate, two heptad repeats (HRA and HRB) are positioned on opposing ends of the F molecule. The collapse of these heptad repeats into a stable six-helix bundle is an energetically favorable conformational change that is coupled to the fusion of the two lipid bilayers. Antibodies that bind to F can interrupt the fusion process, thereby preventing viral entry and reducing the severity of RSV-related disease. Recently, it has been established that antibodies that specifically recognize the prefusion conformation of F are in general more potently neutralizing than antibodies that also bind to postfusion F. See Gilman et al., Nat Commun. 2019, 10(1):2105.

In some embodiments, the viral antigen is a respiratory syncytial (RSV) protein or a fragment or variant thereof. In some embodiments, the viral antigen is an RSV F protein or a fragment or variant thereof. In some embodiments, the RSV F protein or a fragment or variant thereof is derived from RSV A subtype or RSV B subtype. In some embodiments, the RSV viral antigen comprises the amino acid sequence of SEQ ID NO:21 or 22. In some embodiments, the viral nucleoprotein and the viral antigen are derived from the same virus. In some embodiments, the viral nucleoprotein and the viral antigen are derived from different viruses, such as different viral variants, or different viral families, genera, species, or subspecies. In some embodiments, the viral nucleoprotein is derived from RSV. In some embodiments, the viral RNA is derived from RSV. In some embodiments, the viral RNA is about 10 to about 500 nucleotides long. For example, in some embodiments, the viral RNA is about any of 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 nucleotides long, or a range in between any of the nucleotide lengths. In some embodiments, the viral RNA is about 10-50, about 30-60, about 40-70, about 50-80, about 60-90, about 70-100, about 80-110, about 90-120, about 10-100, about 100-130, about 110-140, about 120-150, about 130-160, about 140-170, about 150-180, about 160-190, about 170-200, about 180-210, about 190-220, about 200-230, about 210-240, about 220-250, about 230-260, about 240-270, about 250-280, about 260-290, about 270-300, about 280-310, about 290-320, about 300-330, about 310-340, about 320-350, about 330-360, about 340-370, about 350-380, about 360-390, about 370-400, about 380-410, about 390-420, about 400-430, about 410-440, about 420-450, about 430-460, about 440-470, about 450-480, about 460-490, or about 470-500 nucleotides long. In some embodiments, the viral RNA is at least about 200 nucleotides long. For example, the viral RNA is at least about any of 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 nucleotides long.

### iv. Hepatitis: Hepatitis C virus

Hepatitis C virus (HCV, *Hepacivirus*) belongs to the genus *Hepacivirus* within the family *Flaviviridae.* HCV includes least seven genotypes *(i.e.,* gt1-7) and numerous subtypes. Hepatitis C virus (HCV) is a small, enveloped, positive single-stranded RNA virus approximately 9600 nucleotides long. HCV encodes a single polyprotein, which is processed by cellular and viral proteases to generate 10 polypeptides. The HCV genome also contains an overlapping +1 reading frame that may lead to the synthesis of an additional protein *(i.e.,* F protein from the alternative reading frame). The 10 polypeptides cleaved from the single polyprotein include: three structural proteins that include a core protein and two envelope proteins (core, E1, E2), and seven non-structural (NS) proteins NS1, NS3, NS4A, NS4B, NS5A, and NS5B that are involved in cleavage, assembly, transcription, and additional functions. The structural and non-structural proteins of HCV are multifunctional.

The core protein forms the nucleocapsid. The mature form of the core protein is a dimeric alpha-helical protein, which behaves as a membrane protein and as an RNA-binding protein. Viral spikes on the virion membrane are formed by heterodimers of E1 and E2 glycoproteins. These E1 and E2 proteins are essential for HCV virus entry into host cells and participate in the assembly of infectious viral particles. See Dubuisson, World J Gastroenterol. 2007, 13(17):2406-2415.

**Table 2. Locations of amino acid sequences that encode HC viral proteins.**

| **HCV Subunit/Polypeptide** | **Amino acid Location within HCV reference sequence** |
|---|---|
| Core immature | Position 1 - 191 |
| Core mature | Position 1-174 |
| F protein of ARF protein | Position 126-161 |
| E1 | Position 192-383 |
| E2 | Position 384-746 |
| P7 | Position 747-809 |
| NS2 | Position 810-1026 |
| NS3 | Positions 1027-1657 |
| NS4A | Position 1658-1711 |
| NS4B | Position 1712-1972 |
| NS5A | Position 1973-2420 |
| NS5B | Position 2421-3011 |

In some embodiments, the viral antigen is a hepatitis C (HCV) protein or a fragment or variant thereof. In some embodiments, the viral antigen is an HCV E2 protein or a fragment or variant thereof. In some embodiments, the HCV E2 protein or fragment or variant thereof is derived from HCV genotype 1a or HCV genotype 2a. In some embodiments, the HCV viral antigen comprises the amino acid sequence of SEQ ID NO:23 or 24. In some embodiments, the viral nucleoprotein and the viral antigen are derived from the same virus. In some embodiments, the viral nucleoprotein and the viral antigen are derived from different viruses, such as different viral variants, or different viral families, genera, species, or subspecies. In some embodiments, the viral nucleoprotein is derived from HCV. In some embodiments, the viral RNA is derived from HCV. In some embodiments, the viral RNA is about 10 to about 500 nucleotides long. For example, in some embodiments, the viral RNA is about any of 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 nucleotides long, or a range in between any of the nucleotide lengths. In some embodiments, the viral RNA is about 10-50, about 30-60, about 40-70, about 50-80, about 60-90, about 70-100, about 10-100, about 80-110, about 90-120, about 100-130, about 110-140, about 120-150, about 130-160, about 140-170, about 150-180, about 160-190, about 170-200, about 180-210, about 190-220, about 200-230, about 210-240, about 220-250, about 230-260, about 240-270, about 250-280, about 260-290, about 270-300, about 280-310, about 290-320, about 300-330, about 310-340, about 320-350, about 330-360, about 340-370, about 350-380, about 360-390, about 370-400, about 380-410, about 390-420, about 400-430, about 410-440, about 420-450, about 430-460, about 440-470, about 450-480, about 460-490, or about 470-500 nucleotides long. In some embodiments, the viral RNA is about 200 to about 300 nucleotides long. For example, the viral RNA is at least about any of 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 more nucleotides long.

### Nucleoprotein-antigen fusion peptides

In some embodiments, the viral nucleoprotein, as described herein, and the target antigen peptide, as described herein, are attached via an optional linker (e.g., GS linker), *e.g.*, fused. In some embodiments, the viral nucleoprotein and the viral antigen peptide are fused to create an adaptor-antigen polypeptide. In some embodiments, the viral antigen is fused to the C-terminus of the viral nucleoprotein. In some embodiments, the viral antigen is fused to the N-terminus of the viral nucleoprotein. In some embodiments, the nucleoprotein and the target antigen are derived from the same source, *e.g.,* the same virus. In some embodiments, the nucleoprotein and the target antigen are derived from different sources, e.g., different viruses, such as different subtypes of a virus, different species or genus of a virus, or different virus families.

In some embodiments, the nucleoprotein, e.g., the viral nucleoprotein, is fused to a target antigen peptide, *e.g.,* a viral target antigen, and naturally associates with a viral RNA, thereby forming a large immunogenic complex. In some embodiments, multiple copies of the adaptor-antigen are attached to the viral RNA scaffold. In some embodiments, the viral RNA also acts as an adjuvant, such that the attached adaptor-antigen peptides encase the viral RNA and thereby mask the adjuvant to prevent an innate immune response, including production of inflammatory cytokines, upon mucosal administration of the vaccine composition to an individual *(e.g.,* a human individual).

In some embodiments, the adaptor-antigen polypeptide comprises a viral antigen fused to a viral nucleoprotein that is capable of binding to a viral RNA (e.g., comprising an RNA binding domain). In some embodiments, the adaptor-antigen polypeptide comprises a nucleoprotein derived from SARS-CoV-2 fused with a SARS-CoV-2-derived antigen. In some embodiments, the adaptor-antigen polypeptide comprises a nucleoprotein derived from SARS-CoV-2 fused with an influenza-derived antigen (e.g., IAV, IBV, ICV, or IDV, for example H1N1, H3N2, H5N1, H7N9, Yamagata, or Victoria). In some embodiments, the adaptor-antigen polypeptide comprises a nucleoprotein derived from SARS-CoV-2 fused with a hepatitis C virus (HCV)-derived antigen. In some embodiments, the adaptor-antigen polypeptide comprises a nucleoprotein derived from SARS-CoV-2 fused with a respiratory syncytial virus (RSV)-derived antigen. In some embodiments, the adaptor-antigen polypeptide comprises a nucleoprotein derived from IAV fused with an influenza-derived antigen (e.g., IAV, IBV, ICV, or IDV, for example H1N1, H3N2, H5N1, H7N9, Yamagata, or Victoria). In some embodiments, the adaptor-antigen polypeptide comprises a nucleoprotein derived from IAV H5N1 fused with an IAV-derived antigen selected from the group consisting of H1N1, H3N2, H5N1, and H7N9. In some embodiments, the adaptor-antigen polypeptide comprises a nucleoprotein derived from IAV fused with the IBV-derived antigen, Yamagata or Victoria. In some embodiments, the adaptor-antigen polypeptide comprises a nucleoprotein derived from HCV fused with an HCV-derived antigen. In some embodiments, the adaptor-antigen polypeptide comprises a nucleoprotein derived from RSV fused with an RSV-derived antigen. In some embodiments, the viral antigen is a viral protein or a fragment or variant thereof. In some embodiments, the viral antigen is a viral surface glycoprotein or a fragment or variant thereof. In some embodiments, the viral antigen comprises an amino acid sequence selected from the group consisting of SEQ ID NOs: 14-24 and 31, or a variant thereof having at least about 80% *(e.g.,* at least about any of 80%, 85%, 90%, 95%, 96%, 97%, 98%, 99%, or 100%) sequence identity to an amino acid sequence selected from the group consisting of SEQ ID NOs: 14-24 and 31. In some embodiments, the viral nucleoprotein is derived from SARS-CoV-2. In some embodiments, the viral nucleoprotein is derived from IAV (e.g., IAV H5N1). In some embodiments, the viral nucleoprotein comprises an amino acid sequence of SEQ ID NO: 12 or 13, or a variant thereof having at least about 80% *(e.g.,* at least about any of 80%, 85%, 90%, 95%, 96%, 97%, 98%, 99%, or 100%) sequence identity to SEQ ID NO: 12 or 13. In some embodiments, the adaptor-antigen polypeptide comprises an amino acid sequence selected from the group consisting of SEQ ID NOs: 1-11 and 26-29, or a variant thereof having at least about 80% *(e.g.,* at least about any of 80%, 85%, 90%, 95%, 96%, 97%, 98%, 99%, or 100%) sequence identity to an amino acid sequence selected from the group consisting of SEQ ID NOs: 1-11 and 26-29.

In some embodiments, the adaptor-antigen polypeptide comprises (or consists essentially of, or consists of) a viral antigen derived from a first virus fused to a viral nucleoprotein derived from a second virus that is capable of binding to a viral RNA or DNA. In some embodiments, the viral antigen is a viral protein or a fragment or variant thereof that is derived from a first virus, e.g., SARS-CoV-2, IAV, IBV, RSV, or HCV. In some embodiments, the viral antigen is a viral surface glycoprotein or a fragment or variant thereof that is derived from a first virus. In some embodiments, the viral antigen comprises an amino acid sequence selected from the group consisting of SEQ ID NOs: 14-24 and 31, or a variant thereof having at least about 80% (*e.g.*, at least about any of 80%, 85%, 90%, 95%, 96%, 97%, 98%, 99%, or 100%) sequence identity to an amino acid sequence selected from the group consisting of SEQ ID NOs: 14-24 and 31. In some embodiments, the viral nucleoprotein (or fragment or variant thereof capable of binding to a scaffold molecule, e.g., viral RNA or DNA) is derived from a second virus, e.g., SARS-CoV-2, IAV, IBV, RSV, or HCV. The first virus and the second virus can be the same or different. In some embodiments, the adaptor-antigen polypeptide comprises a nucleoprotein derived from SARS-CoV-2 fused with an influenza A virus (IAV)-derived antigen. In some embodiments, the adaptor-antigen polypeptide comprises a nucleoprotein derived from SARS-CoV-2 fused with an influenza B virus (IBV)-derived antigen. In some embodiments, the adaptor-antigen polypeptide comprises a nucleoprotein derived from SARS-CoV-2 fused with a hepatitis C virus (HCV)-derived antigen. In some embodiments, the adaptor-antigen polypeptide comprises a nucleoprotein derived from SARS-CoV-2 fused with a respiratory syncytial virus (RSV)-derived antigen. In some embodiments, the adaptor-antigen polypeptide comprises a nucleoprotein derived from IAV fused with a SARS-CoV-2-derived antigen. In some embodiments, the adaptor-antigen polypeptide comprises a nucleoprotein derived from IAV fused with an IBV-derived antigen. In some embodiments, the adaptor-antigen polypeptide comprises a nucleoprotein derived from IAV fused with an HCV-derived antigen. In some embodiments, the adaptor-antigen polypeptide comprises a nucleoprotein derived from IAV fused with an RSV-derived antigen. In some embodiments, the adaptor-antigen polypeptide comprises a nucleoprotein derived from IBV fused with a SARS-CoV-2-derived antigen. In some embodiments, the adaptor-antigen polypeptide comprises a nucleoprotein derived from IBV fused with an IAV-derived antigen. In some embodiments, the adaptor-antigen polypeptide comprises a nucleoprotein derived from IBV fused with an HCV-derived antigen. In some embodiments, the adaptor-antigen polypeptide comprises a nucleoprotein derived from IBV fused with an RSV-derived antigen. In some embodiments, the adaptor-antigen polypeptide comprises a nucleoprotein derived from HCV fused with a SARS-CoV-2-derived antigen. In some embodiments, the adaptor-antigen polypeptide comprises a nucleoprotein derived from HCV fused with an IAV-derived antigen. In some embodiments, the adaptor-antigen polypeptide comprises a nucleoprotein derived from HCV fused with an IBV-derived antigen. In some embodiments, the adaptor-antigen polypeptide comprises a nucleoprotein derived from HCV fused with an RSV-derived antigen. In some embodiments, the adaptor-antigen polypeptide comprises a nucleoprotein derived from RSV fused with a SARS-CoV-2-derived antigen. In some embodiments, the adaptor-antigen polypeptide comprises a nucleoprotein derived from RSV fused with an IAV-derived antigen. In some embodiments, the adaptor-antigen polypeptide comprises a nucleoprotein derived from RSV fused with an IBV-derived antigen. In some embodiments, the adaptor-antigen polypeptide comprises a nucleoprotein derived from RSV fused with an HCV-derived antigen. In some embodiments, the viral nucleoprotein comprises an amino acid sequence of SEQ ID NO: 12 or 13, or a variant thereof having at least about 80% (*e.g*., at least about any of 80%, 85%, 90%, 95%, 96%, 97%, 98%, 99%, or 100%) sequence identity to SEQ ID NO:12 or 13. In some embodiments, the adaptor-antigen polypeptide comprises a viral nucleoprotein comprising the amino acid sequence of SEQ ID NO: 12 or 13, and a viral antigen comprising an amino acid sequence selected from the group consisting of SEQ ID NOs: 14-24 and 31, wherein the viral nucleoprotein and the viral antigen are not derived from the same virus. In some embodiments, the adaptor-antigen polypeptide comprises an amino acid sequence selected from the group consisting of SEQ ID NOs: 6-11 and 26-29, or a variant thereof having at least about 80% (*e.g.,* at least about any of 80%, 85%, 90%, 95%, 96%, 97%, 98%, 99%, or 100%) sequence identity to an amino acid sequence selected from the group consisting of SEQ ID NOs: 6-11 and 26-29.

### Scaffold molecules

In some embodiments, the scaffold molecule comprises a nucleic acid. In some embodiments, the scaffold molecule comprises a DNA or an RNA molecule. In some embodiments, the scaffold molecule comprises (or consists of, or consists essentially of) a DNA molecule. In some embodiments, the DNA is a single stranded DNA (ssDNA) molecule. In some embodiments, the DNA is a double stranded DNA (dsDNA) molecule. In some embodiments, the scaffold molecule comprises (or consists of, or consists essentially of) an RNA molecule, such as an ssRNA. In some embodiments, the scaffold molecule comprises a viral RNA molecule. In some embodiments, the scaffold molecule comprises a mini-viral RNA molecule. In some embodiments, the scaffold molecule comprises a non-viral RNA molecule (e.g., host RNA). When the scaffold molecules comprise a viral RNA or DNA, it can be either a wildtype or a mutant form (e.g., contains insertion(s), deletion(s), and/or substitution(s)).

Scaffold RNAs (e.g., viral RNAs) can be small RNA derived from transcription, long and complex RNA derived from large viral genomes, or long genomic RNA derived during replication. In some embodiments, the viral RNA also functions as an internal adjuvant. In some embodiments, the viral RNA, as an adjuvant, activates innate immunity within an individual, *i.e.,* once administered to an individual (*e*.*g*., a human individual). In some embodiments, the adjuvant activity that activates innate immunity within an individual *(e.g.,* a human individual) is mediated by Toll-like receptors and/or RIG-I-like receptors.

In some embodiments, the viral RNA naturally associates with a viral nucleoprotein, for example a fused adaptor-antigen protein, thereby forming a large immunogenic complex, or the nano-complex. In some embodiments, the RNA scaffold (e.g., viral RNA) acts as a scaffold to allow for the binding of multiple copies of the adaptor-antigen. Within this nano-complex, the antigen peptide can comprise any of the antigen peptides described in the present disclosure, including any viral antigen known in the art. In some embodiments, the vaccine composition comprising the nano-complexes is multivalent.

In some embodiments, the scaffold molecule comprises (or consists of, or consists essentially of) a viral RNA molecule. In some embodiments, the scaffold molecule comprises a mini-viral RNA molecule. In some embodiments, the viral RNA also functions as an internal adjuvant. In some embodiments, the viral RNA comprises an untranslated region of a viral gene of a viral genome. In some embodiments, the viral RNA comprises a 5' untranslated region or a 3' untranslated region of a viral gene of a viral genome. In some embodiments, the viral RNA comprises a 5' untranslated region of a viral gene of the viral genome. In some embodiments, the 5' untranslated region folds into a highly structured scaffold for genome replication. In some embodiments, the viral RNA comprises a 3' untranslated region of a viral gene of the viral genome.

In some embodiments, the scaffold molecule is about 10 to about 2000 nucleotides (nt) long, such as any of about 10 to about 300 nt, about 200 to about 300 nt, about 10 to about 200 nt, about 200 to about 500 nt, about 500 to about 1000 nt, about 10 to about 500 nt, about 100 to about 1000 nt, about 1000 to about 2000 nt, or about 100 to about 500 nt in length. In some embodiments, the scaffold molecule is at least about 200 nucleotides long, for example about 200 to about 500 nucleotides long, or about 200 to about 300 nucleotides long. In some embodiments, the scaffold molecule comprises a DNA or an RNA, such as any DNA or RNA molecule that can serve as a scaffold to bind to adaptor-antigen polypeptide. The scaffold molecule can be derived from any organism, including but not limited to, mammal, avian, inset, bacteria, virus, yeast, or plants. In some embodiments, the scaffold molecule comprises a viral RNA. In some embodiments, the scaffold molecule comprises a non-viral RNA, such as a host RNA (e.g., mammalian RNA, such as human RNA). In some embodiments, the scaffold molecule encodes a protein or a fragment or variant thereof. In some embodiments, the scaffold molecule does not encode a protein or a fragment or variant thereof. In some embodiments, the scaffold molecule serves as an adjuvant to enhance host immune response to the target antigen. In some embodiments, the scaffold molecule is about 10 to about 500 nt, or about 200 to about 500 nt in length. For example, in some embodiments, the scaffold molecule (e.g., viral RNA) is about any of 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 nucleotides long, or a range in between any of the nucleotide lengths. In some embodiments, the scaffold molecule (e.g., viral RNA) is about 10-50, about 30-60, about 40-70, about 50-80, about 60-90, about 70-100, about 80-110, about 90-120, about 10-100, about 100-130, about 110-140, about 120-150, about 130-160, about 140-170, about 150-180, about 160-190, about 170-200, about 180-210, about 190-220, about 200-230, about 210-240, about 220-250, about 230-260, about 240-270, about 250-280, about 260-290, about 270-300, about 280-310, about 290-320, about 300-330, about 310-340, about 320-350, about 330-360, about 340-370, about 350-380, about 360-390, about 370-400, about 380-410, about 390-420, about 400-430, about 410-440, about 420-450, about 430-460, about 440-470, about 450-480, about 460-490, or about 470-500 nucleotides long. In some embodiments, the scaffold molecule (e.g., viral RNA) is at least about 200 nucleotides long. In some embodiments, the scaffold molecule (e.g., viral RNA) is about 200 to about 500 nucleotides long. For example, the scaffold molecule (e.g., viral RNA) is at least about any of 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 nucleotides long.

In some embodiments, the scaffold molecule (e.g., viral RNA) comprises an untranslated region of a viral genome. In some embodiments, the viral RNA comprises a 5' untranslated region or a 3' untranslated region of a viral gene of the viral genome. In some embodiments, the viral RNA comprises a 5' untranslated region of a viral gene of the viral genome. In some embodiments, the viral RNA is derived from SARS-CoV-2. In some embodiments, the viral RNA comprises nucleotides 1-266 of a viral gene of the SARS-CoV-2 genome (e.g., SEQ ID NO: 25). In some embodiments, the viral RNA is derived from IAV. In some embodiments, the viral RNA comprises nucleotides 1-326 of a viral gene of the IAV genome (e.g., SEQ ID NO: 30).

In some embodiments, the nano-complexes in the vaccine composition have an average particle size of about 50nm to about 200nm. For example, in some embodiments, the nano-complexes in the vaccine composition have an average particle size of about any of 50nm, 60nm, 70nm, 80nm, 90nm, 100nm, 110nm, 120nm, 130nm, 140nm, or 150nm. In some embodiments, the nano-complexes in the vaccine composition have an average particle size of about 50nm-80nm, about 60nm-90nm, about 70nm-100nm, about 80nm-110nm, about 90nm-120nm, about 100nm-130nm, about 110nm-140nm, about 120nm-150nm, about 130nm-160nm, about 140nm-170nm, about 150nm-180nm, about 160nm-190nm, or about 170nm-200nm. In some embodiments, the nano-complexes in the vaccine composition have an average particle size of about 100nm to about 130nm. In some embodiments, the particle size (e.g., average particle size) of the nano-complexes in the vaccine composition can be measured by any of SDS-PAGE, native-PAGE, high performance size exclusion chromatography (HP-SEC), sedimentation velocity analytical ultracentrifugation (SV-AUC), dynamic light scattering (DLS), flow field-flow fractionation, nanoparticle tracking analysis (NTA), resonant mass measurement (RMM), light obscuration, flow imaging microscopy, visual inspection, *etc.* In some embodiments, HP-SEC or flow field-flow fractionation can be combined with serial online detectors, such as UV/VIS absorbance, static light scattering, fluorescence, or refractive index.

In some embodiments, the nano-complexes in the vaccine composition have a zeta-potential of about 0mV to about -50mV. For example, in some embodiments, the nano-complexes in the vaccine composition have a zeta-potential of about 0mV, about -10mV, about - 20mV, about -30mV, about -40mV, or about -50mV. In some embodiments, the nano-complexes in the vaccine composition have a zeta-potential of about 0mV to about -10mV, about -5mV to about -15mV, about -10mV to about -20mV, about -15mV to about -25mV, about -20mV to about -30mV, about -25mV to about -35mV, about -30mV to about -40mV, about -35mV to about -45mV, or about -40mV to about -50mV. In some embodiments, the nano-complexes in the vaccine composition have a zeta-potential of about -10mV to about -20mV.

Thus, in some embodiments, there is provided a vaccine composition comprising nano-complexes comprising: i) a scaffold molecule comprising a viral RNA and ii) an adaptor-antigen polypeptide comprising a viral antigen comprising an amino acid sequence selected from the group consisting of SEQ ID NOs: 14-24 and 31 fused to a viral nucleoprotein capable of binding to the viral RNA. In some embodiments, the molar ratio of the adaptor-antigen polypeptide to the scaffold molecule is at least about 5:1, such as about 6:1 to about 12:1, about 6:1 to about 10:1, about 8:1 to about 12:1, or about 6:1 to about 8:1. For example, in some embodiments, the molar ratio of the adaptor-antigen polypeptide to the scaffold molecule (e.g., viral RNA) is about any of 5:1, 6:1, 7:1, 8:1, 9:1, 10:1, 11:1, or 12:1, or ranges in between any of the ratios. In some embodiments, the viral antigen is fused to the C-terminus of the viral nucleoprotein via an optional linker (e.g., GS linker). In some embodiments, the viral antigen is a viral protein or a fragment or variant thereof. In some embodiments, the viral antigen is fused to the N-terminus of the viral nucleoprotein via an optional linker (e.g., GS linker). In some embodiments, the viral nucleoprotein and the viral antigen are derived from the same virus. In some embodiments, the viral nucleoprotein and the viral antigen are derived from different viruses, such as different viral variants, or different viral families, genera, species, or subspecies. In some embodiments, the viral nucleoprotein is derived from SARS-CoV-2 or IAV. In some embodiments, the viral nucleoprotein comprises an amino acid sequence of SEQ ID NO: 12 or 13. In some embodiments, the adaptor-antigen polypeptide comprises an amino acid sequence selected from the group consisting of SEQ ID NOs: 1-11 and 26-29. In some embodiments, the viral RNA comprises an untranslated region of a viral genome. In some embodiments, the viral RNA comprises a 5' untranslated region or a 3' untranslated region of a viral gene of the viral genome (e.g., 5' or 3' UTR of a viral gene). In some embodiments, the viral RNA comprises a 5' untranslated region of a viral gene of the viral genome (e.g., 5' UTR of a viral gene). In some embodiments, the viral RNA is about 10 to about 500 nucleotides long. For example, in some embodiments, the viral RNA is about any of 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 nucleotides long. In some embodiments, the viral RNA is about 20-50, about 30-60, about 40-70, about 50-80, about 60-90, about 70-100, about 80-110, about 90-120, about 100-130, about 110-140, about 120-150, about 130-160, about 140-170, about 150-180, about 160-190, about 170-200, about 180-210, about 190-220, about 200-230, about 210-240, about 220-250, about 230-260, about 240-270, about 250-280, about 260-290, about 270-300, about 280-310, about 290-320, about 300-330, about 310-340, about 320-350, about 330-360, about 340-370, about 350-380, about 360-390, about 370-400, about 380-410, about 390-420, about 400-430, about 410-440, about 420-450, about 430-460, about 440-470, about 450-480, about 460-490, or about 470-500 nucleotides long. In some embodiments, the viral RNA is about 200 to about 500 nucleotides long. For example, the viral RNA is at least about any of 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 nucleotides long. In some embodiments, the viral RNA is derived from SARS-CoV-2. In some embodiments, the viral RNA comprises nucleotides 1-266 of a viral gene of the SARS-CoV-2 genome as set forth in SEQ ID NO:25. In some embodiments, the viral RNA is derived from IAV. In some embodiments, the viral RNA is derived from IAV H7N9. In some embodiments, the viral RNA comprises nucleotides 1-326 of a viral gene of the IAV H7N9 genome (e.g., as set forth in SEQ ID NO:30) or a modified genome thereof. In some embodiments, the nano-complexes in the vaccine composition have an average particle size of about 100nm to about 130nm. In some embodiments, the nano-complexes in the vaccine composition have a uniform size. In some embodiments, the nano-complexes in the vaccine composition have a zeta-potential of about -10mV to about -20mV.

### III. HOST CELLS FOR PRODUCTION OF NANO-COMPLEX VACCINES

Also provided herein are host cells for producing the adaptor-antigen polypeptide and/or the nucleic acid scaffold (e.g., viral RNA) components of the vaccine compositions described herein.

Suitable host cells can include, without limitation, higher eukaryotic cells such as mammalian cells. Suitable higher eukaryotic cells include, without limitation, invertebrate cells and insect cells, and vertebrate cells.

The host cell can be transfected as part of the standard methods of genome editing using any suitable methods known in the art, including, but not limited to, DEAE-dextran mediated delivery, calcium phosphate precipitate method, cationic lipids mediated delivery, liposome mediated transfection, electroporation, lentiviral transduction, microprojectile bombardment, receptor- mediated gene delivery, delivery mediated by polylysine, histone, chitosan, and peptides. Standard methods for transfection and transformation of cells for expression of a vector of interest are well known in the art.

Any suitable host cells can be used here. Host cells can include but are not limited to, BHK21 cells, VeroE6 cells, L929 cells, CHO cells, BHK cells, MDCK cells, C3H 10T1/2 cells, FLY cells, Psi-2 cells, BOSC 23 cells, PA317 cells, WEHI cells, COS cells, BSC 1 cells, BSC 40 cells, BMT 10 cells, VERO cells, W138 cells, MRC5 cells, A549 cells, HT1080 cells, 293 cells, 293T cells, B-50 cells, 3T3 cells, NIH3T3 cells, HepG2 cells, Saos-2 cells, Huh7 cells, HeLa cells, W163 cells, 211 cells, NS0 cells, PerC6 cells, Sp2/0 cells, BHK cells, C127 cells, 211 A cells, and any host cells derived from any of these cells. In some embodiments, the suitable host cell is genetically modified to express a component of a vaccine composition described herein (*e.g.,* any one or more of SEQ ID NOs: 1-11 and 26-29 and/or an RNA scaffold molecule, e.g., either of SEQ ID NO:25 or 30).

### III. METHODS OF PREPARATION

The vaccine composition described herein may be prepared by any of the known nucleic acid expression, protein production, and *in situ* synthesis methods in the art. The adaptor-antigen polypeptides can be expressed and produced in a host cell, including any host cell described above. The adaptor-antigen polypeptides can then be harvested from the host cell or the culture media (e.g., upon secretion of the adaptor-antigen polypeptides from the host cell). The nucleic acid scaffold molecule (e.g., viral RNA) can be expressed within a host cell, *in vitro* transcribed, or can be *in vitro* synthesized fully. For example, the RNA scaffold molecule (e.g., viral RNA) can be *in vitro* transcribed using SP6 or T7 polymerase, chemically synthesized, or produced within a host cell (e.g., transcribed from a DNA template such as vector).

In some embodiments, the nucleic acid(s) encoding the viral nucleoprotein, viral antigen, and/or nucleic acid scaffold (e.g., viral RNA) components is inserted into a vector, such as an expression vector, a viral vector, or a cloning vector. Hence also provided are vectors encoding any of the viral nucleoprotein, viral antigen, and/or nucleic acid scaffold (e.g., viral RNA) components described herein. For expression of the viral nucleoprotein, viral antigen, and/or nucleic acid scaffold (e.g., viral RNA) components, the vector may be introduced into a host cell to allow expression of the nucleic acids encoding the viral nucleoprotein, viral antigen, and/or nucleic acid scaffold (e.g., viral RNA) components within the host cell. The expression vectors may contain a variety of elements for controlling expression, including without limitation, promoter sequences, transcription initiation sequences, enhancer sequences, selectable markers, and signal sequences. These elements may be selected as appropriate by a person of ordinary skill in the art. For example, the promoter sequences may be selected to promote the transcription of the nucleic acid in the vector. Suitable promoter sequences include, without limitation, T7 promoter, T3 promoter, SP6 promoter, beta-actin promoter. EF1a promoter, CMV promoter, and SV40 promoter. Enhancer sequences may be selected to enhance the transcription of the nucleic acids. Selectable markers may be selected to allow selection of the host cells inserted with the vector from those not, for example, the selectable markers may be genes that confer antibiotic resistance. Signal sequences may be selected to allow the expressed viral nucleoprotein, viral antigen, and/or nucleic acid scaffold (e.g., viral RNA) components to be transported outside of the host cell.

Suitable vectors include, but are not limited to, nucleic acid molecules that are single-stranded, double-stranded, or partially double-stranded; nucleic acid molecules that comprise one or more free ends, no free ends (e.g., circular); nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the transcription or expression of coding nucleotide sequences to which they are operatively linked. Such vectors are referred to herein as "expression vectors".

In some embodiments, the expression vector encoding the viral nucleoprotein, viral antigen, and/or nucleic acid scaffold (e.g., viral RNA) components is selected from the group consisting of a viral vector, a bacterial vector, and a mammalian cell vector. Viral vector technology is well known in the art and is described, for example, in Sambrook et al. (2012), and in Ausubel et al. (1999), and in other virology and molecular biology manuals. Viruses, which are useful as vectors include, but are not limited to, retroviruses, adenoviruses, adeno-associated viruses (AAV), herpes viruses, and lentiviruses. In some embodiments, a murine stem cell virus (MSCV) vector is used to express a desired nucleic acid. MSCV vectors have been demonstrated to efficiently express desired nucleic acids in cells. Other examples of viral vectors are those based upon Moloney Murine Leukemia Virus (MoMuLV) and human immunodeficiency virus (HIV). In some embodiments, a suitable vector contains an origin of replication functional in at least one organism, a promoter sequence, convenient restriction endonuclease sites, and one or more selectable markers. (See, e.g., WO 01/96584; WO 01/29058; and U.S. Pat. No. 6,326,193). In some embodiments, the vector is a recombinant adeno-associated virus (rAAV) vector. In some embodiments, the rAAV vector is a vector derived from an AAV serotype, including without limitation, AAV ITRs are AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV capsid serotype or the like. In some embodiments, the vector is encapsulated in a rAAV particle. In some embodiments, the vector is a lentiviral vector.

In some embodiments, the method of making the vaccine compositions described herein further comprise transfecting a host cell with a nucleic acid or a vector encoding the viral nucleoprotein, viral antigen, and/or nucleic acid scaffold (e.g., viral RNA) components. In some embodiments, the nucleic acid(s) or vector(s) encoding the viral nucleoprotein, viral antigen, and/or nucleic acid scaffold (e.g., viral RNA) components are transfected into said host cell simultaneously. In some embodiments, the nucleic acid(s) or vector(s) encoding the viral nucleoprotein, viral antigen, and/or nucleic acid scaffold (e.g., viral RNA) components are transfected into said host cell sequentially. The nucleic acids described herein or the vectors (e.g., viral vectors) can be delivered directly into a host cell or an individual (e.g., human) for the purpose of gene therapy. Methods of non-viral delivery of nucleic acids include lipofection, nucleofection, microinjection, biolistics, virosomes, liposomes, immunoliposomes, polycation or lipid: nucleic acid conjugates, electroporation, nanoparticles, exosomes, microvesicles, or gene-gun, naked DNA, and artificial virions. The use of RNA or DNA viral based systems for the delivery of nucleic acids has high efficiency in targeting a virus to specific cells and trafficking the viral payload to the cellular nuclei.

The adaptor-antigen polypeptide (or nucleic acid encoding thereof) or the nucleic acid scaffold (e.g., viral RNA) components of the vaccine compositions described herein can be introduced to the host cell using any suitable methods known in the art, including, but not limited to, DEAE-dextran mediated delivery, calcium phosphate precipitate method, cationic lipids mediated delivery, liposome mediated transfection, electroporation, lentiviral transduction, microprojectile bombardment, receptor-mediated gene delivery, delivery mediated by polylysine, histone, chitosan, and peptides. Standard methods for transfection and transformation of cells are well known in the art.

In some embodiments, the present application provides methods of making the adaptor-antigen polypeptide (or single component of adaptor or antigen) and/or the nucleic acid scaffold (e.g., viral RNA) component of the vaccine compositions described herein, comprising culturing an isolated host cell comprising the adaptor-antigen polypeptide (or single component of adaptor or antigen) and/or the nucleic acid scaffold (e.g., viral RNA) component under a condition suitable for the expression of the component, and obtaining the expressed component from said host cell *(e.g.,* from the cell culture). Suitable conditions for expression may include, without limitation, suitable medium, suitable density of host cells in the culture medium, presence of necessary nutrients, presence of supplemental factors, suitable temperatures and humidity, and absence of microorganism contaminants. A person with ordinary skill in the art can select the suitable conditions as appropriate for the purpose of the expression.

After harvesting the adaptor-antigen polypeptide (or single component of adaptor or antigen) from the host cell and either synthesizing (e.g., *in vitro* transcribed) or harvesting from a host cell the nucleic acid scaffold (e.g., viral RNA), these molecules can then be combined to form the nano-complexes described herein. The adaptor-antigen polypeptide naturally binds to the nucleic acid scaffold molecule (e.g., viral RNA), as described herein, wherein multiple adaptor-antigen polypeptides can bind to the same nucleic acid scaffold molecule, as described herein. The synthesized nano-complexes are then isolated and prepared by standard methods (e.g., purification) for administration to an individual (e.g., human), as described herein.

In some embodiments, the method of making the vaccine compositions described herein further comprise purifying and/or enriching the adaptor, antigen, and/or nucleic acid scaffold molecule (e.g., viral RNA) components. For example, viral RNAs can be purified by phenol-chloroform-isopropanol method. Any protein purification methods known in the art (e.g., ion-exchange, filtration, affinity purification, mixed-mode purification) can be used herein for protein component purification. In some embodiments, the purity of the adaptor, antigen, and/or nucleic scaffold molecule is at least about 90%, such as at least about any of 91%, 92%, 93%, 94%, 95%, 96%, 97%, 98%, 99%, or 100%.

In some embodiments, the purified adaptor, antigen, and nucleic acid scaffold molecule (e.g., viral RNA) components are mixed in a buffer, incubated for a period of time (e.g., at least about 5 minutes) and under a suitable condition (e.g., a temperature suitable for protein/nucleic acid binding), to form the nano-complexes described herein. In some embodiments, the purified adaptor and antigen are mixed in a buffer, incubated for a period of time (e.g., at least about 5 minutes) and under a suitable condition (e.g., a temperature suitable for protein/protein binding), to form the adaptor-antigen polypeptides described herein; then the adaptor-antigen polypeptides and the nucleic acid scaffold molecule (e.g., viral RNA) components are mixed in a buffer, incubated for a period of time (e.g., at least about 5 minutes) and under a suitable condition (e.g., a temperature suitable for protein/nucleic acid binding), to form the nano-complexes described herein.

### IV. PHARMACEUTICAL COMPOSITIONS, UNIT DOSAGES, ARTICLES OF MANUFACTURE, AND KITS

Further provided by the present application are pharmaceutical compositions comprising any one of the vaccine compositions comprising nano-complexes described herein, and optionally a pharmaceutically acceptable carrier.

The pharmaceutical compositions may be suitable for a variety of modes of administration described herein, including for example systemic or localized administration. Exemplary routes of administration of any of the vaccine compositions described herein (or pharmaceutical composition thereof) include, but are not limited to, oral, intravenous, intracavitary, intratumoral, intraarterial, intramuscular, subcutaneous, parenteral, transmucosal, transdermal, ocular, topical, intraperitoneal, intracranial, intrapleural, and epidermal routes, or be delivered into lymph glands, body spaces, organs, or tissues known to house virally infected cells. In some embodiments, the pharmaceutical composition is formulated for mucosal administration. In some embodiments, the pharmaceutical composition is formulated for administration by nasal spray. In some embodiments, the pharmaceutical composition is formulated for administration by nasal drops. In some embodiments, the pharmaceutical composition is administered parenterally, such as by intramuscular or intradermal administration. In some embodiments, the pharmaceutical composition is administered as a single dose. In some embodiments, the pharmaceutical composition is administered as multiple doses. In some embodiments, the individual (such as a human) is any age. In some embodiments, the individual (such as a human) is 65 years of age or older, for example any of 65, 70, 75, 80, 85, 86, 87, 88, 89, 90 years or older. In some embodiments, the individual (such as a human) is 18 years of age or younger, for example any of 18, 17, 16, 15, 14, 13, 12, 11, 10, 9, 8, 7, 6, 5, 4, 3, 2 years or less. In some embodiments, the individual (such as a human) is an infant 1 year of age or younger (e.g., less than about any of 12, 11, 10, 9, 8, 7, 6, 5, 4, 3, 2, 1 month or younger). In some embodiments, the individual (such as a human) is healthy. In some embodiments, the individual (such as a human) has a medical condition, a pre-existing condition, or a condition that reduces heart, lung, brain, or immune system function. In some embodiments, the individual (such as a human) is immunocompromised.

Micro- and nanocarrier-based delivery systems as nasal vaccines induce humoral, cellular, and mucosal immunity. The nasal route of vaccination could also offer immunity at several distant mucosal sites *(e.g.,* oral, rectal, vaginal, and pulmonary). Nasal vaccine delivery blocks pathogen entry at the mucosal site by inducing local immunity, displays increased bioavailability to other administration routes, demonstrates swift uptake to the blood circulatory system via mucosal absorption, and encourages better patient compliance that other administration routes, *e.g.,* parenteral (see, *e.g.,* Ramvikas et al. (2016) Micro and Nanotechnology in Vaccine Development 2017: 279-301, hereby incorporated by reference in its entirety). Nasal vaccine delivery formulations can include, but are not limited to, form of microparticulates, nanoparticulates, and liposomes.

In some cases, a subject method involves administering to an individual in need thereof an effective amount of a vaccine composition comprising nano-complexes (or pharmaceutical composition thereof). In some embodiments, an "effective amount" of a subject vaccine is an amount that, when administered to an individual in one or more doses, in monotherapy or in combination therapy, is effective to prevent a viral infection or transmission or to reduce symptoms of a viral infection in the individual by at least about 10%, at least about 20%, at least about 25%, at least about 30%, at least about 40%, at least about 50%, at least about 60%, at least about 70%, at least about 80%, at least about 90%, at least about 95%, at least about 96%, at least about 97%, at least about 98%, at least about 99%, or 100%, compared to the individual in the absence of administration with the vaccine.

"Carriers" as used herein include pharmaceutically acceptable carriers, excipients, or stabilizers which are nontoxic to the cell or individual being exposed thereto at the dosages and concentrations employed. Often the physiologically acceptable carrier is an aqueous pH buffered solution. Acceptable carriers, excipients, or stabilizers are compatible with nucleic acid constructs (i.e., RNA and/or DNA), and solutions comprising said carriers, excipients, or stabilizers may further include one or more inhibitors of RNase and/or DNase activity. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN^{™}, PLURONICS^{™} or polyethylene glycol (PEG).

In some embodiments, the pharmaceutical composition is formulated to have a pH in the range of about 4.5 to about 9.0, including for example pH ranges of about any one of 5.0 to about 8.0, about 6.5 to about 7.5, or about 6.5 to about 7.0. In some embodiments, the pharmaceutical composition is formulated to have a pH in the range of about 4.5 to about 6.5. In some embodiments, the pharmaceutical composition can also be made to be isotonic with blood by the addition of a suitable tonicity modifier.

The pharmaceutical compositions to be used for *in vivo* administration are generally formulated as sterile, substantially isotonic, and in full compliance with all Good Manufacturing Practice (GMP) regulations of the U.S. Food and Drug Administration. Sterility is readily accomplished by filtration through sterile filtration membranes. In some embodiments, the composition is free of pathogens. For parenteral administration, the pharmaceutical composition can be in the form of liquid solutions, for example in physiologically compatible buffers such as Hank's solution or Ringer's solution. In addition, the pharmaceutical composition can be in a solid form and re-dissolved or suspended, e.g., in water, immediately prior to use. Lyophilized compositions are also included.

An aerosol formulation suitable for administration via inhalation also can be made. The aerosol formulation can be placed into a pressurized acceptable propellant, such as dichlorodifluoromethane, propane, nitrogen, and the like.

In some embodiments, the pharmaceutical composition is formulated in accordance with routine procedures as a pharmaceutical composition adapted for injection intravenously, intraperitoneally, subcutaneously, intramuscularly, or intravitreally. In some embodiments, a subject delivery system comprises a device for delivery to nasal passages or lungs. For example, the compositions described herein can be formulated for delivery by a nebulizer, an inhaler device, or the like.

In some embodiments, the pharmaceutical composition is formulated in accordance with routine procedures as a pharmaceutical composition adapted for mucosal administration, such as intranasal administration. Intranasal administration can be achieved via nasal powders, nasal drops, nasal aerosols, nasal gels, *etc.* In some embodiments, the pharmaceutical composition is formulated in accordance with routine procedures as a pharmaceutical composition adapted for injection intravenously, intraperitoneally, subcutaneously, intramuscularly, or intravitreally. Typically, compositions for injection are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered, it can be reconstituted as needed accordingly.

In some embodiments, the pharmaceutical composition is suitable for administration to a human. In some embodiments, the pharmaceutical composition is suitable for administration to a rodent (*e.g.,* mice, rats), non-human primates (*e.g., Cynomolgus* monkey), simians, felines, canines, equines, bovines, porcines, ovines, caprines, mammalian laboratory animals, mammalian farm animals, mammalian sport animals, and mammalian pets. In some embodiments, the pharmaceutical composition is contained in a single-use vial, such as a single-use sealed vial. In some embodiments, the pharmaceutical composition is contained in a multi-use vial. In some embodiments, the pharmaceutical composition is contained in bulk in a container. In some embodiments, the pharmaceutical composition is cryopreserved.

The term "unit dosage form" refers to a physically discrete unit suitable as unitary dosages for an individual, each unit containing a predetermined quantity of active material calculated to produce the desired therapeutic effect, in association with a suitable pharmaceutical carrier, diluent, or excipient. These unit dosage forms can be stored in a suitable packaging in single or multiple unit dosages and may also be further sterilized and sealed.

The present application further provides articles of manufacture comprising the compositions (such as pharmaceutical compositions) described herein in suitable packaging. Suitable packaging for compositions (such as pharmaceutical compositions) described herein are known in the art, and include, for example, vials (such as sealed vials), nasal spray or nasal drop bottles, vessels, ampules, bottles, jars, flexible packaging (*e*.*g*., sealed Mylar or plastic bags), and the like. These articles of manufacture may further be sterilized and/or sealed.

The present application also provides kits comprising compositions (such as pharmaceutical compositions) described herein and may further comprise instruction(s) on methods of using the composition, such as uses described herein. The kits described herein may further include other materials desirable from a commercial and user standpoint, including other buffers, diluents, filters, spray bottles, nebulizers, inhaler devices, needles, syringes, and package inserts with instructions for performing any methods described herein.

### V. METHODS OF VACCINATING

One aspect of the present application provides a method of preventing (*e*.*g*., vaccinating) or treating viral infection (*e*.*g*., infection from a SARS-CoV-2 virus, an influenza virus, a hepatitis virus, or a respiratory syncytial virus) in an individual (such as a human), comprising administering to the individual an effective amount of any of the vaccine compositions or pharmaceutical compositions thereof described herein. In some embodiments, the vaccine composition is administered by intranasal or intradermal administration. In some embodiments, the vaccine composition is administered with an initial dose, followed by one or more (e.g., two, three, four, five, six, seven, eight, nine, ten, or more) booster doses. In some embodiments, the method for preventing (e.g., vaccinating) or treating a viral infection (e.g., infection from a SARS-CoV-2 virus, an influenza virus, a hepatitis virus, or a respiratory syncytial virus) further prevents or treats an infection from one or more additional virus species from a different virus family (e.g., provides multivalent immunity) and/or one or more viral strains or subtypes of a virus (e.g., provides heterosubtypic immunity) in an individual. In some embodiments, the method induces multivalent immunization against two or more virus species (e.g., two or more of SARS-CoV-2, IAV, IBV, RSV, and HCV). In some embodiments, the method induces heterosubtypic immunization against two or more viral subtypes (*e*.*g*., two or more IAV subtypes selected from H1N1, H5N1, H3N2, and H7N9; IBV subtypes Yamagata and Victoria; two or more SARS-CoV-2 strains; two or more HCV genotypes or subtypes; or two or more RSV strains). In some embodiments, the method for preventing (e.g., vaccinating) or treating a viral infection (e.g., infection from a SARS-CoV-2 virus, an influenza virus, a hepatitis virus, or a respiratory syncytial virus) further comprises strengthening the individual's immunity against said viral infection. In some embodiments, the method for preventing (e.g., vaccinating) or treating a viral infection (e.g., infection from a SARS-CoV-2 virus, an influenza virus, a hepatitis virus, or a respiratory syncytial virus) further comprises reducing the risk of contracting an infection by a variant of said viral infection (e.g., a variant strain of SARS-CoV2, a subtype or variant of IAV, a subtype or variant of IBV, a subtype or variant of RSV, and/or a subtype or variant of HCV).

In some embodiments, the method of vaccinating an individual comprises a method of prophylactically immunizing an individual against a viral infection (*e*.*g*., infection from a SARS-CoV-2 virus, an influenza virus, a hepatitis virus, or a respiratory syncytial virus). In some embodiments, the method of vaccinating an individual comprises a method of preventing an individual from contracting a viral infection (*e*.*g*., infection from a SARS-CoV-2 virus, an influenza virus, a hepatitis virus, or a respiratory syncytial virus). In some embodiments, the method of vaccinating an individual comprises a method of preventing viral transmission from a vaccinated individual to an unvaccinated individual. In some embodiments, the method of vaccinating or treating an individual comprises a method of reducing the severity of a viral infection (e.g., infection from a SARS-CoV-2 virus, an influenza virus, a hepatitis virus, or a respiratory syncytial virus) in an individual. In some embodiments, the method of vaccinating an individual comprises a method of strengthening immunity in an individual against a viral infection (e.g., infection from a SARS-CoV-2 virus, an influenza virus, a hepatitis virus, or a respiratory syncytial virus). In some embodiments, the method of vaccinating an individual comprises a method of reducing the risk of an individual contracting a viral infection (e.g., infection from a SARS-CoV-2 virus, an influenza virus, a hepatitis virus, or a respiratory syncytial virus). In some embodiments, the method of vaccinating or treating an individual further comprises a method of treating an individual having a viral infection (e.g., infection from a SARS-CoV-2 virus, an influenza virus, a hepatitis virus, or a respiratory syncytial virus). In some embodiments, the method of vaccinating or treating an individual comprises a method of eliciting an immune response in an individual against a viral infection (e.g., infection from a SARS-CoV-2 virus, an influenza virus, a hepatitis virus, or a respiratory syncytial virus). In some embodiments, the method of vaccinating or treating an individual comprises a method of enhancing the T cell response in an individual to a viral infection (e.g., infection from a SARS-CoV-2 virus, an influenza virus, a hepatitis virus, or a respiratory syncytial virus). In some embodiments, the method of eliciting an immune response, such as enhancing the T cell response, in an individual further comprises a method of activating CD4+ T cells against a viral infection (e.g., infection from a SARS-CoV-2 virus, an influenza virus, a hepatitis virus, or a respiratory syncytial virus). In some embodiments, the method of vaccinating or treating an individual comprises administering the vaccine composition comprising nano-complexes described herein, wherein the vaccine provides multivalent protection against different viral species within the same family or different families. In some embodiments, the method of vaccinating or treating an individual comprises administering the vaccine composition comprising nano-complexes described herein, wherein the vaccine further provides heterosubtypic protection against different viral species within the same family or against different viral strains, subtypes, or subspecies. In some embodiments, the individual is a mammal, for example, a human, bovine, horse, feline, canine, rodent, or primate. In some embodiments, the individual is a human. In some embodiments, the individual is at risk of contracting or has contracted a viral infection. In some embodiments, the individual is at elevated risk of mortality upon contraction of a viral infection. In some embodiments, the individual (such as a human) is any age. In some embodiments, the individual (such as a human) is 65 years of age or older, for example any of 65, 70, 75, 80, 85, 86, 87, 88, 89, 90 years or older. In some embodiments, the individual (such as a human) is 18 years of age or younger, for example any of 18, 17, 16, 15, 14, 13, 12, 11, 10, 9, 8, 7, 6, 5, 4, 3, 2 years or less. In some embodiments, the individual (such as a human) is 6 months of age or younger, for example any of 6, 5, 4, 3, 2, 1 months or newborn. In some embodiments, the individual (such as a human) is healthy. In some embodiments, the individual (such as a human) has a medical condition, a pre-existing condition, or a condition that reduces heart, lung, brain, or immune system function. In some embodiments, the individual (such as a human) is immunocompromised. In some embodiments, the individual has a compromised adaptive immune system and/or a compromised innate immune system. In some embodiments, the individual has a weakened immune system. In some embodiments, the individual has a weakened adaptive immune system and/or a weakened innate immune system. In some embodiments, the individual is administered one or more courses of immunosuppressants. In some embodiments, the individual had been administered one or more courses of immunosuppressants.

Symptoms of viral infection can present differently based upon the virus and the tissue or organ infected in the individual. Often, some symptoms overlap, for example fatigue, malaise, headache, or in more severe cases, sepsis, organ failure, elevated levels of inflammatory cytokines, and possibly death. Further examples are provided below.

In some embodiments, symptoms of a sarbecovirus infection, *e.g.,* a SARS-CoV-2 infection, include any one of fever, cough, sore throat, nasal congestion, malaise, headache, muscle pain, shortness of breath, mild pneumonia, severe pneumonia, acute pneumonia, sepsis, septic shock, or any combination thereof. Signs of infection include altered mental status, difficult or fast breathing, low oxygen saturation, reduced urine output, fast heart rate, weak pulse, cold extremities or low blood pressure, skin mottling, or laboratory evidence of coagulopathy, thrombocytopenia, acidosis, high lactate, or hyperbilirubinemia. In some embodiments, sarbecovirus infection, e.g., SARS-CoV-2 infection, significantly elevates levels of inflammatory cytokines in an infected individual. In some embodiments, elevated levels of inflammatory cytokines, *i.e.,* cytokine storm, triggers excessive, uncontrolled systemic inflammation. In some instances, uncontrolled systemic inflammation leads to pneumonitis, respiratory failure, shock, organ failure, secondary bacterial pneumonia, and potentially death in an individual infected with a sarbecovirus.

In some embodiments, symptoms of an influenza infection, e.g., H3N2, include any one of fever, chills, cough, sore throat, nasal congestion, malaise, headache, muscle pain, vomiting, diarrhea, shortness of breath, mild pneumonia, severe pneumonia, acute pneumonia, or any combination thereof. Signs of severe influenza infection include pneumonitis, myocarditis, encephalitis, myositis, rhabdomyolysis, multi-organ failure, extreme inflammatory response, sepsis, secondary bacterial pneumonia, and potentially death in an individual infected with an influenza virus.

In some embodiments, symptoms of a hepatitis virus, e.g., Hepatitis C virus (HCV), include any one of jaundice, fatigue, depression, abdominal pain, ascites, clay-colored or pale stool, dark urine, fever, itching, loss of appetite, nausea, vomiting, cirrhosis, liver cancer, or any combination thereof. In some instances, an individual with a hepatitis virus presents with no symptoms or mild symptoms. In some instances, an individual with a hepatitis virus develops symptoms after developing hepatitis-induced cirrhosis or liver cancer.

In some embodiments, symptoms of respiratory syncytial virus (RSV) include any one of nasal congestion, decreased appetite, dehydration, cough, sneezing, fever, shortness of breath or wheezing, difficulty breathing, irritability, decreased activity, malaise, fatigue, pain, or any combination thereof. In more severe instances, RSV symptoms may further include bronchiolitis and/or pneumonia, and potentially death in an individual infected with a respiratory syncytial virus. Individuals at highest risk include older adults *(e.g.,* 65 years of age or older) and infants younger than 6 months of age.

In some embodiments, the severity of a viral infection in an individual is reduced following vaccination or treatment with any one of the vaccine compositions described herein, for example reduced by at least about any of 25%, 30%, 35%, 40%, 45%, 50%, 55%, 60%, 65%, 70%, 75%, 80%, 85%, 90%, 95%, or more compared to the severity of the same viral infection in an individual who did not receive vaccination or treatment with any one of the vaccine compositions described herein. In some embodiments, an immune response in an individual is elicited following vaccination or treatment with any one of the vaccine compositions described herein. In some embodiments the elicited immune response comprises a T cell response, such as T cell activation, in an individual. In some embodiments, the enhanced T cell response in an individual further comprises a method of activating CD4+ T cells. In some embodiments, progression of a viral infection in an individual is delayed by vaccination or treatment with any one of the vaccine compositions described herein. In some embodiments, mortality caused by a viral infection in an individual is prevented by vaccination or treatment with any one of the vaccine compositions described herein. In some embodiments, an individual vaccinated or treated with any one of the vaccine compositions described herein who becomes or already is infected with a viral infection is prevented from transmitting the viral infection to an unvaccinated individual.

In some embodiments, vaccination or treatment with any one of the vaccine compositions described herein may induce heterosubtypic immunization against more than one viral subvariant or subtype, such as against two or more viral subvariants or subtypes. In some embodiments, the virus is IAV and the more than one subvariant or subtype is selected from the group consisting of H1N1, H5N1, H3N2, and H7N9. In some embodiments, the virus is IBV and the more than one subvariant or subtype is Yamagata and Victoria. In some embodiments, the virus is SARS-CoV-2 and the more than one subvariant or subtype is selected from the group consisting of a B.1.1.7 variant, a B.1.351 variant, a B.1.526 variant, a B1.526.1 variant, a B1.617 variant, a B.1.617.1 variant, a B.1.617.2 variant, a B1.617.3 variant, a P.2 variant, a P.1 (also known as B.1.1.28.1) variant, an A.23.1 variant, a CAL.20C variant, a B.1.427 variant, a B.1.429 variant, a B.1.525 variant, a BA.2, BA.5 variant, a BA.2.75.2 variant, a BQ.1 variant, a BQ.1.1 variant, an XBB variant, and a P. 1.351 variant. In some embodiments, the virus is HCV and the more than one subvariant or subtype is derived from any of HCV genotypes 1-7, for example but not limited to any one or more of the HCV genotypes 1a, 1b, 1c, 2a, 2b, 2c, 2d, 2e, 2f, 2g, 2h, 2i, 2j, 2k, 3a, 3b, 3c, 3d, 3e, 3f, 3g, 3h, 3i, 3j, 3k, 4a, 4b, 4c, 4d, 5a, 6a, 6b, 6c, 6d, 6e, 6f, 6g, 6h, 6i, 6j, 6k, and 7a. In some embodiments, the virus is RSV and the more than one subvariant or subtype is RSV A and RSV B.

Efficacy of the treatments described herein can be evaluated, for example, by measuring viral load *(e.g.,* via detection of viral DNA), duration of individual's survival, quality of life, viral protein expression and/or activity, detection of serological antibodies against the coronavirus, assessment of respiratory functions, and/or Computerized Tomography (CT) imaging.

Efficacy of the vaccine compositions can be evaluated, for example, by levels of neutralizing antibodies against one or more proteins of the virus (such as SARS-CoV-2, influenza, hepatitis, or respiratory syncytial virus) in serum or other bodily fluid (such as but not limited to bronchoalveolar lavage fluids), or by the neutralizing activity of serum or other bodily fluid against one or more strains of the virus (such as SARS-CoV-2, influenza, hepatitis, or respiratory syncytial virus).

In some embodiments, the method comprises administrating to the individual an effective amount of any one of the vaccine compositions described herein, the individual displays increased neutralizing antibody level in serum against the target viral antigen (e.g., against the SARS-CoV-2 spike RBD) as compared to before administration of the vaccine composition. In some embodiments, the individual displays increased neutralizing antibody level in serum against a variant target viral antigen as compared to before administration of the vaccine composition. In some embodiments according to any one of the methods described herein, the individual displays an increase in the neutralizing antibody level by about any one of 10%, 20%, 50%, 75%, 100%, 2-fold, 5-fold, 10-fold, 25-fold, 50-fold, 100-fold, 500-fold, or 1000-fold or more, as compared to before administration of the vaccine composition. In some embodiments, the neutralizing antibody level is determined at about any one of 1, 7, 14, 21, 30, 45, 60, 90, 120, 180, 240, 360, 480, or 720 days after administration of the vaccine composition. In some embodiments, the method comprises administering to the individual an effective amount of any one of the vaccine compositions described herein, the individual displays increased neutralizing antibody levels in serum against more than one target viral antigen (e.g., against the SARS-CoV-2 spike RBD and the IAV protein), for example increased by at least about any of 25%, 30%, 35%, 40%, 45%, 50%, 55%, 60%, 65%, 70%, 75%, 80%, 85%, 90%, 95%, or more, as compared to before administration of the vaccine composition. In some embodiments, the individual displays increased neutralizing antibody levels in serum against two or more target viral antigens as compared to before administration of the vaccine composition. In some embodiments, administration of the vaccine composition induces heterosubtypic protection in the subject against two or more target viral antigens that are derived from two or more viral subtypes of the same virus (e.g., two or more IAV subtypes selected from H1N1, H5N1, H3N2, and H7N9) such that.

In some embodiments, the method comprises administrating to the mucosa of the individual an effective amount of any one of the vaccine compositions described herein, the individual displays increased mucosal immunity against the target virus as compared to before administration of the vaccine composition. In some embodiments, the individual displays increased mucosal immunity against two or more target viruses, for example increased by at least about any of 25%, 30%, 35%, 40%, 45%, 50%, 55%, 60%, 65%, 70%, 75%, 80%, 85%, 90%, 95%, 100%, or more or 1.5-fold, 2-fold, 2.5-fold, 3-fold, 3.5-fold, 4-fold, 4.5-fold, 5-fold, 10-fold, 15-fold, 20-fold, 25-fold, 30-fold, 35-fold, 40-fold, 45-fold, 50-fold or higher, as compared to before administration of the vaccine compositions. In some embodiments, the two or more target viruses are part of the same genus, optionally part of the same family. In some embodiments, the two or more target viruses are part of different families. In some embodiments, the individual displays increased mucosal immunity against three or more target viruses (e.g., against three or more subtypes of IAV selected from H1N1, H1N5, H2N3, and H7N9) as compared to before administration of the vaccine composition. In some embodiments, the individual displays induction of lung resident memory B cells subsequent to administration of the vaccine composition. In some embodiments, the individual displays induction of follicular helper T cells subsequent to administration of the vaccine composition. In some embodiments, the individual displays increased neutralizing antibody level in bronchoalveolar lavage (BAL) fluids against the target virus (e.g., against SARS-CoV-2, RSV, or influenza) as compared to before administration of the vaccine composition. In some embodiments, the individual displays increased neutralizing antibody level in bronchoalveolar lavage (BAL) fluids against the two or more target viruses (e.g., against SARS-CoV-2 and IAV) as compared to before administration of the vaccine composition. In some embodiments, the individual displays increased neutralizing antibody level in bronchoalveolar lavage (BAL) fluids against three or more target viruses (e.g., against three or more subtypes of IAV selected from H1N1, H1N5, H2N3, and H7N9) as compared to before administration of the vaccine composition. In some embodiments, the neutralizing antibody comprises IgA. In some embodiments according to any one of the methods described herein, the individual displays an increase in the neutralizing antibody level by about any one of 10%, 20%, 50%, 75%, 100%, 2-fold, 5-fold, 10-fold, 25-fold, 50-fold, 100-fold, 500-fold, or 1000-fold or more, as compared to before administration of the vaccine composition. In some embodiments, the neutralizing antibody level is determined at about any one of 1, 7, 14, 21, 30, 45, 60, 90, 120, 180, 240, 360, 480, or 720 days after administration of the vaccine composition.

In some embodiments, the method comprises administrating to the individual an effective amount of any one of the vaccine compositions described herein, the individual displays increased neutralizing activity in serum against a target virus (e.g., against SARS-CoV-2, influenza, RSV, or HCV) as compared to before administration of the vaccine composition. In some embodiments, the individual displays increased neutralizing activity in serum against two or more target viruses (e.g., against two or more of SARS-CoV-2, IAV, IBV, RSV, and/or HCV) as compared to before administration of the vaccine composition. In some embodiments, the individual displays increased neutralizing activity in serum against three or more target viruses (e.g., against three or more subtypes of IAV selected from H1N1, H1N5, H2N3, and H7N9) as compared to before administration of the vaccine composition. In some embodiments, the individual displays increased neutralizing activity in serum against a variant of the target virus as compared to before administration of the vaccine composition. In some embodiments, the neutralizing activity is quantitatively determined by focus reduction neutralization assay against the virus strain. In some embodiments according to any one of the methods described herein, the individual displays an increase in the neutralizing activity by about any one of 10%, 20%, 50%, 75%, 100%, 2-fold, 5-fold, 10-fold, 25-fold, 50-fold, 100-fold, 500-fold, or 1000-fold or more, as compared to before administration of the vaccine composition. In some embodiments, the neutralizing activity is determined at about any one of 1, 7, 14, 21, 30, 45, 60, 90, 120, 180, 240, 360, 480, or 720 days after administration of the vaccine composition.

In some embodiments, the method comprises administering to the mucosa of the individual an effective amount of any one of the vaccine compositions described herein, the individual displays increased mucosal immunity against a target virus as compared to before administration of the vaccine composition. In some embodiments, the individual displays increased neutralizing activity in bronchoalveolar lavage fluids (BAL) fluids against a target virus (e.g., against SARS-CoV-2, RSV, or influenza) as compared to before administration of the vaccine composition. In some embodiments, the individual displays increased mucosal immunity against more than one target virus as compared to before administration of the vaccine composition. In some embodiments, the individual displays increased mucosal immunity against more than one viral subtype as compared to before administration of the vaccine composition. In some embodiments, the individual displays increased neutralizing activity in bronchoalveolar lavage fluids (BAL) fluids against more than one target virus (e.g., against both SARS-CoV-2 and IAV). In some embodiments, the individual displays increased neutralizing activity in bronchoalveolar lavage fluids (BAL) fluids against more than one viral subtype (e.g., against more than one IAV subtype selected from H1N1, H5N1, H3N2, and H7N9). In some embodiments, the neutralizing activity is quantitatively determined by focus reduction neutralization assay against the authentic live virus. In some embodiments according to any one of the methods described herein, the individual displays an increase in the neutralizing activity by about any one of 10%, 20%, 50%, 75%, 100%, 2-fold, 5-fold, 10-fold, 25-fold, 50-fold, 100-fold, 500-fold, or 1000-fold or more, as compared to before administration of the vaccine composition. In some embodiments, the neutralizing activity is determined at about any one of 1, 7, 14, 21, 30, 45, 60, 90, 120, 180, 240, 360, 480, or 720 days after administration of the vaccine composition.

In some embodiments, the vaccine composition (such as pharmaceutical composition) is administered once. In some embodiments, the vaccine composition (such as pharmaceutical composition) is administered more than once, for example with an interval of about 2 weeks to about 1 year. For methods comprising multiple doses of the vaccine composition, exemplary dosing frequencies can include, but are not limited to, weekly, weekly without break, weekly for two out of three weeks, weekly for three out of four weeks, once every two weeks, once every three weeks, monthly, every six months, yearly, etc. In some embodiments, the vaccine composition is administered about once every 2 weeks, once every 3 weeks, once every 4 weeks, once every 6 weeks, or once every 8 weeks. In some embodiments, the vaccine composition (such as pharmaceutical composition) is administered with an interval of about any of every 2 weeks, 3 weeks, 4 weeks, 5 weeks, 6 weeks, 7 weeks, 8 weeks, 1 month, 2 months, 3 months, 4 months, 5 months, 6 months, 7 months, 8 months, 9 months, 10 months, 11 months, 12 months, 13 months, 14 months, 15 months, 16 months, or 1 year. In some embodiments, the vaccine composition (such as pharmaceutical composition) is administered twice, wherein the subsequent dose is administered 14 days after the initial dose. In some embodiments, the vaccine composition (such as pharmaceutical composition) is administered three times, wherein the subsequent doses are each administered 14 days after the previous dose.

In some embodiments, the method is used to vaccinate or treat an individual (such as human) who has previously been vaccinated. Any of the methods of vaccination or treatment provided herein may be used to vaccinate or treat an individual (such as a human) who has not previously been vaccinated. In some embodiments, the method is used to prophylactically immunize an individual (such as a human). In some embodiments, the method is used to prevent virus transmission from a vaccinated individual (such as a human) to an unvaccinated individual. In some embodiments, the method is used to treat a viral infection in an individual (such as a human). In some embodiments, the method further ameliorates or reduces the infection and associated symptoms in an infected individual (such as a human). In some embodiments, the method is used to elicit an immune response (such as activation of lymphocytes, such as B cells or T cells, including CD4+ T cells and/or CD8+ T cells; and myeloid cells, including but not limited to monocytes, macrophages, neutrophils, granulocytes, mast cells, dendritic cells, and/or eosinophils) in an individual (such as a human). In some embodiments, the method is used as a prophylactic vaccine. In some embodiments, the method is used as a prophylaxis against and/or a treatment for two or more different virus species (i.e., virus species from different families and/or different genera; e.g., against two or more of SARS-CoV-2, IAV, IBV, RSV, and/or HCV). In some embodiments, the method is used as a prophylaxis against and/or a treatment for two or more different virus subspecies (i.e., different strains or subspecies of the same virus species or genus; e.g., against more than one IAV subtype selected from H1N1, H5N1, H3N2, and H7N9). In some embodiments, the method is used as a prophylaxis against and/or a treatment for three or more different virus subspecies (i.e., different strains or subspecies of the same virus species or genus; e.g., against more than one IAV subtype selected from H1N1, H5N1, H3N2, and H7N9). In some embodiments, the method is used to enhance T cell response in an individual (such as a human). In some embodiments, the method is used as a first- or second-line therapy to ameliorate or otherwise reduce the viral infection and associated symptoms thereof. In some embodiments, the individual (such as a human) is any age. In some embodiments, the individual (such as a human) is 65 years of age or older, for example any of 65, 70, 75, 80, 85, 86, 87, 88, 89, 90 years or older. In some embodiments, the individual (such as a human) is 18 years of age or younger, for example any of 18, 17, 16, 15, 14, 13, 12, 11, 10, 9, 8, 7, 6, 5, 4, 3, 2 years or less. In some embodiments, the individual (such as a human) is 6 months of age or younger, for example any of 6, 5, 4, 3, 2, 1 months or newborn. In some embodiments, the individual (such as a human) is healthy. In some embodiments, the individual (such as a human) has a medical condition, a pre-existing condition, or a condition that reduces heart, lung, brain, or immune system function. In some embodiments, the individual (such as a human) is immunocompromised.

The methods or vaccine compositions described herein are suitable for vaccinating against a variety of viruses, and/or treating a disease associated with a variety of viruses. The route of administration, the dosage to be administered, and the degree to which a viral infection is prevented, ameliorated, reduced, treated, or cured is dependent upon the target virus and the target antigen. For example, target antigens including viral envelope, viral membrane, or affiliated proteins may be beneficial targets for preventing viral entry into host cells. Target antigens including polymerases or viral nucleic acids may be beneficial for preventing the replication of viruses. The vaccine composition described herein can be designed to target antigens of interest, such as any of the viral antigens described herein, and the methods of administering the vaccine composition described herein can be modified safely as needed based on the target virus and/or the target antigen due to its improved safety profile compared to current traditional vaccines. In some embodiments, the vaccine composition described herein can be designed to target two or more viral families, genera, or species, such as two or more of SARS-CoV-2, IAV, IBV, RSV, and HCV, and the methods of administering the vaccine composition described herein can be modified safely as needed based on the target viruses and/or the target antigens due to its improved safety profile compared to current traditional vaccines.

For example, in some embodiments, the vaccine composition vaccinates against or treats a sarbecovirus (e.g., SARS-CoV-2), wherein the target antigen comprises an antigenic or immunogenic polypeptide derived from any virus of the sarbecovirus family. In some embodiments, the methods described herein are applicable to all sarbecoviruses, including SARS-CoV/SARS- CoV-1, SARS-CoV-2, SC2r-CoV, SC2r-CoV GX-P5L, Bat CoV BtKY72, Bat CoV BM48-31, 16BO133, JTMC15, Bat SARS CoV Rf1, BtCoV HKU3, LYRa11, Bat SARS-CoV/Rp3, Bat SL-CoV YNLF_31C, Bat SL-CoV YNLF_34C, SHC014-CoV, WIV1, WIV16, Civet SARS-CoV, (Bat) Rc-o319, Bat SL-ZXC21, Bat SL-ZC45, Pangolin SARSr-CoV-GX, Pangolin SARSr-CoV-GD, Rs7327, Rs4231, Rs4084, Rf4092, JL2012, 273-2005, HeB2013, HuB2013, Rs4247, Longquan-140, HKU3-1, GX2013, Shaanxi2011, 279-2005, As6526, Yunnan2011, Rs4237, Rs4081, Bat RshSTT182, Bat RshSTT200, (Bat) RacCS203, (Bat) RmYN02, (Bat) RpYN06, YN2013, (Bat) RaTG13, (Bat) BANAL-52, and SARS-CoV combined variants of concern (VOC) (for example, *see* Nature (2022) 603:913-918, hereby incorporated by reference in its entirety). In some embodiments, the vaccine composition induces heterosubtypic protection against multiple viruses of the coronavirus family, including any of the coronaviruses described above. In some embodiments, the vaccine composition vaccinates against or treats a coronavirus and a virus from another family, genus, or species (e.g., IAV, IBV, RSV, or HCV). In some embodiments, the vaccine composition vaccinates against or treats a sarbecovirus and an influenza virus (e.g., IAV or IBV). In some embodiments, the vaccine composition vaccinates against or treats a sarbecovirus and a hepatitis virus. In some embodiments, the vaccine vaccinates against or treats a sarbecovirus and a respiratory syncytial virus (RSV, e.g., RSV-A or RSV-B).

In some embodiments, the vaccine composition vaccinates against or treats an influenza virus (e.g., IAV or IBV), wherein the target antigen comprises an antigenic or immunogenic polypeptide derived from any virus of the influenza family. In some embodiments, the methods or vaccine compositions described herein are suitable for vaccinating against any influenza virus, for example any of influenza A virus, influenza B virus, influenza C virus, or influenza D virus (*i.e.,* Thogotovirus). For example, the methods described herein are applicable to all influenza viruses, including but not limited to any of H1N1, H1N2, H2N2, H2N3, H3N2, H5N1, H7N7, H7N9, B/Yamagata *(e.g.,* clades Y1, Y2, Y3), B/Victona *(e.g.,* subclades V1A.1, V1A.2, V1A.3), and variants thereof. In some embodiments, the vaccine composition vaccinates against or treats two or more strains of influenza, for example any two or more of influenza A virus (IAV), influenza B virus (IBV), influenza C virus (ICV), or influenza D virus (IDV). In some embodiments, the vaccine composition vaccinates against or treats two or more sub-strains of IAV, for example any two or more of H1N1, H1N2, H2N2, H2N3, H3N2, H5N1, H7N7, and/or H7N9. In some embodiments, the vaccine composition vaccinates against or treats two or more sub-strains of IBV, for example Yamagata and Victoria, or sub-strains thereof, for example, two or more of Yamagata subclades Y1, Y2, and/or Y3, or two or more of Victoria subclades V1A.1, V1A.2, and/or V1A.3.

In some embodiments, the vaccine composition vaccinates against or treats a hepatitis virus (e.g., HCV), wherein the target antigen comprises an antigenic or immunogenic polypeptide derived from any hepatitis virus. In some embodiments, the methods or vaccine compositions described herein are suitable for vaccinating against any hepatitis virus, for example any of hepatitis A virus, hepatitis B virus, hepatitis C virus, hepatitis D virus, hepatitis E virus, or any combination thereof. For example, the methods described herein are applicable to all hepatitis C viruses, including HCV genotype 1a, HCV genotype 1b, HCV genotype 2a, HCV genotype 2b, HCV genotype 2c, HCV genotype 3a, or HCV genotype 3b. In some embodiments, the vaccine composition vaccinates against any two or more species of hepatitis virus, for example any of hepatitis A virus, hepatitis B virus, hepatitis C virus, hepatitis D virus, hepatitis E virus, or any combination thereof. In some embodiments, the vaccine composition provides heterosubtypic protection against any two or more hepatitis C strains, for example HCV genotype 1a, HCV genotype 1b, HCV genotype 2a, HCV genotype 2b, HCV genotype 2c, HCV genotype 3a, and/or HCV genotype 3b.

In some embodiments, the vaccine composition vaccinates against or treats respiratory syncytial virus (RSV), wherein the target antigen comprises an antigenic or immunogenic polypeptide derived from any RSV strain, variant, or subspecies. In some embodiments, the methods or vaccine compositions described herein are suitable for vaccinating against any respiratory syncytial virus, for example any of the 16 RSV-A or 22 RSV-B subtypes *(e.g.,* GA1, GA2, GA5, GA7, BA, *etc.*)*.* In some embodiments, the vaccine composition vaccinates against any two or more strains, variants, or subspecies of respiratory syncytial virus, for example any two or more strains, variants, or subspecies selected from the 16 RSV-A or 22 RSV-B subtypes *(e.g.,* GA1, GA2, GA5, GA7, BA, *etc.*)*.* In some embodiments, the vaccine composition provides heterosubtypic protection against any two or more strains, variants, or subspecies of respiratory syncytial virus, for example any two or more strains, variants, or subspecies selected from the 16 RSV-A or 22 RSV-B subtypes (e.g., GA1, GA2, GA5, GA7, BA, *etc.*)*.*

In some embodiments, there is provided a vaccine composition for use in vaccinating or treating a first virus and a second virus, wherein the vaccine composition comprises a target antigenic or immunogenic polypeptide (i.e., a viral antigen) derived from the first virus, and a nucleoprotein derived from the second virus. In some embodiments, the first virus and the second virus are each independently selected from the group consisting of SARS-CoV-2, IAV, IBV, HCV, and RSV, or a strain, variant, or subspecies thereof. In some embodiments, there is provided a vaccine composition for use in vaccinating against SARS-CoV-2 and IAV, wherein the vaccine composition comprises a viral nucleoprotein derived from SARS-CoV-2 and a viral antigen derived from IAV. In some embodiments, there is provided a vaccine composition for use in vaccinating against SARS-CoV-2 and IBV, wherein the vaccine composition comprises a viral nucleoprotein derived from SARS-CoV-2 and a viral antigen derived from IBV. In some embodiments, there is provided a vaccine composition for use in vaccinating against SARS-CoV-2 and RSV, wherein the vaccine composition comprises a viral nucleoprotein derived from SARS-CoV-2 and a viral antigen derived from RSV. In some embodiments, there is provided a vaccine composition for use in vaccinating against SARS-CoV-2 and HCV, wherein the vaccine composition comprises a viral nucleoprotein derived from SARS-CoV-2 and a viral antigen derived from HCV. In some embodiments there is provided a vaccine composition for use in vaccinating against IAV and SARS-CoV-2, wherein the vaccine composition comprises a viral nucleoprotein derived from IAV and a viral antigen derived from SARS-CoV-2. In some embodiments, there is provided a vaccine composition for use in vaccinating against IAV and IBV, wherein the vaccine composition comprises a viral nucleoprotein derived from IAV and a viral antigen derived from IBV. In some embodiments, there is provided a vaccine composition for use in vaccinating against IAV and HCV, wherein the vaccine composition comprises a viral nucleoprotein derived from IAV and a viral antigen derived from HCV. In some embodiments, there is provided a vaccine composition for use in vaccinating against IAV and RSV, wherein the vaccine composition comprises a viral nucleoprotein derived from IAV and a viral antigen derived from RSV. In some embodiments, there is provided a vaccine composition for use in vaccinating against IBV and SARS-CoV-2, wherein the vaccine composition comprises a viral nucleoprotein derived from IBV and a viral antigen derived from SARS-CoV-2. In some embodiments, there is provided a vaccine composition for use in vaccinating against IBV and IAV, wherein the vaccine composition comprises a viral nucleoprotein derived from IBV and a viral antigen derived from IAV. In some embodiments, there is provided a vaccine composition for use in vaccinating against IBV and HCV, wherein the vaccine composition comprises a viral nucleoprotein derived from IBV and a viral antigen derived from HCV. In some embodiments, there is provided a vaccine composition for use in vaccinating against IBV and RSV, wherein the vaccine composition comprises a viral nucleoprotein derived from IBV and a viral antigen derived from RSV. In some embodiments, there is provided a vaccine composition for use in vaccinating against HCV and SARS-CoV-2, wherein the vaccine composition comprises a viral nucleoprotein derived from HCV and a viral antigen derived from SARS-CoV-2. In some embodiments, there is provided a vaccine composition for use in vaccinating against HCV and IAV, wherein the vaccine composition comprises a viral nucleoprotein derived from HCV and a viral antigen derived from IAV. In some embodiments, there is provided a vaccine composition for use in vaccinating against HCV and IBV, wherein the vaccine composition comprises a viral nucleoprotein derived from HCV and a viral antigen derived from IBV. In some embodiments, there is provided a vaccine composition for use in vaccinating against HCV and RSV, wherein the vaccine composition comprises a viral nucleoprotein derived from HCV and a viral antigen derived from RSV. In some embodiments, there is provided a vaccine composition for use in vaccinating against RSV and SARS-CoV-2, wherein the vaccine composition comprises a viral nucleoprotein derived from RSV and a viral antigen derived from SARS-CoV-2. In some embodiments, there is provided a vaccine composition for use in vaccinating against RSV and IAV, wherein the vaccine composition comprises a viral nucleoprotein derived from RSV and a viral antigen derived from IAV. In some embodiments, there is provided a vaccine composition for use in vaccinating against RSV and IBV, wherein the vaccine composition comprises a viral nucleoprotein derived from RSV and a viral antigen derived from IBV. In some embodiments, there is provided a vaccine composition for use in vaccinating against RSV and HCV, wherein the vaccine composition comprises a viral nucleoprotein derived from RSV and a viral antigen derived from HCV.

The methods described herein may be used as a vaccination, a viral transmission preventative, or a treatment to reduce disease, wherein the treatment may act as a first therapy, second therapy, third therapy, or combination therapy with other types of anti-viral therapies known in the art, such as therapeutic agents selected from the group consisting of a corticosteroid, an anti-inflammatory signal transduction modulator, a β2-adrenoreceptor agonist bronchodilator, an anticholinergic, a mucolytic agent, an antiviral agent, an anti-fibrotic agent, hypertonic saline, an antibody, a vaccine, and mixtures thereof or the like, in an adjuvant setting or a neoadjuvant setting. For example, the antiviral agent can be further selected from the group consisting of remdesivir, lopinavir/ritonavir, IFN-α, lopinavir, ritonavir, penciclovir, galidesivir, disulfiram, darunavir, cobicistat, ASC09F, disulfiram, nafamostat, griffithsin, alisporivir, chloroquine, nitazoxanide, baloxavir marboxil, oseltamivir, zanamivir, peramivir, amantadine, rimantadine, favipiravir, laninamivir, ribavirin, umifenovir, and any combinations thereof. In one aspect, there is provided a method of vaccinating an individual against a virus, comprising administering a vaccine composition comprising nano-complexes to the individual.

The dosing regimen of the vaccine composition (or pharmaceutical composition thereof) administered to the individual (such as human) may vary with the particular vaccine composition, the method of administration, and the particular type and stage of viral infection being treated. In some embodiments, the effective amount of the vaccine is below the level that induces a toxicological effect (*i.e.,* an effect above a clinically acceptable level of toxicity) or is at a level where a potential side effect can be controlled or tolerated when the composition is administered to the individual.

### EXAMPLES

The following examples are included for illustrative purposes only and are not intended to limit the scope of the present disclosure.

### Example 1: Generation of NanoComplex vaccines to various target viral infections

The NanoComplex vaccine is made up of two parts: a nucleoprotein fused with i) a viral antigen, also known as the adaptor-antigen, and ii) a mini-viral RNA (mini-vRNA). The mini-vRNA acts as a scaffold to allow for the attachment of multiple copies of the adaptor-antigen. The nucleoprotein has a high affinity for the vRNA and serves as an adaptor in the NanoComplex vaccine. The vaccine's efficacy is significantly improved by both the formation of the complex, which increases the size of the immunogenic complex, and the presence of an RNA adjuvant, which activates innate immunity. Therefore, the NanoComplex technology can be applied to any vaccine by fusing the corresponding antigen into the adaptor protein, making it a versatile option for vaccine development.

As shown in **FIGs. 1A-1D****,** **FIG. 2****,** and **FIG. 10****,** a total of eleven exemplary NanoComplex vaccines were designed to vaccinate against infections caused by the SARS-CoV-2 coronavirus, influenza virus, respiratory syncytial virus, and hepatitis C virus. Each type of NanoComplex vaccine contained antigens derived from various strains or subtypes of the respective pathogen.

Further, bivalent NanoComplex vaccines can be generated wherein the adaptor protein and the viral antigen are derived from different viruses. Exemplary bivalent NanoComplex vaccines (BiNC) are provided in **FIG. 7** (BiNC-01 through BiNC-04). BiNC-01 and BiNC-02 include a SARS-CoV-2 adaptor fused with an influenza A virus (IAV) antigen and then complexed with the SARS-CoV-2 R266 mini vRNA scaffold (nucleotide position 1 to 266 of the 5' untranslated region of a viral gene of the SARS-CoV-2 genome). BiNC-03 and BiNC-04 include an IAV adaptor fused with a SARS-CoV-2 antigen and then complexed with either the SARS-CoV-2 R266 mini vRNA scaffold or the IAV R326 mini vRNA scaffold (nucleotide position 1 to 326 of the 5' untranslated region of a viral gene of the IAV genome).

### Example 2: NanoComplex vaccine protects against various target virus infection

### Exemplary SARS-CoV-2 NanoComplex vaccines

An exemplary NanoComplex vaccine targeting against SARS-CoV-2 infection was called NC-CoV-01. The adaptor-antigen component included the SARS-CoV-2 nucleoprotein (adaptor) fused to the SARS-CoV-2 spike RBD (antigen), while the mini viral RNA was derived from the 5' untranslated region of the SARS-CoV-2 genome (nucleotide position 1 to 266; namely "R266 mini-vRNA scaffold").

**FIG. 3A** depicts the predicted structure of the adaptor-antigen, and its quality and expression were confirmed by Coomassie blue staining **(****FIG. 3B****).** The secondary and tertiary structures of mini-vRNA, R266, were predicted, as shown in **FIG. 3C** (left and right panels, respectively). The 5' untranslated region folded into a highly structured scaffold for genome replication. The *in vitro* transcribed R266 was visualized using urea-PAGE **(****FIG. 3D****).** In addition, the NanoComplex of NC-CoV-01 was confirmed by gel-shift assay and fluorescence polarization assay, both of which demonstrated the complex formation of adaptor-antigen with a mini-vRNA scaffold **(****FIGs. 3E** and **3F****).**

NC-CoV-01 elicited mucosal and systemic immunity, as demonstrated by murine *in vivo* assays. Traditional adjuvants, such as alum, have been inadequate for use in mucosal vaccination in the human respiratory tract due to the irritative response caused by various exogenous adjuvants. However, the NanoComplex vaccines, for example, NC-CoV-1, were designed to prevent this side-effect by wrapping the immunostimulating mini-vRNA (in this case, R266) with multiple copies of the adaptor-antigen fusion peptide. Thus, the NanoComplex would be expected to be phagocytosed by antigen-presenting cells (APCs), and R266 within the NanoComplex would then active the APCs directly.

As shown via a mouse intranasal immunization model **(****FIG. 4A****),** the induction of both IgA and IgG in lung bronchoalveolar fluid (BALF) demonstrated a strong mucosal immune response **(****FIGs. 4B** and **4C****),** while the potent induction of spike RBD-specific IgG in serum samples indicated a robust systemic adaptive immune response **(****FIG. 4D****).** A mini-RNA (R20, which was 20 nucleotides long) was included as a negative control to evaluate the importance of complex formation and size. Mice immunized with the mixture of adaptor-antigen and R20 elicited antibody response, but less when compared to the NC-CoV-01 NanoComplex vaccine comprising R266 mini-vRNA scaffold.

Furthermore, activation of the innate immune response occurs through the sensing of viral RNA, which is mediated predominantly by Toll-like receptors and/or RIG-I-like receptors. Viral RNAs can be small RNA derived from transcription, long and complex RNA derived from large viral genomes, or long genomic RNA derived during replication. As shown using an intranasal immunization murine model **(****FIG. 5A****),** the vaccination of both NP-RBD+R20 and NP-RBD+R266 activated interferon-β (IFN-β) production **(****FIG. 5B****),** interferon-stimulated genes (IFITM3, **FIG. 5C****;** and RIG-I, **FIG. 5D****),** chemokines (CXCL10, **FIG. 5F****;** and CCL5, **FIG. 5G****)** and pro-inflammatory cytokines (IL-6, **FIG. 5E****;** IL-1β, **FIG. 5H****;** and TNFα, **FIG. 5I****).** NP-RBD without RNA scaffold served as control.

The NC-CoV-01 NanoComplex vaccine efficacy was evaluated in a mouse intranasal infection model **(****FIG. 6A****).** Groups of BALB/c mice were mock-immunized or intranasally immunized with three doses of NP-RBD+R266 (NC-CoV-01). 7-days after the third dose was administered, mice were lethally infected with the SARS-CoV-2 Beta (B. 1.351) virus via intranasal inoculation. Lungs and nasal turbinate were harvested on day 2 post-infection. It was found that mice were completely protected from contracting the SARS-CoV-2 viral infection, as evidenced by the absence of viral titers in lung **(****FIG. 6B****)** and nasal turbinate **(****FIG. 6C****).** In addition, the absence of nucleoprotein staining indicated the absence of viral replication in the lungs of vaccinated mice **(****FIG. 6D****).**

### Exemplary Influenza A Virus NanoComplex vaccines

Two exemplary NanoComplex vaccines targeting against Influenza A infection were called NC-IAV-01 (targeting H1N1) and NC-IAV-02 (targeting H5N1). See **FIG. 10** for exemplary structures. In NC-IAV-01, the adaptor-antigen component included the IAV H5N1/1194 nucleoprotein (adaptor) fused to the IAV H1N1 pdm09 hemagglutinin (HA) protein (antigen), while the mini viral RNA was derived from the 5' untranslated region of the IAV genome (nucleotide position 1 to 326; namely "R326 mini-vRNA scaffold"). In NC-IAV-02, the adaptor-antigen component included the IAV H5N1/1194 nucleoprotein (adaptor) fused to the IAV H5N1/1194 hemagglutinin (HA) protein (antigen), while the mini viral RNA was R326 mini-vRNA scaffold.

### H1N1-targeting NanoComplex vaccine

**FIG. 11A** depicts the predicted ribbon structure of the IAV adaptor-antigen protein that is part of NC-IAV-01. **FIG. 11B** provides a schematic diagram that outlines the immunization protocol of BALB/c mice with 2 doses of: (i) the IAV adaptor-antigen protein alone (NP-H1N1-HA, negative control), or (ii) NC-IAV-01 comprising R326 mini-vRNA scaffold, each administered intranasally 2 times, 14 days apart (n=3 mice per group).

NC-IAV-01 elicited mucosal and systemic immunity, as demonstrated by murine *in vivo* assays. As shown via the mouse intranasal immunization model **(****FIG. 11B****),** the induction of both IgA and IgG in lung bronchoalveolar fluid (BALF) demonstrated a strong mucosal immune response **(****FIGs. 12B** and **12C****),** while the potent induction of H1N1 HA-specific IgG in serum samples indicated a robust systemic adaptive immune response **(****FIG. 12A****).** Mice immunized with NP-H1N1-HA alone elicited an antibody response, but this response was significantly lower than the antibody response triggered by the NC-IAV-01 NanoComplex vaccine comprising the R326 mini-vRNA scaffold. Further, the titer of H1N1-HA-neutralizing antibodies after vaccination was significantly higher in the serum of mice administered intranasal NC-IAV-01 NanoComplex vaccine comprising the R326 mini-vRNA scaffold compared to NP-H1N1-HA alone **(****FIG. 13A****),** indicative of increased H1N1 HA inhibition. Immune neutralization of live H1N1 was assessed by Focus Reduction Neutralization Test, wherein serum samples that were harvested from vaccinated mice were mixed with live virus. FRNT50 titers, as show in **FIG. 13B****,** are representative of the reciprocal dilution of serum that neutralizes 50% of the input virus (i.e., H1N1). As shown in **FIG. 13B****,** serum harvested from mice administered intranasal NC-IAV-01 NanoComplex vaccine comprising the R326 mini-vRNA scaffold showed markedly higher neutralization compared to serum harvested from mice administered intranasal NP-H1N1-HA alone, indicative of higher levels of functional HA-neutralizing antibodies in the serum.

The NC-IAV-01 NanoComplex vaccine efficacy also was evaluated in a mouse intranasal infection model **(****FIG. 14A****).** Groups of BALB/c mice were intranasally immunized with two doses (14-day apart) of (i) PBS, (ii) NP-H1N1-HA alone, or (iii) NP-H1N1-HA+R326 (NC-IAV-01 NanoComplex vaccine comprising R326 mini-vRNA scaffold). On day 14 after the second dose was administered, mice were then infected with the influenza A virus subtype H1N1/pdm09 via intranasal inoculation. Body weight was tracked for 14 days after infection, and mice that were immunized with NP-H1N1-HA+R326 showed no change in body weight over time, whereas mice administered PBS or NP-H1N1-HA alone similarly progressively lost weight **(****FIG. 14B****).** Furthermore, mice that were immunized with NP-H1N1-HA+R326 showed higher survival rates compared to mice administered PBS or NP-H1N1-HA alone **(****FIG. 14C****).**

### H5N1-targeting NanoComplex vaccine

**FIG. 15A** depicts the predicted ribbon structure of the IAV adaptor-antigen protein that is part of NC-IAV-02. **FIG. 15B** provides a schematic diagram that outlines the immunization protocol of BALB/c mice with 2 doses of: (i) the IAV adaptor-antigen protein alone (NP-H5N1-HA, negative control) or (ii) NC-IAV-02 comprising R326 mini-vRNA scaffold, each administered intranasally 2 times, 14 days apart (n=3 mice per group).

NC-IAV-02 elicited mucosal and systemic immunity, as demonstrated by murine *in vivo* assays. As shown via the mouse intranasal immunization model **(****FIG. 15B****),** the induction of both IgA and IgG in lung bronchoalveolar fluid (BALF) demonstrated a strong mucosal immune response **(****FIGs. 16B** and **16C****),** while the potent induction of HSN1 HA-specific IgG in serum samples indicated a robust systemic adaptive immune response **(****FIG. 16A****).** Mice immunized with NP-H5N1-HA alone elicited an antibody response, but this response was significantly lower than the antibody response triggered by the NC-IAV-02 NanoComplex vaccine comprising the R326 mini-vRNA scaffold.

Further, the NC-IAV-02 NanoComplex vaccine efficacy also was evaluated in a mouse intranasal infection model **(****FIG. 17A****).** Groups of BALB/c mice were intranasally immunized with two doses (14-day apart) of (i) PBS, (ii) NP-H5N1-HA alone, or (iii) NP-H5N1-HA+R326 (NC-IAV-02 NanoComplex vaccine comprising R326 mini-vRNA scaffold). On day 14 after the second dose was administered, mice were then infected with the influenza A virus subtype H5N1/1194 via intranasal inoculation. Body weight was tracked for 14 days after infection, and mice that were immunized with NP-H5N1-HA+R326 showed no change in body weight over time, whereas mice administered PBS or NP-H5N1-HA alone similarly progressively lost weight **(****FIG. 17B****).** Furthermore, mice that were immunized with NP-H5N1-HA+R326 showed higher survival rates compared to mice administered PBS or NP-H5N1-HA alone **(****FIG. 17C****).**

Taken together, these data demonstrate that NanoComplex vaccines provided herein are not only effective in protecting against the infection of various viruses *in vivo,* but also safe.

### Example 3: Influenza Virus A (IAV) NanoComplex vaccines cross-react to different IAV subtypic immunogens

BALB/c mice were intranasally immunized with either the exemplary NC-IAV-01 or the exemplary NC-IAV-02 NanoComplex vaccine, or with PBS as a negative control, for 2 doses (14-day apart). BALF cells and spleen cells were harvested 7-days after administration of the second vaccine dose and assayed by ELISpot, using the original immunogen or nucleocapsid protein from different IAV subtypes (i.e., H1N1, H5N1, N3N2, or H7N9) for stimulation. See **FIG. 18A** for test workflow. When stimulating with the same immunogen (H1N1 or H5N1, respectively), both NC-IAV-01 **(****FIG. 18B****)** and NC-IAV-02 **(****FIG. 18C****)** induced strong immune response in BALF and spleen. Surprisingly, when stimulating with H1N1, H5N1, N3N2, or H7N9 nucleocapsid proteins, the exemplary NC-IAV-02 NanoComplex vaccine was capable of stimulating an immune response against each subtype tested (FIG. 18D).

Next, the capacity of NC-IAV-02 to cross-protect against different IAV subtypes after vaccination was tested as shown in **FIG. 19A****.** BALB/c mice were intranasally immunized with the exemplary NC-IAV-02 NanoComplex vaccine or with PBS as a negative control, for 2 doses (14-day apart). On day 14 after the second dose was administered, mice were then infected with the IAV subtype H1N1/PR8 or with the IAV subtype H7N9/AH1 via intranasal inoculation. Body weight was tracked for 14 days after infection, and mice that were immunized with NP-H5N1-HA+R326 significantly less body weight loss compared to mice administered PBS (H1N1/PR8 inoculation: **FIG. 19B****;** and H7N9/AH1 inoculation: **FIG. 19D****).** Furthermore, mice that were immunized with NP-H5N1-HA+R326 showed higher survival rates compared to mice administered PBS (H1N1/PR8 inoculation: **FIG. 19C****;** and H7N9/AH1 inoculation: **FIG. 19E****).**

Thus, these data demonstrate that the IAV NanoComplex vaccines, such as NC-IAV-02, can protect the mice from contracting IAV viral infection even against other subtypes of IAV, indicating that the NanoComplex vaccines described herein are capable of effectively protecting against heterosubtypic viruses.

### Example 4: Bivalent NanoComplex vaccines protect against target virus infection

As described in Example 1 above, bivalent NanoComplex vaccines can be generated wherein the adaptor protein and the viral antigen are derived from different viruses. Exemplary bivalent NanoComplex vaccines (BiNC) are provided in **FIG. 7** (BiNC-01 through BiNC-04). BiNC-01 and BiNC-02 include a SARS-CoV-2 adaptor fused with an influenza A virus (IAV) antigen and then complexed with the SARS-CoV-2 R266 mini vRNA scaffold. BiNC-03 and BiNC-04 include an IAV adaptor fused with a SARS-CoV-2 antigen and then complexed with either the SARS-CoV-2 R266 mini vRNA scaffold or the IAV R326 mini vRNA scaffold.

BiNC-01 elicited mucosal and systemic immunity against H1N1, as demonstrated by murine *in vivo* assays. Following the mouse intranasal immunization model as described above with 2-dose (14-day apart) immunization (e.g., for vaccination with NC-CoV-01, NC-IAV-01, or NC-IAV-02), the induction of both IgA and IgG in lung bronchoalveolar fluid (BALF) demonstrated a strong mucosal immune response **(****FIGs. 8B** and **8C****),** while the potent induction of H1N1 HA-specific IgG in serum samples indicated a robust systemic adaptive immune response **(****FIG. 8A****).** Mice immunized with CoV-NP/H1N1-HA alone elicited an antibody response, but this response was significantly lower than the antibody response triggered by the BiNC-01 NanoComplex vaccine comprising the SARS-CoV-2 R266 mini-vRNA scaffold.

BiNC-02 elicited mucosal and systemic immunity against H5N1, as demonstrated by murine *in vivo* assays. Following the mouse intranasal immunization model as described above with 2-dose (14-day apart) immunization (e.g., for vaccination with NC-CoV-01, NC-IAV-01, or NC-IAV-02), the induction of both IgA and IgG in lung bronchoalveolar fluid (BALF) demonstrated a strong mucosal immune response **(****FIGs. 9B** and **9C****),** while the potent induction of H5N1 HA-specific IgG in serum samples indicated a robust systemic adaptive immune response **(****FIG. 9A****).** Mice immunized with CoV-NP/H5N1-HA alone elicited an antibody response, but this response was significantly lower than the antibody response triggered by the BiNC-02 NanoComplex vaccine comprising the SARS-CoV-2 R266 mini-vRNA scaffold.

Taken together, these data demonstrate that the NanoComplex vaccine, such as NC-CoV-01, NC-IAV-01, NC-IAV-02, BiNC-01, and BiNC-02, completely protected the mice from contracting the viral infection (*e.g*., SARS-CoV-2 infection, influenza A infection) by promoting a robust immune response, including providing heterosubtypic protection against multiple virus subtypes, as well as providing multivalent protection against multiple virus families (i.e., SARS-CoV-2 and IAV). Furthermore, the NanoComplex vaccine was demonstrated to be safe for *in vivo* use.

### SEQUENCE LISTING

**SEQ ID NO: 1 (NC-CoV-01: adaptor + antigen: SARS2 NP + SARS2 Spike RBD ancestor strain; reference sequence of ancestor strain spike (EPI_ISL_434571))** 
GPSDNGPQNQRNAPRITFGGPSDSTGSNQNGERSGARSKQRRPQGLPNNTASWFTALTQHGKEDLKFPRGQ
GVPINTNSSPDDQIGYYRRATRRIRGGDGKMKDLSPRWYFYYLGTGPEAGLPYGANKDGIIWVATEGALN
TPKDHIGTRNPANNAAIVLQLPQGTTLPKGFYAEGSRGGSQASSRSSSRSRNSSRNSTPGSSRGTSPARMAG
NGGDAALALLLLDRLNQLESKMSGKGQQQQGQTVTKKSAAEASKKPRQKRTATKAYNVTQAFGRRGPE
QTQGNFGDQELIRQGTDYKHWPQIAQFAPSASAFFGMSRIGMEVTPSGTWLTYTGAIKLDDKDPNFKDQVI
LLNKHIDAYKTFPPTEPKKDKKKKADETQALPQRQKKQQTVTLLPAADLDDFSKQLQQSMSSADSTQAFT
VEKGIYQTSNFRVQPTESIVRFPNITNLCPFGEVFNATRFASVYAWNRKRISNCVADYSVLYNSASFSTFKC
YGVSPTKLNDLCFTNVYADSFVIRGDEVRQIAPGQTGKIADYNYKLPDDFTGCVIAWNSNNLDSKVGGNY
NYLYRLFRKSNLKPFERDISTEIYQAGSTPCNGVEGFNCYFPLQSYGFQPTNGVGYQPYRVVVLSFELLHAP
ATVCGPKKSTNLVKNKCVNFNF
**SEQ ID NO: 2 (NC-CoV-02: adaptor + antigen: SARS2 NP - SARS2 Spike RBD Omicron BA.5; reference sequence of Omicron BA.5 Spike (EPI_ISL_13777658 - hCoV-19/Hong Kong/HKU-220712-005/2022))** 
GPSDNGPQNQRNAPRITFGGPSDSTGSNQNGERSGARSKQRRPQGLPNNTASWFTALTQHGKEDLKFPRGQ
GVPINTNSSPDDQIGYYRRATRRIRGGDGKMKDLSPRWYFYYLGTGPEAGLPYGANKDGIIWVATEGALN
TPKDHIGTRNPANNAAIVLQLPQGTTLPKGFYAEGSRGGSQASSRSSSRSRNSSRNSTPGSSRGTSPARMAG
 
NGGDAALALLLLDRLNQLESKMSGKGQQQQGQTVTKKSAAEASKKPRQKRTATKAYNVTQAFGRRGPE
QTQGNFGDQELIRQGTDYKHWPQIAQFAPSASAFFGMSRIGMEVTPSGTWLTYTGAIKLDDKDPNFKDQVI
LLNKHIDAYKTFPPTEPKKDKKKKADETQALPQRQKKQQTVTLLPAADLDDFSKQLQQSMSSADSTQAFT
VEKGIYQTSNFRVQPTESIVRFPNITNLCPFDEVFNATRFASVYAWNRKRISNCVADYSVLYNFAPFFAFKC
YGVSPTKLNDLCFTNVYADSFVIRGNEVSQIAPGQTGNIADYNYKLPDDFTGCVIAWNSNKLDSKVGGNY
NYRYRLFRKSNLKPFERDISTEIYQAGNKPCNGVAGVNCYFPLRSYGFRPTYGVGHQPYRVVVLSFELLHA
PATVCGPKKSTNLVKNKCVNF
**SEQ ID NO: 3 (NC-IAV-01: adaptor + antigen: IAV NP - IAV H1N1 pdm09 HA head; reference sequence of H1N1 pdm09 HA (GU931802.1 - A/Hong Kong/415742Md/2009))** 
GPAAAGTAASYEQMETGGERQNATEIRASVGRMVSGIGRFYIQMCTELKLSDYEGRLIQNSITIERMVLSAF
DERRNRYLEEHPSAGKDPKKTGGPIYRRRDGKWVRELILYDKEEIRRIWRQANNGEDATAGLTHLMIWHS
NLNDATYQRTRALVRTGMDPRMCSLMQGSTLPRRSGAAGAAVKGVGTMVMELIRMIKRGINDRNFWRG
ENGRRTRIAYERMCNILKGKFQTAAQRAMMDQVRESRNPGNAEIEDLIFLARSALILRGSVAHKSCLPACV
YGLAVASGYDFEREGYSLVGIDPFRLLQNSQVFSLIRPNENPAHKSQLVWMACHSAAFEDLRVSSFIRGTR
VVPRGQLSTRGVQIASNENMEAMDSNTLELRSRYWAIRTRSGGNTNQQRASAGQISVQPTFSVQRNLPFER
ATIMAAFTGNTEGRTSDMRTEIIRMMESARPEDVSFQGRGVFELSDEKATNPIVPSFDMNNEGSYFFGDNA
EEYDSGSLCKLRGVAPLHLGKCNIAGWILGNPECESLSTASSWSYIVETSSSDNGTCYPGDFIDYEELREQLS
SVSSFERFEIFPKTSSWPNHDSNKGVTAACPHAGAKSFYKNLIWLVKKENSYPKLSKSYINDKGKEVLVLW
GIHHPSTSADQQSLYQNADAYVFVGSSRYSKKFKPEIAIRPKVRGQEGRMNYYWTLVEPGDKITFEATGNL
VVPRYAFAMERNAGSGIIISDTPVHDCN
**SEQ ID NO: 4 (NC-IAV-02: adaptor + antigen: IAV NP - IAV HSNl 1194 HA head; reference sequence of H5N1 1194 HA (EF541402.1 - A/Vietnam/1194/2004))** 
GPAAAGTAASYEQMETGGERQNATEIRASVGRMVSGIGRFYIQMCTELKLSDYEGRLIQNSITIERMVLSAF
DERRNRYLEEHPSAGKDPKKTGGPIYRRRDGKWVRELILYDKEEIRRIWRQANNGEDATAGLTHLMIWHS
NLNDATYQRTRALVRTGMDPRMCSLMQGSTLPRRSGAAGAAVKGVGTMVMELIRMIKRGINDRNFWRG
ENGRRTRIAYERMCNILKGKFQTAAQRAMMDQVRESRNPGNAEIEDLIFLARSALILRGSVAHKSCLPACV
YGLAVASGYDFEREGYSLVGIDPFRLLQNSQVFSLIRPNENPAHKSQLVWMACHSAAFEDLRVSSFIRGTR
VVPRGQLSTRGVQIASNENMEAMDSNTLELRSRYWAIRTRSGGNTNQQRASAGQISVQPTFSVQRNLPFER
ATIMAAFTGNTEGRTSDMRTEIIRMMESARPEDVSFQGRGVFELSDEKATNPIVPSFDMNNEGSYFFGDNA
EEYDSGSKLCDLDGVKPLILRDCSVAGWLLGNPMCDEFINVPEWSYIVEKANPVNDLCYPGDFNDYEELK
HLLSRINHFEKIQIIPKSSWSSHEASLGVSSACPYQGKSSFFRNVVWLIKKNSTYPTIKRSYNNTNQEDLLVL
WGIHHPNDAAEQTKLYQNPTTYISVGTSTLNQRLVPRIATRSKVNGQSGRMEFFWTILKPNDAINFESNGN
FIAPEYAYKIVKKGDSTIMKSELEYGNCNT
**SEQ ID NO: 5 (NC-IAV-03: adaptor + antigen: IAV NP + IAV H3N2 4801 HA head; reference sequence of H3N2 4801 HA (EPI675797 - A/Hong Kong/4801/2014))** 
GPAAAGTAASYEQMETGGERQNATEIRASVGRMVSGIGRFYIQMCTELKLSDYEGRLIQNSITIERMVLSAF
DERRNRYLEEHPSAGKDPKKTGGPIYRRRDGKWVRELILYDKEEIRRIWRQANNGEDATAGLTHLMIWHS
NLNDATYQRTRALVRTGMDPRMCSLMQGSTLPRRSGAAGAAVKGVGTMVMELIRMIKRGINDRNFWRG
ENGRRTRIAYERMCNILKGKFQTAAQRAMMDQVRESRNPGNAEIEDLIFLARSALILRGSVAHKSCLPACV
YGLAVASGYDFEREGYSLVGIDPFRLLQNSQVFSLIRPNENPAHKSQLVWMACHSAAFEDLRVSSFIRGTR
VVPRGQLSTRGVQIASNENMEAMDSNTLELRSRYWAIRTRSGGNTNQQRASAGQISVQPTFSVQRNLPFER
ATIMAAFTGNTEGRTSDMRTEIIRMMESARPEDVSFQGRGVFELSDEKATNPIVPSFDMNNEGSYFFGDNA
EEYDSGSIDALLGDPQCDGFQNKKWDLFVERSKAYSNCYPYDVPDYASLRSLVASSGTLEFNNESFNWTG
VTQNGTSSACIRRSSSSFFSRLNWLTHLNYKYPALNVTMPNNEQFDKLYIWGVHHPGTDKDQIFLYAQSSG
RITVSTKRSQQAVIPNIGSRPRIRDIPSRISIYWTIVKPGDILLINSTGNLIAPRGYFKIRSGKSSIMRSDAPIGKC
KSECITPNGSIPNDKPF
**SEQ ID NO: 6 (NC-IBV-01: adaptor + antigen: IAV NP - IBV Yamagata HA head; reference sequence of IBV Yamagata HA (EPI529345 - B/PHUKET/3073/2013))** 
GPAAAGTAASYEQMETGGERQNATEIRASVGRMVSGIGRFYIQMCTELKLSDYEGRLIQNSITIERMVLSAF
DERRNRYLEEHPSAGKDPKKTGGPIYRRRDGKWVRELILYDKEEIRRIWRQANNGEDATAGLTHLMIWHS
 
NLNDATYQRTRALVRTGMDPRMCSLMQGSTLPRRSGAAGAAVKGVGTMVMELIRMIKRGINDRNFWRG
ENGRRTRIAYERMCNILKGKFQTAAQRAMMDQVRESRNPGNAEIEDLIFLARSALILRGSVAHKSCLPACV
YGLAVASGYDFEREGYSLVGIDPFRLLQNSQVFSLIRPNENPAHKSQLVWMACHSAAFEDLRVSSFIRGTR
VVPRGQLSTRGVQIASNENMEAMDSNTLELRSRYWAIRTRSGGNTNQQRASAGQISVQPTFSVQRNLPFER
ATIMAAFTGNTEGRTSDMRTEIIRMMESARPEDVSFQGRGVFELSDEKATNPIVPSFDMNNEGSYFFGDNA
EEYDSGSFANLKGTRTRGKLCPDCLNCTDLDVALGRPMCVGTTPSAKASILHEVRPVTSGCFPIMHDRTKI
RQLPNLLRGYEKIRLSTQNVIDAEKAPGGPYRLGTSGSCPNATSKIGFFATMAWAVPKDNYKNATNPLTVE
VPYICTEGEDQITVWGFHSDNKTQMKSLYGDSNPQKFTSSANGVTTHYVSQIGDFPDQTEDGGLPQSGRIV
VDYMMQKPGKTGTIVYQRGVLLPQKVWCASGRSKVIKGSLPLIGEADCL
**SEQ ID NO: 7 (NC-IBV-02: adaptor + antigen: IAV NP - IBV Victoria HA head; reference sequence of IBV Victoria HA (EPI1845793 - B/Austria/1359417/2021))** 
GPAAAGTAASYEQMETGGERQNATEIRASVGRMVSGIGRFYIQMCTELKLSDYEGRLIQNSITIERMVLSAF
DERRNRYLEEHPSAGKDPKKTGGPIYRRRDGKWVRELILYDKEEIRRIWRQANNGEDATAGLTHLMIWHS
NLNDATYQRTRALVRTGMDPRMCSLMQGSTLPRRSGAAGAAVKGVGTMVMELIRMIKRGINDRNFWRG
ENGRRTRIAYERMCNILKGKFQTAAQRAMMDQVRESRNPGNAEIEDLIFLARSALILRGSVAHKSCLPACV
YGLAVASGYDFEREGYSLVGIDPFRLLQNSQVFSLIRPNENPAHKSQLVWMACHSAAFEDLRVSSFIRGTR
VVPRGQLSTRGVQIASNENMEAMDSNTLELRSRYWAIRTRSGGNTNQQRASAGQISVQPTFSVQRNLPFER
ATIMAAFTGNTEGRTSDMRTEIIRMMESARPEDVSFQGRGVFELSDEKATNPIVPSFDMNNEGSYFFGDNA
EEYDSGSFANLKGTETRGKLCPKCLNCTDLDVALGRPKCTGKIPSARVSILHEVRPVTSGCFPIMHDRTKIR
QLPNLLRGYEHVRLSTHNVINTEDAPGGPYEIGTSGSCLNITNGKGFFATMAWAVPKNKTATNPLTIEVPYI
CTEEEDQITVWGFHSDDETQMARLYGDSKPQKFTSSANGVTTHYVSQIGGFPNQTEDGGLPQSGRIVVDY
MVQKSGKTGTITYQRGILLPQKVWCASGKSKVIKGSLPLIGEADCL
**SEQ ID NO: 8 (NC-RSV-01: adaptor + antigen: SARS2 NP - RSV subtype A F; reference sequence of RSV subtype A F (EPI_ISL_412866 - hRSV/A/England/397/2017))** 
GPSDNGPQNQRNAPRITFGGPSDSTGSNQNGERSGARSKQRRPQGLPNNTASWFTALTQHGKEDLKFPRGQ
GVPINTNSSPDDQIGYYRRATRRIRGGDGKMKDLSPRWYFYYLGTGPEAGLPYGANKDGIIWVATEGALN
TPKDHIGTRNPANNAAIVLQLPQGTTLPKGFYAEGSRGGSQASSRSSSRSRNSSRNSTPGSSRGTSPARMAG
NGGDAALALLLLDRLNQLESKMSGKGQQQQGQTVTKKSAAEASKKPRQKRTATKAYNVTQAFGRRGPE
QTQGNFGDQELIRQGTDYKHWPQIAQFAPSASAFFGMSRIGMEVTPSGTWLTYTGAIKLDDKDPNFKDQVI
LLNKHIDAYKTFPPTEPKKDKKKKADETQALPQRQKKQQTVTLLPAADLDDFSKQLQQSMSSADSTQAGS
QNITEEFYQSTCSAVSKGYLSALRTGWYTSVITIELSNIKENKCNGTDAKVKLIKQELDKYKNAVTELQLL
MQSTPAANSRARQQQQRFLGFLLGVGSAIASGIAVSKVLHLEGEVNKIKSALLSTNKAVVSLSNGVSVLTS
KVLDLKNYIDKQLLPIVNKQSCSISNIETVIEFQQKNNRLLEITREFSVNAGVTTPVSTYMLTNSELLSLIND
MPITNDQKKLMSSNVQIVRQQSYSIMSIIKEEVLAYVVQLPLYGVIDTPCWKLHTSPLCTTNTKEGSNICLT
RTDRGWYCDNAGSVSFFPQAETCKVQSNRVFCDTMNSLTLPSEVNLCNIDIFNPKYDCKIMTSKTDVSSSVI
TSLGAIVSCYGKTKCTASNKNRGIIKTFSNGCDYVSNKGVDTVSVGNTLYYVNKQEGKSLYVKGEPIINFY
DPLVFPSDEFDASISQVNEKINQSLAFIRKSDELLHNVNAGKSTTN
**SEQ ID NO: 9 (NC-RSV-02: adaptor + antigen: SARS2 NP - RSV subtype B F; reference sequence of RSV subtype B F (EPI_ISL_1653999 - hRSV/B/Australia/VIC-RCH056/2019))** 
GPSDNGPQNQRNAPRITFGGPSDSTGSNQNGERSGARSKQRRPQGLPNNTASWFTALTQHGKEDLKFPRGQ
GVPINTNSSPDDQIGYYRRATRRIRGGDGKMKDLSPRWYFYYLGTGPEAGLPYGANKDGIIWVATEGALN
TPKDHIGTRNPANNAAIVLQLPQGTTLPKGFYAEGSRGGSQASSRSSSRSRNSSRNSTPGSSRGTSPARMAG
NGGDAALALLLLDRLNQLESKMSGKGQQQQGQTVTKKSAAEASKKPRQKRTATKAYNVTQAFGRRGPE
QTQGNFGDQELIRQGTDYKHWPQIAQFAPSASAFFGMSRIGMEVTPSGTWLTYTGAIKLDDKDPNFKDQVI
LLNKHIDAYKTFPPTEPKKDKKKKADETQALPQRQKKQQTVTLLPAADLDDFSKQLQQSMSSADSTQAGS
QNITEEFYQSTCSAVSRGYLSALRTGWYTSVITIELSNIKETKCNGTDTKVKLIKQELDKYKNAVTELQLLM
QNTPAVNNRARQQQQRFLGFLLGVGSAIASGIAVSKVLHLEGEVNKIKNALQLTNKAVVSLSNGVSVLTSR
VLDLKNYINNQLLPMVNRQSCRISNIETVIEFQQKNSRLLEITREFSVNAGVTTPLSTYMLTNSELLSLINDM
PITNDQKKLMSSNVQIVRQQSYSIMSIIKEEVLAYVVQLPIYGVIDTPCWKLHTSPLCTTNIKEGSNICLTRTD
RGWYCDNAGSVSFFPQADTCKVQSNRVFCDTMNSLTLPSEVSLCNTDIFNSKYDCKIMTSKTDISSSVITSL
 
GAIVSCYGKTKCTASNKNRGIIKTFSNGCDYVSNKGVDTVSVGNTLYYVNKLEGKNLYVKGEPIINYYDPL
VFPSDEFDASISQVNEKINQSLAFIRRSDELLHNVNTGKSTTN
**SEQ ID NO: 10 (NC-HCV-01: adaptor + antigen: SARS2 NP - HCV genotype 1a E2; reference sequence of genotype 1a E2 (NC_038882.1; isolate H77))** 
GPSDNGPQNQRNAPRITFGGPSDSTGSNQNGERSGARSKQRRPQGLPNNTASWFTALTQHGKEDLKFPRGQ
GVPINTNSSPDDQIGYYRRATRRIRGGDGKMKDLSPRWYFYYLGTGPEAGLPYGANKDGIIWVATEGALN
TPKDHIGTRNPANNAAIVLQLPQGTTLPKGFYAEGSRGGSQASSRSSSRSRNSSRNSTPGSSRGTSPARMAG
NGGDAALALLLLDRLNQLESKMSGKGQQQQGQTVTKKSAAEASKKPRQKRTATKAYNVTQAFGRRGPE
QTQGNFGDQELIRQGTDYKHWPQIAQFAPSASAFFGMSRIGMEVTPSGTWLTYTGAIKLDDKDPNFKDQVI
LLNKHIDAYKTFPPTEPKKDKKKKADETQALPQRQKKQQTVTLLPAADLDDFSKQLQQSMSSADSTQAGS
ETHVTGGNAGRTTAGLVGLLTPGAKQNIQLINTNGSWHINSTALNCNESLNTGWLAGLFYQHKFNSSGCP
ERLASCRRLTDFAQGWGPISYANGSGLDERPYCWHYPPRPCGIVPAKSVCGPVYCFTPSPVVVGTTDRSGA
PTYSWGANDTDVFVLNNTRPPLGNWFGCTWMNSTGFTKVCGAPPCVIGGVGNNTLLCPTDCFRKHPEAT
YSRCGSGPWITPRCMVDYPYRLWHYPCTINYTIFKVRMYVGGVEHRLEAACNWTRGERCDLEDRDRSELS
PLLLSTTQWQVLPCSFTTLPALSTGLIHLHQNIVDVQYLYGVGSSIASWAIKWEYVVLLFLLLADARVCSCL
WMMLLISQAEA
**SEQ ID NO: 11 (NC-HCV-02: adaptor + antigen: SARS2 NP - HCV genotype 2a E2; reference sequence of genotype 2a E2 (NC_009823.1; isolate HC-J6))** 
GPSDNGPQNQRNAPRITFGGPSDSTGSNQNGERSGARSKQRRPQGLPNNTASWFTALTQHGKEDLKFPRGQ
GVPINTNSSPDDQIGYYRRATRRIRGGDGKMKDLSPRWYFYYLGTGPEAGLPYGANKDGIIWVATEGALN
TPKDHIGTRNPANNAAIVLQLPQGTTLPKGFYAEGSRGGSQASSRSSSRSRNSSRNSTPGSSRGTSPARMAG
NGGDAALALLLLDRLNQLESKMSGKGQQQQGQTVTKKSAAEASKKPRQKRTATKAYNVTQAFGRRGPE
QTQGNFGDQELIRQGTDYKHWPQIAQFAPSASAFFGMSRIGMEVTPSGTWLTYTGAIKLDDKDPNFKDQVI
LLNKHIDAYKTFPPTEPKKDKKKKADETQALPQRQKKQQTVTLLPAADLDDFSKQLQQSMSSADSTQAGS
GTTTVGGAVARSTNVIAGVFSHGPQQNIQLINTNGSWHINRTALNCNDSLNTGFLAALFYTNRFNSSGCPG
RLSACRNIEAFRIGWGTLQYEDNVTNPEDMRPYCWHYPPKPCGVVPARSVCGPVYCFTPSPVVVGTTDRR
GVPTYTWGENETDVFLLNSTRPPQGSWFGCTWMNSTGFTKTCGAPPCRTRADFNASTDLLCPTDCFRKHP
DATYIKCGSGPWLTPKCLVHYPYRLWHYPCTVNFTIFKIRMYVGGVEHRLTAACNFTRGDRCDLEDRDRS
QLSPLLHSTTEWAILPCTYSDLPALSTGLLHLHQNIVDVQYMYGLSPAITKYVVRWEWVVLLFLLLADARV
CACLWMLILLGQAEA
**SEQ ID NO: 12 SARS2 viral nucleoprotein amino acid sequence)** 
SDNGPQNQRNAPRITFGGPSDSTGSNQNGERSGARSKQRRPQGLPNNTASWFTALTQHGKEDLKFPRGQG
VPINTNSSPDDQIGYYRRATRRIRGGDGKMKDLSPRWYFYYLGTGPEAGLPYGANKDGIIWVATEGALNTP
KDHIGTRNPANNAAIVLQLPQGTTLPKGFYAEGSRGGSQASSRSSSRSRNSSRNSTPGSSRGTSPARMAGNG
GDAALALLLLDRLNQLESKMSGKGQQQQGQTVTKKSAAEASKKPRQKRTATKAYNVTQAFGRRGPEQTQ
GNFGDQELIRQGTDYKHWPQIAQFAPSASAFFGMSRIGMEVTPSGTWLTYTGAIKLDDKDPNFKDQVILLN
KHIDAYKTFPPTEPKKDKKKKADETQALPQRQKKQQTVTLLPAADLDDFSKQLQQSMSSADSTQA
**SEQ** ID NO: 13 (IAV viral nucleoprotein amino acid **sequence)** 
AAAGTAASYEQMETGGERQNATEIRASVGRMVSGIGRFYIQMCTELKLSDYEGRLIQNSITIERMVLSAFDE
RRNRYLEEHPSAGKDPKKTGGPIYRRRDGKWVRELILYDKEEIRRIWRQANNGEDATAGLTHLMIWHSNL
NDATYQRTRALVRTGMDPRMCSLMQGSTLPRRSGAAGAAVKGVGTMVMELIRMIKRGINDRNFWRGEN
GRRTRIAYERMCNILKGKFQTAAQRAMMDQVRESRNPGNAEIEDLIFLARSALILRGSVAHKSCLPACVYG
LAVASGYDFEREGYSLVGIDPFRLLQNSQVFSLIRPNENPAHKSQLVWMACHSAAFEDLRVSSFIRGTRVVP
RGQLSTRGVQIASNENMEAMDSNTLELRSRYWAIRTRSGGNTNQQRASAGQISVQPTFSVQRNLPFERATI
MAAFTGNTEGRTSDMRTEIIRMMESARPEDVSFQGRGVFELSDEKATNPIVPSFDMNNEGSYFFGDNAEEY
DS
**SEQ ID NO: 14 (NC-CoV-01 and BiNC-03 antigen amino acid sequence: SARS2 Spike RBD ancestor strain; reference sequence of ancestor strain spike (EPI_ISL_434571))** 
FTVEKGIYQTSNFRVQPTESIVRFPNITNLCPFGEVFNATRFASVYAWNRKRISNCVADYSVLYNSASFSTFK
CYGVSPTKLNDLCFTNVYADSFVIRGDEVRQIAPGQTGKIADYNYKLPDDFTGCVIAWNSNNLDSKVGGN
 
YNYLYRLFRKSNLKPFERDISTEIYQAGSTPCNGVEGFNCYFPLQSYGFQPTNGVGYQPYRVVVLSFELLHA
PATVCGPKKSTNLVKNKCVNFNF
**SEQ ID NO: 15 (NC-CoV-02 and BiNC-04 antigen amino acid sequence: SARS2 Spike RBD Omicron BA.5; reference sequence of Omicron BA.5 Spike (EPI_ISL_13777658 - hCoV-19/Hong Kong/HKU-220712-005/2022))** 
FTVEKGIYQTSNFRVQPTESIVRFPNITNLCPFDEVFNATRFASVYAWNRKRISNCVADYSVLYNFAPFFAFK
CYGVSPTKLNDLCFTNVYADSFVIRGNEVSQIAPGQTGNIADYNYKLPDDFTGCVIAWNSNKLDSKVGGN
YNYRYRLFRKSNLKPFERDISTEIYQAGNKPCNGVAGVNCYFPLRSYGFRPTYGVGHQPYRVVVLSFELLH
APATVCGPKKSTNLVKNKCVNF
**SEQ ID NO: 16 (NC-IAV-01 antigen amino acid sequence: IAV H1N1 pdm09 HA head; reference sequence of H1N1 dm09 HA (GU931802.1 - A/Hong Kong/415742Md/2009))** 
LCKLRGVAPLHLGKCNIAGWILGNPECESLSTASSWSYIVETSSSDNGTCYPGDFIDYEELREQLSSVSSFER
FEIFPKTSSWPNHDSNKGVTAACPHAGAKSFYKNLIWLVKKENSYPKLSKSYINDKGKEVLVLWGIHHPST
SADQQSLYQNADAYVFVGSSRYSKKFKPEIAIRPKVRGQEGRMNYYWTLVEPGDKITFEATGNLVVPRYA
FAMERNAGSGIIISDTPVHDCN
**SEQ ID NO: 17 (BiNC-01 antigen amino acid sequence: IAV H1N1 pdm09 HA head; reference sequence of H1N1 pdm09 HA (GU931802.1 - A/Hong Kong/415742Md/2009))** 
KLCKLRGVAPLHLGKFNIAGWILGNPECESLSTASSWSYIVETSSSDNGTCYPGDFIDYEELREQLSSVSSFE
RFEIFPKTSSWPNHDSNKGVTAACPHAGARSFYKNLIWLVKKENSYPKLSKSYINDKGKEVHVLWGIHHPS
TSADQQSLYQNADAYVFVGSSRYSKKFKPEIAIRPKVRGQEGRMNYYWTLVEPGDRITFEATGNLVVPRY
AFAMERNAGSGIIISDTPVHDCNT
**SEQ ID NO: 18 (NC-IAV-02 and BiNC-02 antigen amino acid sequence: IAV H5N1 1194 HA head; reference sequence of H5N1 1194 HA (EF541402.1 - A/Vietnam/1194/2004))** 
KLCDLDGVKPLILRDCSVAGWLLGNPMCDEFINVPEWSYIVEKANPVNDLCYPGDFNDYEELKHLLSRINH
FEKIQIIPKSSWSSHEASLGVSSACPYQGKSSFFRNVVWLIKKNSTYPTIKRSYNNTNQEDLLVLWGIHHPND
AAEQTKLYQNPTTYISVGTSTLNQRLVPRIATRSKVNGQSGRMEFFWTILKPNDAINFESNGNFIAPEYAYKI
VKKGDSTIMKSELEYGNCNT
**SEQ ID NO: 19 (NC-IBV-01 antigen amino acid sequence: IBV Yamagata HA head; reference sequence of IBV Yamagata HA (EPI529345 - B/PHUKET/3073/2013))** 
FANLKGTRTRGKLCPDCLNCTDLDVALGRPMCVGTTPSAKASILHEVRPVTSGCFPIMHDRTKIRQLPNLLR
GYEKIRLSTQNVIDAEKAPGGPYRLGTSGSCPNATSKIGFFATMAWAVPKDNYKNATNPLTVEVPYICTEG
EDQITVWGFHSDNKTQMKSLYGDSNPQKFTSSANGVTTHYVSQIGDFPDQTEDGGLPQSGRIVVDYMMQK
PGKTGTIVYQRGVLLPQKVWCASGRSKVIKGSLPLIGEADCL
**SEQ ID NO: 20 (NC-IBV-02 antigen amino acid sequence: IBV Victoria HA head; reference sequence of IBV Victoria HA (EPI1845793 - B/Austria/1359417/2021))** 
FANLKGTETRGKLCPKCLNCTDLDVALGRPKCTGKIPSARVSILHEVRPVTSGCFPIMHDRTKIRQLPNLLR
GYEHVRLSTHNVINTEDAPGGPYEIGTSGSCLNITNGKGFFATMAWAVPKNKTATNPLTIEVPYICTEEEDQ
ITVWGFHSDDETQMARLYGDSKPQKFTSSANGVTTHYVSQIGGFPNQTEDGGLPQSGRIVVDYMVQKSGK
TGTITYQRGILLPQKVWCASGKSKVIKGSLPLIGEADCL
**SEQ ID NO: 21 (NC-RSV-01 antigen amino acid sequence: RSV subtype A F; reference sequence of RSV subtype A F (EPI_ISL_412866 - hRSV/A/England/397/2017))** 
QNITEEFYQSTCSAVSKGYLSALRTGWYTSVITIELSNIKENKCNGTDAKVKLIKQELDKYKNAVTELQLLM
QSTPAANSRARQQQQRFLGFLLGVGSAIASGIAVSKVLHLEGEVNKIKSALLSTNKAVVSLSNGVSVLTSKV
LDLKNYIDKQLLPIVNKQSCSISNIETVIEFQQKNNRLLEITREFSVNAGVTTPVSTYMLTNSELLSLINDMPIT
NDQKKLMSSNVQIVRQQSYSIMSIIKEEVLAYVVQLPLYGVIDTPCWKLHTSPLCTTNTKEGSNICLTRTDR
GWYCDNAGSVSFFPQAETCKVQSNRVFCDTMNSLTLPSEVNLCNIDIFNPKYDCKIMTSKTDVSSSVITSLG
AIVSCYGKTKCTASNKNRGIIKTFSNGCDYVSNKGVDTVSVGNTLYYVNKQEGKSLYVKGEPIINFYDPLV
FPSDEFDASISQVNEKINQSLAFIRKSDELLHNVNAGKSTTN
**SEQ ID NO: 22 (NC-RSV-02 antigen amino acid sequence: RSV subtype B F; reference sequence of RSV subtype B F (EPI_ISL_1653999 - hRSV/B/Australia/VIC-RCH056/2019))** 
QNITEEFYQSTCSAVSRGYLSALRTGWYTSVITIELSNIKETKCNGTDTKVKLIKQELDKYKNAVTELQLLM
QNTPAVNNRARQQQRFLGFLLGVGSAIASGIAVSKVLHLEGEVNKIKNALQLTNKAVVSLSNGVSVLTSR
VLDLKNYINNQLLPMVNRQSCRISNIETVIEFQQKNSRLLEITREFSVNAGVTTPLSTYMLTNSELLSLINDM
PITNDQKKLMSSNVQIVRQQSYSIMSIIKEEVLAYVVQLPIYGVIDTPCWKLHTSPLCTTNIKEGSNICLTRTD
RGWYCDNAGSVSFFPQADTCKVQSNRVFCDTMNSLTLPSEVSLCNTDIFNSKYDCKIMTSKTDISSSVITSL
GAIVSCYGKTKCTASNKNRGIIKTFSNGCDYVSNKGVDTVSVGNTLYYVNKLEGKNLYVKGEPIINYYDPL
VFPSDEFDASISQVNEKINQSLAFIRRSDELLHNVNTGKSTTN
**SEQ ID NO: 23 (NC-HCV-01 antigen amino acid sequence: HCV genotype 1a E2; reference sequence of genotype 1a E2 (NC_038882.1; isolate H77))** 
ETHVTGGNAGRTTAGLVGLLTPGAKQNIQLINTNGSWHINSTALNCNESLNTGWLAGLFYQHKFNSSGCPE
RLASCRRLTDFAQGWGPISYANGSGLDERPYCWHYPPRPCGIVPAKSVCGPVYCFTPSPVVVGTTDRSGAP
TYSWGANDTDVFVLNNTRPPLGNWFGCTWMNSTGFTKVCGAPPCVIGGVGNNTLLCPTDCFRKHPEATY
SRCGSGPWITPRCMVDYPYRLWHYPCTINYTIFKVRMYVGGVEHRLEAACNWTRGERCDLEDRDRSELSP
LLLSTTQWQVLPCSFTTLPALSTGLIHLHQNIVDVQYLYGVGSSIASWAIKWEYVVLLFLLLADARVCSCL
WMMLLISQAEA
**SEQ ID NO: 24 (NC-HCV-02 antigen amino acid sequence: HCV genotype 2a E2; reference sequence of genotype 2a E2 (NC 009823.1; isolate HC-J6))** 
[?]
GTTTVGGAVARSTNVIAGVFSHGPQQNIQLINTNGSWHINRTALNCNDSLNTGFLAALFYTNRFNSSGCPG
RLSACRNIEAFRIGWGTLQYEDNVTNPEDMRPYCWHYPPKPCGVVPARSVCGPVYCFTPSPVVVGTTDRR
GVPTYTWGENETDVFLLNSTRPPQGSWFGCTWMNSTGFTKTCGAPPCRTRADFNASTDLLCPTDCFRKHP
DATYIKCGSGPWLTPKCLVHYPYRLWHYPCTVNFTIFKIRMYVGGVEHRLTAACNFTRGDRCDLEDRDRS
QLSPLLHSTTEWAILPCTYSDLPALSTGLLHLHQNIVDVQYMYGLSPAITKYVVRWEWVVLLFLLLADARV
CACLWMLILLGQAEA
**SEQ ID NO: 25 (SARS-CoV-2 DNA sequence, reference sequence)** 
ATTAAAGGTTTATACCTTCCCAGGTAACAAACCAACCAACTTTCGATCTCTTGTAGATCTGTTCTCTAA
ACGAACTTTAAAATCTGTGTGGCTGTCACTCGGCTGCATGCTTAGTGCACTCACGCAGTATAATTAATA
ACTAATTACTGTCGTTGACAGGACACGAGTAACTCGTCTATCTTCTGCAGGCTGCTTACGGTTTCGTCC
GTGTTGCAGCCGATCATCAGCACATCTAGGTTTCGTCCGGGTGTGACCGAAAGGTAAGATGGAGAGCC
TTGTCCCTGGTTTCAACGAGAAAACACACGTCCAACTCAGTTTGCCTGTTTTACAGGTTCGCGACGTGC
TCGTACGTGGCTTTGGAGACTCCGTGGAGGAGGTCTTATCAGAGGCACGTCAACATCTTAAAGATGGC
ACTTGTGGCTTAGTAGAAGTTGAAAAAGGCGTTTTGCCTCAACTTGAACAGCCCTATGTGTTCATCAAA
CGTTCGGATGCTCGAACTGCACCTCATGGTCATGTTATGGTTGAGCTGGTAGCAGAACTCGAAGGCAT
TCAGTACGGTCGTAGTGGTGAGACACTTGGTGTCCTTGTCCCTCATGTGGGCGAAATACCAGTGGCTTA
CCGCAAGGTTCTTCTTCGTAAGAACGGTAATAAAGGAGCTGGTGGCCATAGTTACGGCGCCGATCTAA
AGTCATTTGACTTAGGCGACGAGCTTGGCACTGATCCTTATGAAGATTTTCAAGAAAACTGGAACACT
AAACATAGCAGTGGTGTTACCCGTGAACTCATGCGTGAGCTTAACGGAGGGGCATACACTCGCTATGT
CGATAACAACTTCTGTGGCCCTGATGGCTACCCTCTTGAGTGCATTAAAGACCTTCTAGCACGTGCTGG
TAAAGCTTCATGCACTTTGTCCGAACAACTGGACTTTATTGACACTAAGAGGGGTGTATACTGCTGCCG
TGAACATGAGCATGAAATTGCTTGGTACACGGAACGTTCTGAAAAGAGCTATGAATTGCAGACACCTT
TTGAAATTAAATTGGCAAAGAAATTTGACACCTTCAATGGGGAATGTCCAAATTTTGTATTTCCCTTAA
ATTCCATAATCAAGACTATTCAACCAAGGGTTGAAAAGAAAAAGCTTGATGGCTTTATGGGTAGAATT
CGATCTGTCTATCCAGTTGCGTCACCAAATGAATGCAACCAAATGTGCCTTTCAACTCTCATGAAGTGT
GATCATTGTGGTGAAACTTCATGGCAGACGGGCGATTTTGTTAAAGCCACTTGCGAATTTTGTGGCACT
GAGAATTTGACTAAAGAAGGTGCCACTACTTGTGGTTACTTACCCCAAAATGCTGTTGTTAAAATTTAT
TGTCCAGCATGTCACAATTCAGAAGTAGGACCTGAGCATAGTCTTGCCGAATACCATAATGAATCTGG
CTTGAAAACCATTCTTCGTAAGGGTGGTCGCACTATTGCCTTTGGAGGCTGTGTGTTCTCTTATGTTGG
TTGCCATAACAAGTGTGCCTATTGGGTTCCACGTGCTAGCGCTAACATAGGTTGTAACCATACAGGTGT
TGTTGGAGAAGGTTCCGAAGGTCTTAATGACAACCTTCTTGAAATACTCCAAAAAGAGAAAGTCAACA
TCAATATTGTTGGTGACTTTAAACTTAATGAAGAGATCGCCATTATTTTGGCATCTTTTTCTGCTTCCAC
AAGTGCTTTTGTGGAAACTGTGAAAGGTTTGGATTATAAAGCATTCAAACAAATTGTTGAATCCTGTG
GTAATTTTAAAGTTACAAAAGGAAAAGCTAAAAAAGGTGCCTGGAATATTGGTGAACAGAAATCAAT
 
ACTGAGTCCTCTTTATGCATTTGCATCAGAGGCTGCTCGTGTTGTACGATCAATTTTCTCCCGCACTCTT
GAAACTGCTCAAAATTCTGTGCGTGTTTTACAGAAGGCCGCTATAACAATACTAGATGGAATTTCACA
GTATTCACTGAGACTCATTGATGCTATGATGTTCACATCTGATTTGGCTACTAACAATCTAGTTGTAAT
GGCCTACATTACAGGTGGTGTTGTTCAGTTGACTTCGCAGTGGCTAACTAACATCTTTGGCACTGTTTA
TGAAAAACTCAAACCCGTCCTTGATTGGCTTGAAGAGAAGTTTAAGGAAGGTGTAGAGTTTCTTAGAG
ACGGTTGGGAAATTGTTAAATTTATCTCAACCTGTGCTTGTGAAATTGTCGGTGGACAAATTGTCACCT
GTGCAAAGGAAATTAAGGAGAGTGTTCAGACATTCTTTAAGCTTGTAAATAAATTTTTGGCTTTGTGTG
CTGACTCTATCATTATTGGTGGAGCTAAACTTAAAGCCTTGAATTTAGGTGAAACATTTGTCACGCACT
CAAAGGGATTGTACAGAAAGTGTGTTAAATCCAGAGAAGAAACTGGCCTACTCATGCCTCTAAAAGCC
CCAAAAGAAATTATCTTCTTAGAGGGAGAAACACTTCCCACAGAAGTGTTAACAGAGGAAGTTGTCTT
GAAAACTGGTGATTTACAACCATTAGAACAACCTACTAGTGAAGCTGTTGAAGCTCCATTGGTTGGTA
CACCAGTTTGTATTAACGGGCTTATGTTGCTCGAAATCAAAGACACAGAAAAGTACTGTGCCCTTGCA
CCTAATATGATGGTAACAAACAATACCTTCACACTCAAAGGCGGTGCACCAACAAAGGTTACTTTTGG
TGATGACACTGTGATAGAAGTGCAAGGTTACAAGAGTGTGAATATCACTTTTGAACTTGATGAAAGGA
TTGATAAAGTACTTAATGAGAAGTGCTCTGCCTATACAGTTGAACTCGGTACAGAAGTAAATGAGTTC
GCCTGTGTTGTGGCAGATGCTGTCATAAAAACTTTGCAACCAGTATCTGAATTACTTACACCACTGGGC
ATTGATTTAGATGAGTGGAGTATGGCTACATACTACTTATTTGATGAGTCTGGTGAGTTTAAATTGGCT
TCACATATGTATTGTTCTTTCTACCCTCCAGATGAGGATGAAGAAGAAGGTGATTGTGAAGAAGAAGA
GTTTGAGCCATCAACTCAATATGAGTATGGTACTGAAGATGATTACCAAGGTAAACCTTTGGAATTTG
GTGCCACTTCTGCTGCTCTTCAACCTGAAGAAGAGCAAGAAGAAGATTGGTTAGATGATGATAGTCAA
CAAACTGTTGGTCAACAAGACGGCAGTGAGGACAATCAGACAACTACTATTCAAACAATTGTTGAGGT
TCAACCTCAATTAGAGATGGAACTTACACCAGTTGTTCAGACTATTGAAGTGAATAGTTTTAGTGGTTA
TTTAAAACTTACTGACAATGTATACATTAAAAATGCAGACATTGTGGAAGAAGCTAAAAAGGTAAAAC
CAACAGTGGTTGTTAATGCAGCCAATGTTTACCTTAAACATGGAGGAGGTGTTGCAGGAGCCTTAAAT
AAGGCTACTAACAATGCCATGCAAGTTGAATCTGATGATTACATAGCTACTAATGGACCACTTAAAGT
GGGTGGTAGTTGTGTTTTAAGCGGACACAATCTTGCTAAACACTGTCTTCATGTTGTCGGCCCAAATGT
TAACAAAGGTGAAGACATTCAACTTCTTAAGAGTGCTTATGAAAATTTTAATCAGCACGAAGTTCTAC
TTGCACCATTATTATCAGCTGGTATTTTTGGTGCTGACCCTATACATTCTTTAAGAGTTTGTGTAGATAC
TGTTCGCACAAATGTCTACTTAGCTGTCTTTGATAAAAATCTCTATGACAAACTTGTTTCAAGCTTTTTG
GAAATGAAGAGTGAAAAGCAAGTTGAACAAAAGATCGCTGAGATTCCTAAAGAGGAAGTTAAGCCAT
TTATAACTGAAAGTAAACCTTCAGTTGAACAGAGAAAACAAGATGATAAGAAAATCAAAGCTTGTGTT
GAAGAAGTTACAACAACTCTGGAAGAAACTAAGTTCCTCACAGAAAACTTGTTACTTTATATTGACAT
TAATGGCAATCTTCATCCAGATTCTGCCACTCTTGTTAGTGACATTGACATCACTTTCTTAAAGAAAGA
TGCTCCATATATAGTGGGTGATGTTGTTCAAGAGGGTGTTTTAACTGCTGTGGTTATACCTACTAAAAA
GGCTGGTGGCACTACTGAAATGCTAGCGAAAGCTTTGAGAAAAGTGCCAACAGACAATTATATAACC
ACTTACCCGGGTCAGGGTTTAAATGGTTACACTGTAGAGGAGGCAAAGACAGTGCTTAAAAAGTGTAA
AAGTGCCTTTTACATTCTACCATCTATTATCTCTAATGAGAAGCAAGAAATTCTTGGAACTGTTTCTTG
GAATTTGCGAGAAATGCTTGCACATGCAGAAGAAACACGCAAATTAATGCCTGTCTGTGTGGAAACTA
AAGCCATAGTTTCAACTATACAGCGTAAATATAAGGGTATTAAAATACAAGAGGGTGTGGTTGATTAT
GGTGCTAGATTTTACTTTTACACCAGTAAAACAACTGTAGCGTCACTTATCAACACACTTAACGATCTA
AATGAAACTCTTGTTACAATGCCACTTGGCTATGTAACACATGGCTTAAATTTGGAAGAAGCTGCTCG
GTATATGAGATCTCTCAAAGTGCCAGCTACAGTTTCTGTTTCTTCACCTGATGCTGTTACAGCGTATAA
TGGTTATCTTACTTCTTCTTCTAAAACACCTGAAGAACATTTTATTGAAACCATCTCACTTGCTGGTTCC
TATAAAGATTGGTCCTATTCTGGACAATCTACACAACTAGGTATAGAATTTCTTAAGAGAGGTGATAA
AAGTGTATATTACACTAGTAATCCTACCACATTCCACCTAGATGGTGAAGTTATCACCTTTGACAATCT
TAAGACACTTCTTTCTTTGAGAGAAGTGAGGACTATTAAGGTGTTTACAACAGTAGACAACATTAACC
TCCACACGCAAGTTGTGGACATGTCAATGACATATGGACAACAGTTTGGTCCAACTTATTTGGATGGA
GCTGATGTTACTAAAATAAAACCTCATAATTCACATGAAGGTAAAACATTTTATGTTTTACCTAATGAT
GACACTCTACGTGTTGAGGCTTTTGAGTACTACCACACAACTGATCCTAGTTTTCTGGGTAGGTACATG
TCAGCATTAAATCACACTAAAAAGTGGAAATACCCACAAGTTAATGGTTTAACTTCTATTAAATGGGC
AGATAACAACTGTTATCTTGCCACTGCATTGTTAACACTCCAACAAATAGAGTTGAAGTTTAATCCACC
TGCTCTACAAGATGCTTATTACAGAGCAAGGGCTGGTGAAGCTGCTAACTTTTGTGCACTTATCTTAGC
CTACTGTAATAAGACAGTAGGTGAGTTAGGTGATGTTAGAGAAACAATGAGTTACTTGTTTCAACATG
CCAATTTAGATTCTTGCAAAAGAGTCTTGAACGTGGTGTGTAAAACTTGTGGACAACAGCAGACAACC
CTTAAGGGTGTAGAAGCTGTTATGTACATGGGCACACTTTCTTATGAACAATTTAAGAAAGGTGTTCA
GATACCTTGTACGTGTGGTAAACAAGCTACAAAATATCTAGTACAACAGGAGTCACCTTTTGTTATGA
 
TGTCAGCACCACCTGCTCAGTATGAACTTAAGCATGGTACATTTACTTGTGCTAGTGAGTACACTGGTA
ATTACCAGTGTGGTCACTATAAACATATAACTTCTAAAGAAACTTTGTATTGCATAGACGGTGCTTTAC
TTACAAAGTCCTCAGAATACAAAGGTCCTATTACGGATGTTTTCTACAAAGAAAACAGTTACACAACA
ACCATAAAACCAGTTACTTATAAATTGGATGGTGTTGTTTGTACAGAAATTGACCCTAAGTTGGACAA
TTATTATAAGAAAGACAATTCTTATTTCACAGAGCAACCAATTGATCTTGTACCAAACCAACCATATCC
AAACGCAAGCTTCGATAATTTTAAGTTTGTATGTGATAATATCAAATTTGCTGATGATTTAAACCAGTT
AACTGGTTATAAGAAACCTGCTTCAAGAGAGCTTAAAGTTACATTTTTCCCTGACTTAAATGGTGATGT
GGTGGCTATTGATTATAAACACTACACACCCTCTTTTAAGAAAGGAGCTAAATTGTTACATAAACCTAT
TGTTTGGCATGTTAACAATGCAACTAATAAAGCCACGTATAAACCAAATACCTGGTGTATACGTTGTCT
TTGGAGCACAAAACCAGTTGAAACATCAAATTCGTTTGATGTACTGAAGTCAGAGGACGCGCAGGGA
ATGGATAATCTTGCCTGCGAAGATCTAAAACCAGTCTCTGAAGAAGTAGTGGAAAATCCTACCATACA
GAAAGACGTTCTTGAGTGTAATGTGAAAACTACCGAAGTTGTAGGAGACATTATACTTAAACCAGCAA
ATAATAGTTTAAAAATTACAGAAGAGGTTGGCCACACAGATCTAATGGCTGCTTATGTAGACAATTCT
AGTCTTACTATTAAGAAACCTAATGAATTATCTAGAGTATTAGGTTTGAAAACCCTTGCTACTCATGGT
TTAGCTGCTGTTAATAGTGTCCCTTGGGATACTATAGCTAATTATGCTAAGCCTTTTCTTAACAAAGTT
GTTAGTACAACTACTAACATAGTTACACGGTGTTTAAACCGTGTTTGTACTAATTATATGCCTTATTTCT
TTACTTTATTGCTACAATTGTGTACTTTTACTAGAAGTACAAATTCTAGAATTAAAGCATCTATGCCGA
CTACTATAGCAAAGAATACTGTTAAGAGTGTCGGTAAATTTTGTCTAGAGGCTTCATTTAATTATTTGA
AGTCACCTAATTTTTCTAAACTGATAAATATTATAATTTGGTTTTTACTATTAAGTGTTTGCCTAGGTTC
TTTAATCTACTCAACCGCTGCTTTAGGTGTTTTAATGTCTAATTTAGGCATGCCTTCTTACTGTACTGGT
TACAGAGAAGGCTATTTGAACTCTACTAATGTCACTATTGCAACCTACTGTACTGGTTCTATACCTTGT
AGTGTTTGTCTTAGTGGTTTAGATTCTTTAGACACCTATCCTTCTTTAGAAACTATACAAATTACCATTT
CATCTTTTAAATGGGATTTAACTGCTTTTGGCTTAGTTGCAGAGTGGTTTTTGGCATATATTCTTTTCAC
TAGGTTTTTCTATGTACTTGGATTGGCTGCAATCATGCAATTGTTTTTCAGCTATTTTGCAGTACATTTT
ATTAGTAATTCTTGGCTTATGTGGTTAATAATTAATCTTGTACAAATGGCCCCGATTTCAGCTATGGTT
AGAATGTACATCTTCTTTGCATCATTTTATTATGTATGGAAAAGTTATGTGCATGTTGTAGACGGTTGT
AATTCATCAACTTGTATGATGTGTTACAAACGTAATAGAGCAACAAGAGTCGAATGTACAACTATTGT
TAATGGTGTTAGAAGGTCCTTTTATGTCTATGCTAATGGAGGTAAAGGCTTTTGCAAACTACACAATTG
GAATTGTGTTAATTGTGATACATTCTGTGCTGGTAGTACATTTATTAGTGATGAAGTTGCGAGAGACTT
GTCACTACAGTTTAAAAGACCAATAAATCCTACTGACCAGTCTTCTTACATCGTTGATAGTGTTACAGT
GAAGAATGGTTCCATCCATCTTTACTTTGATAAAGCTGGTCAAAAGACTTATGAAAGACATTCTCTCTC
TCATTTTGTTAACTTAGACAACCTGAGAGCTAATAACACTAAAGGTTCATTGCCTATTAATGTTATAGT
TTTTGATGGTAAATCAAAATGTGAAGAATCATCTGCAAAATCAGCGTCTGTTTACTACAGTCAGCTTAT
GTGTCAACCTATACTGTTACTAGATCAGGCATTAGTGTCTGATGTTGGTGATAGTGCGGAAGTTGCAGT
TAAAATGTTTGATGCTTACGTTAATACGTTTTCATCAACTTTTAACGTACCAATGGAAAAACTCAAAAC
ACTAGTTGCAACTGCAGAAGCTGAACTTGCAAAGAATGTGTCCTTAGACAATGTCTTATCTACTTTTAT
TTCAGCAGCTCGGCAAGGGTTTGTTGATTCAGATGTAGAAACTAAAGATGTTGTTGAATGTCTTAAATT
GTCACATCAATCTGACATAGAAGTTACTGGCGATAGTTGTAATAACTATATGCTCACCTATAACAAAG
TTGAAAACATGACACCCCGTGACCTTGGTGCTTGTATTGACTGTAGTGCGCGTCATATTAATGCGCAGG
TAGCAAAAAGTCACAACATTGCTTTGATATGGAACGTTAAAGATTTCATGTCATTGTCTGAACAACTA
CGAAAACAAATACGTAGTGCTGCTAAAAAGAATAACTTACCTTTTAAGTTGACATGTGCAACTACTAG
ACAAGTTGTTAATGTTGTAACAACAAAGATAGCACTTAAGGGTGGTAAAATTGTTAATAATTGGTTGA
AGCAGTTAATTAAAGTTACACTTGTGTTCCTTTTTGTTGCTGCTATTTTCTATTTAATAACACCTGTTCA
TGTCATGTCTAAACATACTGACTTTTCAAGTGAAATCATAGGATACAAGGCTATTGATGGTGGTGTCAC
TCGTGACATAGCATCTACAGATACTTGTTTTGCTAACAAACATGCTGATTTTGACACATGGTTTAGCCA
GCGTGGTGGTAGTTATACTAATGACAAAGCTTGCCCATTGATTGCTGCAGTCATAACAAGAGAAGTGG
GTTTTGTCGTGCCTGGTTTGCCTGGCACGATATTACGCACAACTAATGGTGACTTTTTGCATTTCTTACC
TAGAGTTTTTAGTGCAGTTGGTAACATCTGTTACACACCATCAAAACTTATAGAGTACACTGACTTTGC
AACATCAGCTTGTGTTTTGGCTGCTGAATGTACAATTTTTAAAGATGCTTCTGGTAAGCCAGTACCATA
TTGTTATGATACCAATGTACTAGAAGGTTCTGTTGCTTATGAAAGTTTACGCCCTGACACACGTTATGT
GCTCATGGATGGCTCTATTATTCAATTTCCTAACACCTACCTTGAAGGTTCTGTTAGAGTGGTAACAAC
TTTTGATTCTGAGTACTGTAGGCACGGCACTTGTGAAAGATCAGAAGCTGGTGTTTGTGTATCTACTAG
TGGTAGATGGGTACTTAACAATGATTATTACAGATCTTTACCAGGAGTTTTCTGTGGTGTAGATGCTGT
AAATTTACTTACTAATATGTTTACACCACTAATTCAACCTATTGGTGCTTTGGACATATCAGCATCTAT
AGTAGCTGGTGGTATTGTAGCTATCGTAGTAACATGCCTTGCCTACTATTTTATGAGGTTTAGAAGAGC
TTTTGGTGAATACAGTCATGTAGTTGCCTTTAATACTTTACTATTCCTTATGTCATTCACTGTACTCTGT
 
TTAACACCAGTTTACTCATTCTTACCTGGTGTTTATTCTGTTATTTACTTGTACTTGACATTTTATCTTAC
TAATGATGTTTCTTTTTTAGCACATATTCAGTGGATGGTTATGTTCACACCTTTAGTACCTTTCTGGATA
ACAATTGCTTATATCATTTGTATTTCCACAAAGCATTTCTATTGGTTCTTTAGTAATTACCTAAAGAGAC
GTGTAGTCTTTAATGGTGTTTCCTTTAGTACTTTTGAAGAAGCTGCGCTGTGCACCTTTTTGTTAAATAA
AGAAATGTATCTAAAGTTGCGTAGTGATGTGCTATTACCTCTTACGCAATATAATAGATACTTAGCTCT
TTATAATAAGTACAAGTATTTTAGTGGAGCAATGGATACAACTAGCTACAGAGAAGCTGCTTGTTGTC
ATCTCGCAAAGGCTCTCAATGACTTCAGTAACTCAGGTTCTGATGTTCTTTACCAACCACCACAAACCT
CTATCACCTCAGCTGTTTTGCAGAGTGGTTTTAGAAAAATGGCATTCCCATCTGGTAAAGTTGAGGGTT
GTATGGTACAAGTAACTTGTGGTACAACTACACTTAACGGTCTTTGGCTTGATGACGTAGTTTACTGTC
CAAGACATGTGATCTGCACCTCTGAAGACATGCTTAACCCTAATTATGAAGATTTACTCATTCGTAAGT
CTAATCATAATTTCTTGGTACAGGCTGGTAATGTTCAACTCAGGGTTATTGGACATTCTATGCAAAATT
GTGTACTTAAGCTTAAGGTTGATACAGCCAATCCTAAGACACCTAAGTATAAGTTTGTTCGCATTCAAC
CAGGACAGACTTTTTCAGTGTTAGCTTGTTACAATGGTTCACCATCTGGTGTTTACCAATGTGCTATGA
GGCCCAATTTCACTATTAAGGGTTCATTCCTTAATGGTTCATGTGGTAGTGTTGGTTTTAACATAGATT
ATGACTGTGTCTCTTTTTGTTACATGCACCATATGGAATTACCAACTGGAGTTCATGCTGGCACAGACT
TAGAAGGTAACTTTTATGGACCTTTTGTTGACAGGCAAACAGCACAAGCAGCTGGTACGGACACAACT
ATTACAGTTAATGTTTTAGCTTGGTTGTACGCTGCTGTTATAAATGGAGACAGGTGGTTTCTCAATCGA
TTTACCACAACTCTTAATGACTTTAACCTTGTGGCTATGAAGTACAATTATGAACCTCTAACACAAGAC
CATGTTGACATACTAGGACCTCTTTCTGCTCAAACTGGAATTGCCGTTTTAGATATGTGTGCTTCATTA
AAAGAATTACTGCAAAATGGTATGAATGGACGTACCATATTGGGTAGTGCTTTATTAGAAGATGAATT
TACACCTTTTGATGTTGTTAGACAATGCTCAGGTGTTACTTTCCAAAGTGCAGTGAAAAGAACAATCA
AGGGTACACACCACTGGTTGTTACTCACAATTTTGACTTCACTTTTAGTTTTAGTCCAGAGTACTCAAT
GGTCTTTGTTCTTTTTTTTGTATGAAAATGCCTTTTTACCTTTTGCTATGGGTATTATTGCTATGTCTGCT
TTTGCAATGATGTTTGTCAAACATAAGCATGCATTTCTCTGTTTGTTTTTGTTACCTTCTCTTGCCACTG
TAGCTTATTTTAATATGGTCTATATGCCTGCTAGTTGGGTGATGCGTATTATGACATGGTTGGATATGG
TTGATACTAGTTTGTCTGGTTTTAAGCTAAAAGACTGTGTTATGTATGCATCAGCTGTAGTGTTACTAA
TCCTTATGACAGCAAGAACTGTGTATGATGATGGTGCTAGGAGAGTGTGGACACTTATGAATGTCTTG
ACACTCGTTTATAAAGTTTATTATGGTAATGCTTTAGATCAAGCCATTTCCATGTGGGCTCTTATAATCT
CTGTTACTTCTAACTACTCAGGTGTAGTTACAACTGTCATGTTTTTGGCCAGAGGTATTGTTTTTATGTG
TGTTGAGTATTGCCCTATTTTCTTCATAACTGGTAATACACTTCAGTGTATAATGCTAGTTTATTGTTTC
TTAGGCTATTTTTGTACTTGTTACTTTGGCCTCTTTTGTTTACTCAACCGCTACTTTAGACTGACTCTTGG
TGTTTATGATTACTTAGTTTCTACACAGGAGTTTAGATATATGAATTCACAGGGACTACTCCCACCCAA
GAATAGCATAGATGCCTTCAAACTCAACATTAAATTGTTGGGTGTTGGTGGCAAACCTTGTATCAAAG
TAGCCACTGTACAGTCTAAAATGTCAGATGTAAAGTGCACATCAGTAGTCTTACTCTCAGTTTTGCAAC
AACTCAGAGTAGAATCATCATCTAAATTGTGGGCTCAATGTGTCCAGTTACACAATGACATTCTCTTAG
CTAAAGATACTACTGAAGCCTTTGAAAAAATGGTTTCACTACTTTCTGTTTTGCTTTCCATGCAGGGTG
CTGTAGACATAAACAAGCTTTGTGAAGAAATGCTGGACAACAGGGCAACCTTACAAGCTATAGCCTCA
GAGTTTAGTTCCCTTCCATCATATGCAGCTTTTGCTACTGCTCAAGAAGCTTATGAGCAGGCTGTTGCT
AATGGTGATTCTGAAGTTGTTCTTAAAAAGTTGAAGAAGTCTTTGAATGTGGCTAAATCTGAATTTGAC
CGTGATGCAGCCATGCAACGTAAGTTGGAAAAGATGGCTGATCAAGCTATGACCCAAATGTATAAAC
AGGCTAGATCTGAGGACAAGAGGGCAAAAGTTACTAGTGCTATGCAGACAATGCTTTTCACTATGCTT
AGAAAGTTGGATAATGATGCACTCAACAACATTATCAACAATGCAAGAGATGGTTGTGTTCCCTTGAA
CATAATACCTCTTACAACAGCAGCCAAACTAATGGTTGTCATACCAGACTATAACACATATAAAAATA
CGTGTGATGGTACAACATTTACTTATGCATCAGCATTGTGGGAAATCCAACAGGTTGTAGATGCAGAT
AGTAAAATTGTTCAACTTAGTGAAATTAGTATGGACAATTCACCTAATTTAGCATGGCCTCTTATTGTA
ACAGCTTTAAGGGCCAATTCTGCTGTCAAATTACAGAATAATGAGCTTAGTCCTGTTGCACTACGACA
GATGTCTTGTGCTGCCGGTACTACACAAACTGCTTGCACTGATGACAATGCGTTAGCTTACTACAACAC
AACAAAGGGAGGTAGGTTTGTACTTGCACTGTTATCCGATTTACAGGATTTGAAATGGGCTAGATTCC
CTAAGAGTGATGGAACTGGTACTATCTATACAGAACTGGAACCACCTTGTAGGTTTGTTACAGACACA
CCTAAAGGTCCTAAAGTGAAGTATTTATACTTTATTAAAGGATTAAACAACCTAAATAGAGGTATGGT
ACTTGGTAGTTTAGCTGCCACAGTACGTCTACAAGCTGGTAATGCAACAGAAGTGCCTGCCAATTCAA
CTGTATTATCTTTCTGTGCTTTTGCTGTAGATGCTGCTAAAGCTTACAAAGATTATCTAGCTAGTGGGG
GACAACCAATCACTAATTGTGTTAAGATGTTGTGTACACACACTGGTACTGGTCAGGCAATAACAGTT
ACACCGGAAGCCAATATGGATCAAGAATCCTTTGGTGGTGCATCGTGTTGTCTGTACTGCCGTTGCCAC
ATAGATCATCCAAATCCTAAAGGATTTTGTGACTTAAAAGGTAAGTATGTACAAATACCTACAACTTG
TGCTAATGACCCTGTGGGTTTTACACTTAAAAACACAGTCTGTACCGTCTGCGGTATGTGGAAAGGTTA
 
TGGCTGTAGTTGTGATCAACTCCGCGAACCCATGCTTCAGTCAGCTGATGCACAATCGTTTTTAAACGG
GTTTGCGGTGTAAGTGCAGCCCGTCTTACACCGTGCGGCACAGGCACTAGTACTGATGTCGTATACAG
GGCTTTTGACATCTACAATGATAAAGTAGCTGGTTTTGCTAAATTCCTAAAAACTAATTGTTGTCGCTT
CCAAGAAAAGGACGAAGATGACAATTTAATTGATTCTTACTTTGTAGTTAAGAGACACACTTTCTCTA
ACTACCAACATGAAGAAACAATTTATAATTTACTTAAGGATTGTCCAGCTGTTGCTAAACATGACTTCT
TTAAGTTTAGAATAGACGGTGACATGGTACCACATATATCACGTCAACGTCTTACTAAATACACAATG
GCAGACCTCGTCTATGCTTTAAGGCATTTTGATGAAGGTAATTGTGACACATTAAAAGAAATACTTGTC
ACATACAATTGTTGTGATGATGATTATTTCAATAAAAAGGACTGGTATGATTTTGTAGAAAACCCAGA
TATATTACGCGTATACGCCAACTTAGGTGAACGTGTACGCCAAGCTTTGTTAAAAACAGTACAATTCT
GTGATGCCATGCGAAATGCTGGTATTGTTGGTGTACTGACATTAGATAATCAAGATCTCAATGGTAAC
TGGTATGATTTCGGTGATTTCATACAAACCACGCCAGGTAGTGGAGTTCCTGTTGTAGATTCTTATTAT
TCATTGTTAATGCCTATATTAACCTTGACCAGGGCTTTAACTGCAGAGTCACATGTTGACACTGACTTA
ACAAAGCCTTACATTAAGTGGGATTTGTTAAAATATGACTTCACGGAAGAGAGGTTAAAACTCTTTGA
CCGTTATTTTAAATATTGGGATCAGACATACCACCCAAATTGTGTTAACTGTTTGGATGACAGATGCAT
TCTGCATTGTGCAAACTTTAATGTTTTATTCTCTACAGTGTTCCCACCTACAAGTTTTGGACCACTAGTG
AGAAAAATATTTGTTGATGGTGTTCCATTTGTAGTTTCAACTGGATACCACTTCAGAGAGCTAGGTGTT
GTACATAATCAGGATGTAAACTTACATAGCTCTAGACTTAGTTTTAAGGAATTACTTGTGTATGCTGCT
GACCCTGCTATGCACGCTGCTTCTGGTAATCTATTACTAGATAAACGCACTACGTGCTTTTCAGTAGCT
GCACTTACTAACAATGTTGCTTTTCAAACTGTCAAACCCGGTAATTTTAACAAAGACTTCTATGACTTT
GCTGTGTCTAAGGGTTTCTTTAAGGAAGGAAGTTCTGTTGAATTAAAACACTTCTTCTTTGCTCAGGAT
GGTAATGCTGCTATCAGCGATTATGACTACTATCGTTATAATCTACCAACAATGTGTGATATCAGACAA
CTACTATTTGTAGTTGAAGTTGTTGATAAGTACTTTGATTGTTACGATGGTGGCTGTATTAATGCTAAC
CAAGTCATCGTCAACAACCTAGACAAATCAGCTGGTTTTCCATTTAATAAATGGGGTAAGGCTAGACT
TTATTATGATTCAATGAGTTATGAGGATCAAGATGCACTTTTCGCATATACAAAACGTAATGTCATCCC
TACTATAACTCAAATGAATCTTAAGTATGCCATTAGTGCAAAGAATAGAGCTCGCACCGTAGCTGGTG
TCTCTATCTGTAGTACTATGACCAATAGACAGTTTCATCAAAAATTATTGAAATCAATAGCCGCCACTA
GAGGAGCTACTGTAGTAATTGGAACAAGCAAATTCTATGGTGGTTGGCACAACATGTTAAAAACTGTT
TATAGTGATGTAGAAAACCCTCACCTTATGGGTTGGGATTATCCTAAATGTGATAGAGCCATGCCTAA
CATGCTTAGAATTATGGCCTCACTTGTTCTTGCTCGCAAACATACAACGTGTTGTAGCTTGTCACACCG
TTTCTATAGATTAGCTAATGAGTGTGCTCAAGTATTGAGTGAAATGGTCATGTGTGGCGGTTCACTATA
TGTTAAACCAGGTGGAACCTCATCAGGAGATGCCACAACTGCTTATGCTAATAGTGTTTTTAACATTTG
TCAAGCTGTCACGGCCAATGTTAATGCACTTTTATCTACTGATGGTAACAAAATTGCCGATAAGTATGT
CCGCAATTTACAACACAGACTTTATGAGTGTCTCTATAGAAATAGAGATGTTGACACAGACTTTGTGA
ATGAGTTTTACGCATATTTGCGTAAACATTTCTCAATGATGATACTCTCTGACGATGCTGTTGTGTGTTT
CAATAGCACTTATGCATCTCAAGGTCTAGTGGCTAGCATAAAGAACTTTAAGTCAGTTCTTTATTATCA
AAACAATGTTTTTATGTCTGAAGCAAAATGTTGGACTGAGACTGACCTTACTAAAGGACCTCATGAAT
TTTGCTCTCAACATACAATGCTAGTTAAACAGGGTGATGATTATGTGTACCTTCCTTACCCAGATCCAT
CAAGAATCCTAGGGGCCGGCTGTTTTGTAGATGATATCGTAAAAACAGATGGTACACTTATGATTGAA
CGGTTCGTGTCTTTAGCTATAGATGCTTACCCACTTACTAAACATCCTAATCAGGAGTATGCTGATGTC
TTTCATTTGTACTTACAATACATAAGAAAGCTACATGATGAGTTAACAGGACACATGTTAGACATGTA
TTCTGTTATGCTTACTAATGATAACACTTCAAGGTATTGGGAACCTGAGTTTTATGAGGCTATGTACAC
ACCGCATACAGTCTTACAGGCTGTTGGGGCTTGTGTTCTTTGCAATTCACAGACTTCATTAAGATGTGG
TGCTTGCATACGTAGACCATTCTTATGTTGTAAATGCTGTTACGACCATGTCATATCAACATCACATAA
ATTAGTCTTGTCTGTTAATCCGTATGTTTGCAATGCTCCAGGTTGTGATGTCACAGATGTGACTCAACT
TTACTTAGGAGGTATGAGCTATTATTGTAAATCACATAAACCACCCATTAGTTTTCCATTGTGTGCTAA
TGGACAAGTTTTTGGTTTATATAAAAATACATGTGTTGGTAGCGATAATGTTACTGACTTTAATGCAAT
TGCAACATGTGACTGGACAAATGCTGGTGATTACATTTTAGCTAACACCTGTACTGAAAGACTCAAGC
TTTTTGCAGCAGAAACGCTCAAAGCTACTGAGGAGACATTTAAACTGTCTTATGGTATTGCTACTGTAC
GTGAAGTGCTGTCTGACAGAGAATTACATCTTTCATGGGAAGTTGGTAAACCTAGACCACCACTTAAC
CGAAATTATGTCTTTACTGGTTATCGTGTAACTAAAAACAGTAAAGTACAAATAGGAGAGTACACCTT
TGAAAAAGGTGACTATGGTGATGCTGTTGTTTACCGAGGTACAACAACTTACAAATTAAATGTTGGTG
ATTATTTTGTGCTGACATCACATACAGTAATGCCATTAAGTGCACCTACACTAGTGCCACAAGAGCACT
ATGTTAGAATTACTGGCTTATACCCAACACTCAATATCTCAGATGAGTTTTCTAGCAATGTTGCAAATT
ATCAAAAGGTTGGTATGCAAAAGTATTCTACACTCCAGGGACCACCTGGTACTGGTAAGAGTCATTTT
GCTATTGGCCTAGCTCTCTACTACCCTTCTGCTCGCATAGTGTATACAGCTTGCTCTCATGCCGCTGTTG
ATGCACTATGTGAGAAGGCATTAAAATATTTGCCTATAGATAAATGTAGTAGAATTATACCTGCACGT
 
GCTCGTGTAGAGTGTTTTGATAAATTCAAAGTGAATTCAACATTAGAACAGTATGTCTTTTGTACTGTA
AATGCATTGCCTGAGACGACAGCAGATATAGTTGTCTTTGATGAAATTTCAATGGCCACAAATTATGA
TTTGAGTGTTGTCAATGCCAGATTACGTGCTAAGCACTATGTGTACATTGGCGACCCTGCTCAATTACC
TGCACCACGCACATTGCTAACTAAGGGCACACTAGAACCAGAATATTTCAATTCAGTGTGTAGACTTA
TGAAAACTATAGGTCCAGACATGTTCCTCGGAACTTGTCGGCGTTGTCCTGCTGAAATTGTTGACACTG
TGAGTGCTTTGGTTTATGATAATAAGCTTAAAGCACATAAAGACAAATCAGCTCAATGCTTTAAAATG
TTTTATAAGGGTGTTATCACGCATGATGTTTCATCTGCAATTAACAGGCCACAAATAGGCGTGGTAAG
AGAATTCCTTACACGTAACCCTGCTTGGAGAAAAGCTGTCTTTATTTCACCTTATAATTCACAGAATGC
TGTAGCCTCAAAGATTTTGGGACTACCAACTCAAACTGTTGATTCATCACAGGGCTCAGAATATGACT
ATGTCATATTCACTCAAACCACTGAAACAGCTCACTCTTGTAATGTAAACAGATTTAATGTTGCTATTA
CCAGAGCAAAAGTAGGCATACTTTGCATAATGTCTGATAGAGACCTTTATGACAAGTTGCAATTTACA
AGTCTTGAAATTCCACGTAGGAATGTGGCAACTTTACAAGCTGAAAATGTAACAGGACTCTTTAAAGA
TTGTAGTAAGGTAATCACTGGGTTACATCCTACACAGGCACCTACACACCTCAGTGTTGACACTAAATT
CAAAACTGAAGGTTTATGTGTTGACATACCTGGCATACCTAAGGACATGACCTATAGAAGACTCATCT
CTATGATGGGTTTTAAAATGAATTATCAAGTTAATGGTTACCCTAACATGTTTATCACCCGCGAAGAAG
CTATAAGACATGTACGTGCATGGATTGGCTTCGATGTCGAGGGGTGTCATGCTACTAGAGAAGCTGTT
GGTACCAATTTACCTTTACAGCTAGGTTTTTCTACAGGTGTTAACCTAGTTGCTGTACCTACAGGTTAT
GTTGATACACCTAATAATACAGATTTTTCCAGAGTTAGTGCTAAACCACCGCCTGGAGATCAATTTAA
ACACCTCATACCACTTATGTACAAAGGACTTCCTTGGAATGTAGTGCGTATAAAGATTGTACAAATGTT
AAGTGACACACTTAAAAATCTCTCTGACAGAGTCGTATTTGTCTTATGGGCACATGGCTTTGAGTTGAC
ATCTATGAAGTATTTTGTGAAAATAGGACCTGAGCGCACCTGTTGTCTATGTGATAGACGTGCCACAT
GCTTTTCCACTGCTTCAGACACTTATGCCTGTTGGCATCATTCTATTGGATTTGATTACGTCTATAATCC
GTTTATGATTGATGTTCAACAATGGGGTTTTACAGGTAACCTACAAAGCAACCATGATCTGTATTGTCA
AGTCCATGGTAATGCACATGTAGCTAGTTGTGATGCAATCATGACTAGGTGTCTAGCTGTCCACGAGT
GCTTTGTTAAGCGTGTTGACTGGACTATTGAATATCCTATAATTGGTGATGAACTGAAGATTAATGCGG
CTTGTAGAAAGGTTCAACACATGGTTGTTAAAGCTGCATTATTAGCAGACAAATTCCCAGTTCTTCACG
ACATTGGTAACCCTAAAGCTATTAAGTGTGTACCTCAAGCTGATGTAGAATGGAAGTTCTATGATGCA
CAGCCTTGTAGTGACAAAGCTTATAAAATAGAAGAATTATTCTATTCTTATGCCACACATTCTGACAAA
TTCACAGATGGTGTATGCCTATTTTGGAATTGCAATGTCGATAGATATCCTGCTAATTCCATTGTTTGT
AGATTTGACACTAGAGTGCTATCTAACCTTAACTTGCCTGGTTGTGATGGTGGCAGTTTGTATGTAAAT
AAACATGCATTCCACACACCAGCTTTTGATAAAAGTGCTTTTGTTAATTTAAAACAATTACCATTTTTC
TATTACTCTGACAGTCCATGTGAGTCTCATGGAAAACAAGTAGTGTCAGATATAGATTATGTACCACT
AAAGTCTGCTACGTGTATAACACGTTGCAATTTAGGTGGTGCTGTCTGTAGACATCATGCTAATGAGTA
CAGATTGTATCTCGATGCTTATAACATGATGATCTCAGCTGGCTTTAGCTTGTGGGTTTACAAACAATT
TGATACTTATAACCTCTGGAACACTTTTACAAGACTTCAGAGTTTAGAAAATGTGGCTTTTAATGTTGT
AAATAAGGGACACTTTGATGGACAACAGGGTGAAGTACCAGTTTCTATCATTAATAACACTGTTTACA
CAAAAGTTGATGGTGTTGATGTAGAATTGTTTGAAAATAAAACAACATTACCTGTTAATGTAGCATTT
GAGCTTTGGGCTAAGCGCAACATTAAACCAGTACCAGAGGTGAAAATACTCAATAATTTGGGTGTGGA
CATTGCTGCTAATACTGTGATCTGGGACTACAAAAGAGATGCTCCAGCACATATATCTACTATTGGTGT
TTGTTCTATGACTGACATAGCCAAGAAACCAACTGAAACGATTTGTGCACCACTCACTGTCTTTTTTGA
TGGTAGAGTTGATGGTCAAGTAGACTTATTTAGAAATGCCCGTAATGGTGTTCTTATTACAGAAGGTA
GTGTTAAAGGTTTACAACCATCTGTAGGTCCCAAACAAGCTAGTCTTAATGGAGTCACATTAATTGGA
GAAGCCGTAAAAACACAGTTCAATTATTATAAGAAAGTTGATGGTGTTGTCCAACAATTACCTGAAAC
TTACTTTACTCAGAGTAGAAATTTACAAGAATTTAAACCCAGGAGTCAAATGGAAATTGATTTCTTAG
AATTAGCTATGGATGAATTCATTGAACGGTATAAATTAGAAGGCTATGCCTTCGAACATATCGTTTATG
GAGATTTTAGTCATAGTCAGTTAGGTGGTTTACATCTACTGATTGGACTAGCTAAACGTTTTAAGGAAT
CACCTTTTGAATTAGAAGATTTTATTCCTATGGACAGTACAGTTAAAAACTATTTCATAACAGATGCGC
AAACAGGTTCATCTAAGTGTGTGTGTTCTGTTATTGATTTATTACTTGATGATTTTGTTGAAATAATAA
AATCCCAAGATTTATCTGTAGTTTCTAAGGTTGTCAAAGTGACTATTGACTATACAGAAATTTCATTTA
TGCTTTGGTGTAAAGATGGCCATGTAGAAACATTTTACCCAAAATTACAATCTAGTCAAGCGTGGCAA
CCGGGTGTTGCTATGCCTAATCTTTACAAAATGCAAAGAATGCTATTAGAAAAGTGTGACCTTCAAAA
TTATGGTGATAGTGCAACATTACCTAAAGGCATAATGATGAATGTCGCAAAATATACTCAACTGTGTC
AATATTTAAACACATTAACATTAGCTGTACCCTATAATATGAGAGTTATACATTTTGGTGCTGGTTCTG
ATAAAGGAGTTGCACCAGGTACAGCTGTTTTAAGACAGTGGTTGCCTACGGGTACGCTGCTTGTCGAT
TCAGATCTTAATGACTTTGTCTCTGATGCAGATTCAACTTTGATTGGTGATTGTGCAACTGTACATACA
GCTAATAAATGGGATCTCATTATTAGTGATATGTACGACCCTAAGACTAAAAATGTTACAAAAGAAAA
 
TGACTCTAAAGAGGGTTTTTTCACTTACATTTGTGGGTTTATACAACAAAAGCTAGCTCTTGGAGGTTC
CGTGGCTATAAAGATAACAGAACATTCTTGGAATGCTGATCTTTATAAGCTCATGGGACACTTCGCAT
GGTGGACAGCCTTTGTTACTAATGTGAATGCGTCATCATCTGAAGCATTTTTAATTGGATGTAATTATC
TTGGCAAACCACGCGAACAAATAGATGGTTATGTCATGCATGCAAATTACATATTTTGGAGGAATACA
AATCCAATTCAGTTGTCTTCCTATTCTTTATTTGACATGAGTAAATTTCCCCTTAAATTAAGGGGTACTG
CTGTTATGTCTTTAAAAGAAGGTCAAATCAATGATATGATTTTATCTCTTCTTAGTAAAGGTAGACTTA
TAATTAGAGAAAACAACAGAGTTGTTATTTCTAGTGATGTTCTTGTTAACAACTAAACGAACAATGTTT
GTTTTTCTTGTTTTATTGCCACTAGTCTCTAGTCAGTGTGTTAATCTTACAACCAGAACTCAATTACCCC
CTGCATACACTAATTCTTTCACACGTGGTGTTTATTACCCTGACAAAGTTTTCAGATCCTCAGTTTTACA
TTCAACTCAGGACTTGTTCTTACCTTTCTTTTCCAATGTTACTTGGTTCCATGCTATACATGTCTCTGGG
ACCAATGGTACTAAGAGGTTTGATAACCCTGTCCTACCATTTAATGATGGTGTTTATTTTGCTTCCACT
GAGAAGTCTAACATAATAAGAGGCTGGATTTTTGGTACTACTTTAGATTCGAAGACCCAGTCCCTACTT
ATTGTTAATAACGCTACTAATGTTGTTATTAAAGTCTGTGAATTTCAATTTTGTAATGATCCATTTTTGG
GTGTTTATTACCACAAAAACAACAAAAGTTGGATGGAAAGTGAGTTCAGAGTTTATTCTAGTGCGAAT
AATTGCACTTTTGAATATGTCTCTCAGCCTTTTCTTATGGACCTTGAAGGAAAACAGGGTAATTTCAAA
AATCTTAGGGAATTTGTGTTTAAGAATATTGATGGTTATTTTAAAATATATTCTAAGCACACGCCTATT
AATTTAGTGCGTGATCTCCCTCAGGGTTTTTCGGCTTTAGAACCATTGGTAGATTTGCCAATAGGTATT
AACATCACTAGGTTTCAAACTTTACTTGCTTTACATAGAAGTTATTTGACTCCTGGTGATTCTTCTTCAG
GTTGGACAGCTGGTGCTGCAGCTTATTATGTGGGTTATCTTCAACCTAGGACTTTTCTATTAAAATATA
ATGAAAATGGAACCATTACAGATGCTGTAGACTGTGCACTTGACCCTCTCTCAGAAACAAAGTGTACG
TTGAAATCCTTCACTGTAGAAAAAGGAATCTATCAAACTTCTAACTTTAGAGTCCAACCAACAGAATC
TATTGTTAGATTTCCTAATATTACAAACTTGTGCCCTTTTGGTGAAGTTTTTAACGCCACCAGATTTGCA
TCTGTTTATGCTTGGAACAGGAAGAGAATCAGCAACTGTGTTGCTGATTATTCTGTCCTATATAATTCC
GCATCATTTTCCACTTTTAAGTGTTATGGAGTGTCTCCTACTAAATTAAATGATCTCTGCTTTACTAATG
TCTATGCAGATTCATTTGTAATTAGAGGTGATGAAGTCAGACAAATCGCTCCAGGGCAAACTGGAAAG
ATTGCTGATTATAATTATAAATTACCAGATGATTTTACAGGCTGCGTTATAGCTTGGAATTCTAACAAT
CTTGATTCTAAGGTTGGTGGTAATTATAATTACCTGTATAGATTGTTTAGGAAGTCTAATCTCAAACCT
TTTGAGAGAGATATTTCAACTGAAATCTATCAGGCCGGTAGCACACCTTGTAATGGTGTTGAAGGTTTT
AATTGTTACTTTCCTTTACAATCATATGGTTTCCAACCCACTAATGGTGTTGGTTACCAACCATACAGA
GTAGTAGTACTTTCTTTTGAACTTCTACATGCACCAGCAACTGTTTGTGGACCTAAAAAGTCTACTAAT
TTGGTTAAAAACAAATGTGTCAATTTCAACTTCAATGGTTTAACAGGCACAGGTGTTCTTACTGAGTCT
AACAAAAAGTTTCTGCCTTTCCAACAATTTGGCAGAGACATTGCTGACACTACTGATGCTGTCCGTGAT
CCACAGACACTTGAGATTCTTGACATTACACCATGTTCTTTTGGTGGTGTCAGTGTTATAACACCAGGA
ACAAATACTTCTAACCAGGTTGCTGTTCTTTATCAGGATGTTAACTGCACAGAAGTCCCTGTTGCTATT
CATGCAGATCAACTTACTCCTACTTGGCGTGTTTATTCTACAGGTTCTAATGTTTTTCAAACACGTGCA
GGCTGTTTAATAGGGGCTGAACATGTCAACAACTCATATGAGTGTGACATACCCATTGGTGCAGGTAT
ATGCGCTAGTTATCAGACTCAGACTAATTCTCCTCGGCGGGCACGTAGTGTAGCTAGTCAATCCATCAT
TGCCTACACTATGTCACTTGGTGCAGAAAATTCAGTTGCTTACTCTAATAACTCTATTGCCATACCCAC
AAATTTTACTATTAGTGTTACCACAGAAATTCTACCAGTGTCTATGACCAAGACATCAGTAGATTGTAC
AATGTACATTTGTGGTGATTCAACTGAATGCAGCAATCTTTTGTTGCAATATGGCAGTTTTTGTACACA
ATTAAACCGTGCTTTAACTGGAATAGCTGTTGAACAAGACAAAAACACCCAAGAAGTTTTTGCACAAG
TCAAACAAATTTACAAAACACCACCAATTAAAGATTTTGGTGGTTTTAATTTTTCACAAATATTACCAG
ATCCATCAAAACCAAGCAAGAGGTCATTTATTGAAGATCTACTTTTCAACAAAGTGACACTTGCAGAT
GCTGGCTTCATCAAACAATATGGTGATTGCCTTGGTGATATTGCTGCTAGAGACCTCATTTGTGCACAA
AAGTTTAACGGCCTTACTGTTTTGCCACCTTTGCTCACAGATGAAATGATTGCTCAATACACTTCTGCA
CTGTTAGCGGGTACAATCACTTCTGGTTGGACCTTTGGTGCAGGTGCTGCATTACAAATACCATTTGCT
ATGCAAATGGCTTATAGGTTTAATGGTATTGGAGTTACACAGAATGTTCTCTATGAGAACCAAAAATT
GATTGCCAACCAATTTAATAGTGCTATTGGCAAAATTCAAGACTCACTTTCTTCCACAGCAAGTGCACT
TGGAAAACTTCAAGATGTGGTCAACCAAAATGCACAAGCTTTAAACACGCTTGTTAAACAACTTAGCT
CCAATTTTGGTGCAATTTCAAGTGTTTTAAATGATATCCTTTCACGTCTTGACAAAGTTGAGGCTGAAG
TGCAAATTGATAGGTTGATCACAGGCAGACTTCAAAGTTTGCAGACATATGTGACTCAACAATTAATT
AGAGCTGCAGAAATCAGAGCTTCTGCTAATCTTGCTGCTACTAAAATGTCAGAGTGTGTACTTGGACA
ATCAAAAAGAGTTGATTTTTGTGGAAAGGGCTATCATCTTATGTCCTTCCCTCAGTCAGCACCTCATGG
TGTAGTCTTCTTGCATGTGACTTATGTCCCTGCACAAGAAAAGAACTTCACAACTGCTCCTGCCATTTG
TCATGATGGAAAAGCACACTTTCCTCGTGAAGGTGTCTTTGTTTCAAATGGCACACACTGGTTTGTAAC
ACAAAGGAATTTTTATGAACCACAAATCATTACTACAGACAACACATTTGTGTCTGGTAACTGTGATG
 
TTGTAATAGGAATTGTCAACAACACAGTTTATGATCCTTTGCAACCTGAATTAGACTCATTCAAGGAG
GAGTTAGATAAATATTTTAAGAATCATACATCACCAGATGTTGATTTAGGTGACATCTCTGGCATTAAT
GCTTCAGTTGTAAACATTCAAAAAGAAATTGACCGCCTCAATGAGGTTGCCAAGAATTTAAATGAATC
TCTCATCGATCTCCAAGAACTTGGAAAGTATGAGCAGTATATAAAATGGCCATGGTACATTTGGCTAG
GTTTTATAGCTGGCTTGATTGCCATAGTAATGGTGACAATTATGCTTTGCTGTATGACCAGTTGCTGTA
GTTGTCTCAAGGGCTGTTGTTCTTGTGGATCCTGCTGCAAATTTGATGAAGACGACTCTGAGCCAGTGC
TCAAAGGAGTCAAATTACATTACACATAAACGAACTTATGGATTTGTTTATGAGAATCTTCACAATTG
GAACTGTAACTTTGAAGCAAGGTGAAATCAAGGATGCTACTCCTTCAGATTTTGTTCGCGCTACTGCA
ACGATACCGATACAAGCCTCACTCCCTTTCGGATGGCTTATTGTTGGCGTTGCACTTCTTGCTGTTTTTC
AGAGCGCTTCCAAAATCATAACCCTCAAAAAGAGATGGCAACTAGCACTCTCCAAGGGTGTTCACTTT
GTTTGCAACTTGCTGTTGTTGTTTGTAACAGTTTACTCACACCTTTTGCTCGTTGCTGCTGGCCTTGAAG
CCCCTTTTCTCTATCTTTATGCTTTAGTCTACTTCTTGCAGAGTATAAACTTTGTAAGAATAATAATGAG
GCTTTGGCTTTGCTGGAAATGCCGTTCCAAAAACCCATTACTTTATGATGCCAACTATTTTCTTTGCTGG
CATACTAATTGTTACGACTATTGTATACCTTACAATAGTGTAACTTCTTCAATTGTCATTACTTCAGGTG
ATGGCACAACAAGTCCTATTTCTGAACATGACTACCAGATTGGTGGTTATACTGAAAAATGGGAATCT
GGAGTAAAAGACTGTGTTGTATTACACAGTTACTTCACTTCAGACTATTACCAGCTGTACTCAACTCAA
TTGAGTACAGACACTGGTGTTGAACATGTTACCTTCTTCATCTACAATAAAATTGTTGATGAGCCTGAA
GAACATGTCCAAATTCACACAATCGACGGTTCATCCGGAGTTGTTAATCCAGTAATGGAACCAATTTA
TGATGAACCGACGACGACTACTAGCGTGCCTTTGTAAGCACAAGCTGATGAGTACGAACTTATGTACT
CATTCGTTTCGGAAGAGACAGGTACGTTAATAGTTAATAGCGTACTTCTTTTTCTTGCTTTCGTGGTATT
CTTGCTAGTTACACTAGCCATCCTTACTGCGCTTCGATTGTGTGCGTACTGCTGCAATATTGTTAACGT
GAGTCTTGTAAAACCTTCTTTTTACGTTTACTCTCGTGTTAAAAATCTGAATTCTTCTAGAGTTCCTGAT
CTTCTGGTCTAAACGAACTAAATATTATATTAGTTTTTCTGTTTGGAACTTTAATTTTAGCCATGGCAGA
TTCCAACGGTACTATTACCGTTGAAGAGCTTAAAAAGCTCCTTGAACAATGGAACCTAGTAATAGGTT
TCCTATTCCTTACATGGATTTGTCTTCTACAATTTGCCTATGCCAACAGGAATAGGTTTTTGTATATAAT
TAAGTTAATTTTCCTCTGGCTGTTATGGCCAGTAACTTTAGCTTGTTTTGTGCTTGCTGCTGTTTACAGA
ATAAATTGGATCACCGGTGGAATTGCTATCGCAATGGCTTGTCTTGTAGGCTTGATGTGGCTCAGCTAC
TTCATTGCTTCTTTCAGACTGTTTGCGCGTACGCGTTCCATGTGGTCATTCAATCCAGAAACTAACATTC
TTCTCAACGTGCCACTCCATGGCACTATTCTGACCAGACCGCTTCTAGAAAGTGAACTCGTAATCGGA
GCTGTGATCCTTCGTGGACATCTTCGTATTGCTGGACACCATCTAGGACGCTGTGACATCAAGGACCTG
CCTAAAGAAATCACTGTTGCTACATCACGAACGCTTTCTTATTACAAATTGGGAGCTTCGCAGCGTGTA
GCAGGTGACTCAGGTTTTGCTGCATACAGTCGCTACAGGATTGGCAACTATAAATTAAACACAGACCA
TTCCAGTAGCAGTGACAATATTGCTTTGCTTGTACAGTAAGTGACAACAGATGTTTCATCTCGTTGACT
TTCAGGTTACTATAGCAGAGATATTACTAATTATTATGAGGACTTTTAAAGTTTCCATTTGGAATCTTG
ATTACATCATAAACCTCATAATTAAAAATTTATCTAAGTCACTAACTGAGAATAAATATTCTCAATTAG
ATGAAGAGCAACCAATGGAGATTGATTAAACGAACATGAAAATTATTCTTTTCTTGGCACTGATAACA
CTCGCTACTTGTGAGCTTTATCACTACCAAGAGTGTGTTAGAGGTACAACAGTACTTTTAAAAGAACCT
TGCTCTTCTGGAACATACGAGGGCAATTCACCATTTCATCCTCTAGCTGATAACAAATTTGCACTGACT
TGCTTTAGCACTCAATTTGCTTTTGCTTGTCCTGACGGCGTAAAACACGTCTATCAGTTACGTGCCAGA
TCAGTTTCACCTAAACTGTTCATCAGACAAGAGGAAGTTCAAGAACTTTACTCTCCAATTTTTCTTATT
GTTGCGGCAATAGTGTTTATAACACTTTGCTTCACACTCAAAAGAAAGACAGAATGATTGAACTTTCA
TTAATTGACTTCTATTTGTGCTTTTTAGCCTTTCTGCTATTCCTTGTTTTAATTATGCTTATTATCTTTTG
GTTCTCACTTGAACTGCAAGATCATAATGAAACTTGTCACGCCTAAACGAACATGAAATTTCTTGTTTT
CTTAGGAATCATCACAACTGTAGCTGCATTTCACCAAGAATGTAGTTTACAGTCATGTACTCAACATCA
ACCATATGTAGTTGATGACCCGTGTCCTATTCACTTCTATTCTAAATGGTATATTAGAGTAGGAGCTAG
AAAATCAGCACCTTTAATTGAATTGTGCGTGGATGAGGCTGGTTCTAAATCACCCATTCAGTACATCG
ATATCGGTAATTATACAGTTTCCTGTTTACCTTTTACAATTAATTGCCAGGAACCTAAATTGGGTAGTC
TTGTAGTGCGTTGTTCGTTCTATGAAGACTTTTTAGAGTATCATGACGTTCGTGTTGTTTTAGATTTCAT
CTAAACGAACAAACTAAAATGTCTGATAATGGACCCCAAAATCAGCGAAATGCACCCCGCATTACGTT
TGGTGGACCCTCAGATTCAACTGGCAGTAACCAGAATGGAGAACGCAGTGGGGCGCGATCAAAACAA
CGTCGGCCCCAAGGTTTACCCAATAATACTGCGTCTTGGTTCACCGCTCTCACTCAACATGGCAAGGA
AGACCTTAAATTCCCTCGAGGACAAGGCGTTCCAATTAACACCAATAGCAGTCCAGATGACCAAATTG
GCTACTACCGAAGAGCTACCAGACGAATTCGTGGTGGTGACGGTAAAATGAAAGATCTCAGTCCAAG
ATGGTATTTCTACTACCTAGGAACTGGGCCAGAAGCTGGACTTCCCTATGGTGCTAACAAAGACGGCA
TCATATGGGTTGCAACTGAGGGAGCCTTGAATACACCAAAAGATCACATTGGCACCCGCAATCCTGCT
AACAATGCTGCAATCGTGCTACAACTTCCTCAAGGAACAACATTGCCAAAAGGCTTCTACGCAGAAGG
 
GAGCAGAGGCGGCAGTCAAGCCTCTTCTCGTTCCTCATCACGTAGTCGCAACAGTTCAAGAAATTCAA
CTCCAGGCAGCAGTAGGGGAACTTCTCCTGCTAGAATGGCTGGCAATGGCGGTGATGCTGCTCTTGCT
TTGCTGCTGCTTGACAGATTGAACCAGCTTGAGAGCAAAATGTCTGGTAAAGGCCAACAACAACAAGG
CCAAACTGTCACTAAGAAATCTGCTGCTGAGGCTTCTAAGAAGCCTCGGCAAAAACGTACTGCCACTA
AAGCATACAATGTAACACAAGCTTTCGGCAGACGTGGTCCAGAACAAACCCAAGGAAATTTTGGGGA
CCAGGAACTAATCAGACAAGGAACTGATTACAAACATTGGCCGCAAATTGCACAATTTGCCCCCAGCG
CTTCAGCGTTCTTCGGAATGTCGCGCATTGGCATGGAAGTCACACCTTCGGGAACGTGGTTGACCTAC
ACAGGTGCCATCAAATTGGATGACAAAGATCCAAATTTCAAAGATCAAGTCATTTTGCTGAATAAGCA
TATTGACGCATACAAAACATTCCCACCAACAGAGCCTAAAAAGGACAAAAAGAAGAAGGCTGATGAA
ACTCAAGCCTTACCGCAGAGACAGAAGAAACAGCAAACTGTGACTCTTCTTCCTGCTGCAGATTTGGA
TGATTTCTCCAAACAATTGCAACAATCCATGAGCAGTGCTGACTCAACTCAGGCCTAAACTCATGCAG
ACCACACAAGGCAGATGGGCTATATAAACGTTTTCGCTTTTCCGTTTACGATATATAGTCTACTCTTGT
GCAGAATGAATTCTCGTAACTACATAGCACAAGTAGATGTAGTTAACTTTAATCTCACATAGCAATCTT
TAATCAGTGTGTAACATTAGGGAGGACTTGAAAGAGCCACCACATTTTCACCGAGGCCACGCGGAGTA
CGATCGAGTGTACAGTGAACAATGCTAGGGAGAGCTGCCTATATGGAAGAGCCCTAATGTGTAAAATT
AATTTTAGTAGTGCTATCCCCATGTGATTTTAATAGCTTCTTAGGAGAATGACAAAAAAAAAAAAAAA
AAAAAAAAAAAAAAAAAA
**SEQ ID NO: 26 (BiNC-01: adaptor + antigen: SARS2 NP + IAV H1N1 pdm09 HA head; reference sequence of H1N1 pdm09 HA (GU931802.1 - A/Hong Kong/415742Md/2009))** 
GPSDNGPQNQRNAPRITFGGPSDSTGSNQNGERSGARSKQRRPQGLPNNTASWFTALTQHGKEDLKFPRGQ
GVPINTNSSPDDQIGYYRRATRRIRGGDGKMKDLSPRWYFYYLGTGPEAGLPYGANKDGIIWVATEGALN
TPKDHIGTRNPANNAAIVLQLPQGTTLPKGFYAEGSRGGSQASSRSSSRSRNSSRNSTPGSSRGTSPARMAG
NGGDAALALLLLDRLNQLESKMSGKGQQQQGQTVTKKSAAEASKKPRQKRTATKAYNVTQAFGRRGPE
QTQGNFGDQELIRQGTDYKHWPQIAQFAPSASAFFGMSRIGMEVTPSGTWLTYTGAIKLDDKDPNFKDQVI
LLNKHIDAYKTFPPTEPKKDKKKKADETQALPQRQKKQQTVTLLPAADLDDFSKQLQQSMSSADSTQAGS
KLCKLRGVAPLHLGKFNIAGWILGNPECESLSTASSWSYIVETSSSDNGTCYPGDFIDYEELREQLSSVSSFE
RFEIFPKTSSWPNHDSNKGVTAACPHAGARSFYKNLIWLVKKENSYPKLSKSYINDKGKEVHVLWGIHHPS
TSADQQSLYQNADAYVFVGSSRYSKKFKPEIAIRPKVRGQEGRMNYYWTLVEPGDRITFEATGNLVVPRY
AFAMERNAGSGIIISDTPVHDCNT
**SEQ ID NO: 27 (BiNC-02: adaptor + antigen: SARS2 NP + IAV HSN1 1194 HA head; reference sequence of H5N1 1194 HA (EF541402.1 - A/Vietnam/1194/2004))** 
GPSDNGPQNQRNAPRITFGGPSDSTGSNQNGERSGARSKQRRPQGLPNNTASWFTALTQHGKEDLKFPRGQ
GVPINTNSSPDDQIGYYRRATRRIRGGDGKMKDLSPRWYFYYLGTGPEAGLPYGANKDGIIWVATEGALN
TPKDHIGTRNPANNAAIVLQLPQGTTLPKGFYAEGSRGGSQASSRSSSRSRNSSRNSTPGSSRGTSPARMAG
NGGDAALALLLLDRLNQLESKMSGKGQQQQGQTVTKKSAAEASKKPRQKRTATKAYNVTQAFGRRGPE
QTQGNFGDQELIRQGTDYKHWPQIAQFAPSASAFFGMSRIGMEVTPSGTWLTYTGAIKLDDKDPNFKDQVI
LLNKHIDAYKTFPPTEPKKDKKKKADETQALPQRQKKQQTVTLLPAADLDDFSKQLQQSMSSADSTQAGS
KLCDLDGVKPLILRDCSVAGWLLGNPMCDEFINVPEWSYIVEKANPVNDLCYPGDFNDYEELKHLLSRINH
FEKIQIIPKSSWSSHEASLGVSSACPYQGKSSFFRNVVWLIKKNSTYPTIKRSYNNTNQEDLLVLWGIHHPND
AAEQTKLYQNPTTYISVGTSTLNQRLVPRIATRSKVNGQSGRMEFFWTILKPNDAINFESNGNFIAPEYAYK
IVKKGDSTIMKSELEYGNCNT
**SEQ ID NO: 28 (BiNC-03: adaptor + antigen: IAV NP + SARS2 Spike RBD ancestor strain; reference sequence of ancestor strain spike (EPI_ISL_434571))** 
GPAAAGTAASYEQMETGGERQNATEIRASVGRMVSGIGRFYIQMCTELKLSDYEGRLIQNSITIERMVLSAF
DERRNRYLEEHPSAGKDPKKTGGPIYRRRDGKWVRELILYDKEEIRRIWRQANNGEDATAGLTHLMIWHS
NLNDATYQRTRALVRTGMDPRMCSLMQGSTLPRRSGAAGAAVKGVGTMVMELIRMIKRGINDRNFWRG
ENGRRTRIAYERMCNILKGKFQTAAQRAMMDQVRESRNPGNAEIEDLIFLARSALILRGSVAHKSCLPACV
YGLAVASGYDFEREGYSLVGIDPFRLLQNSQVFSLIRPNENPAHKSQLVWMACHSAAFEDLRVSSFIRGTR
VVPRGQLSTRGVQIASNENMEAMDSNTLELRSRYWAIRTRSGGNTNQQRASAGQISVQPTFSVQRNLPFER
ATIMAAFTGNTEGRTSDMRTEIIRMMESARPEDVSFQGRGVFELSDEKATNPIVPSFDMNNEGSYFFGDNA
EEYDSGSFTVEKGIYQTSNFRVQPTESIVRFPNITNLCPFGEVFNATRFASVYAWNRKRISNCVADYSVLYN
SASFSTFKCYGVSPTKLNDLCFTNVYADSFVIRGDEVRQIAPGQTGKIADYNYKLPDDFTGCVIAWNSNNL
 
DSKVGGNYNYLYRLFRKSNLKPFERDISTEIYQAGSTPCNGVEGFNCYFPLQSYGFQPTNGVGYQPYRVVV
LSFELLHAPATVCGPKKSTNLVKNKCVNFNF
**SEQ ID NO: 29 (BiNC-04: adaptor + antigen: IAV NP + SARS2 Spike RBD Omicron BA.5; reference sequence of Omicron BA.5 Spike (EPI_ISL_13777658 - hCoV-19/Hong Kong/HKU-220712- 005/2022))** 
GPAAAGTAASYEQMETGGERQNATEIRASVGRMVSGIGRFYIQMCTELKLSDYEGRLIQNSITIERMVLSAF
DERRNRYLEEHPSAGKDPKKTGGPIYRRRDGKWVRELILYDKEEIRRIWRQANNGEDATAGLTHLMIWHS
NLNDATYQRTRALVRTGMDPRMCSLMQGSTLPRRSGAAGAAVKGVGTMVMELIRMIKRGINDRNFWRG
ENGRRTRIAYERMCNILKGKFQTAAQRAMMDQVRESRNPGNAEIEDLIFLARSALILRGSVAHKSCLPACV
YGLAVASGYDFEREGYSLVGIDPFRLLQNSQVFSLIRPNENPAHKSQLVWMACHSAAFEDLRVSSFIRGTR
VVPRGQLSTRGVQIASNENMEAMDSNTLELRSRYWAIRTRSGGNTNQQRASAGQISVQPTFSVQRNLPFER
ATIMAAFTGNTEGRTSDMRTEIIRMMESARPEDVSFQGRGVFELSDEKATNPIVPSFDMNNEGSYFFGDNA
EEYDSFTVEKGIYQTSNFRVQPTESIVRFPNITNLCPFDEVFNATRFASVYAWNRKRISNCVADYSVLYNFA
PFFAFKCYGVSPTKLNDLCFTNVYADSFVIRGNEVSQIAPGQTGNIADYNYKLPDDFTGCVIAWNSNKLDS
KVGGNYNYRYRLFRKSNLKPFERDISTEIYQAGNKPCNGVAGVNCYFPLRSYGFRPTYGVGHQPYRVVVL
SFELLHAPATVCGPKKSTNLVKNKCVNF
**SEQ ID NO: 30 (H7N9/ZJ, EPI_ISL_148744)** 
aaagaactaagagatttgatgtcacagtctcgcactcgcgagatactgacaaaaacaactgtggaccatatggccataatcaagaaatatacatcaggaagacaggagaa
gaatcctgcccttaggatgaagtggatgatggcaatgaaatatccaattacagcaaacaaaaggataatggagatgatcccggaaagaaatgagcaaggtcagacccttt
ggagcaagacaaatgatgctggatcaaacagagtgatggtgtcacctctggctgtgacgtggtggaacagaaatggaccaacgacaagcacagtccattatccaaaggt
ctataaaacctattttgaaaaggtcgaaaggctaaaacatggaaccttcggccccgttcacttcagaaaccaggttaaaatacgccgcagggtcgacataaacccaggcc
atgcagaccttagtgctaaagaagcacaagatgtcatcatggaggtcgtattcccaaacgaagttggagccagaatattgacatcagagtcacagttaacgataaccaag
gaaaagaagaaggagcttcaggactgcagaattgcccctttaatggtggcttacatgttggagagagaactggttcgcaaaacaagattcctaccagtagctggaggga
caagcagcgtgtatatcgaggtgttgcatttgacccaagggacctgctgggagcaaatgtacacaccgggaggggaagtgagaaatgatgatgttgatcagagtttaatt
attgctgctagaaatattgttagaagagcaacagtatcagcagacccgttggcttcgcttttggagatgtgccatagtacacaaattggcggggttaggatggttgacatcct
tagacaaaacccaacagaagaacaggctgtggatatatgtaaggcagcaatgggtctaaggatcagttcatccttcagctttggaggtttcactttcaaaaggacaagtgg
gtcatctgtcaaaagggaagaagaagtgctcacaggcaacctccaaacattgaaaataagagtacatgaaggatatgaggaattcacaatggtcgggcgaagagcaac
agccattctaaggaaagcaaccagaagactgatccaactgatagtgagtgggaaagacgagcaatcaatcgccgaggcaatcatagtggcaatggtattctcacaagag
gattgtatgataaaggcagtgaggggtgatttgaactttgtcaacagagcaaaccagcggctaaatcccatgcatcaactcctgaggcatttccaaaaggatgcaaaggtc
ctgtttcaaaactggggaattgaacccattgacaatgtaatggggatgatcggaatattgcctgacatgacccccagcacagagatgtcattgagaggagtgagagttagt
aaaatgggagtagatgaatattccagtactgagagagtggtcgtgagtattgatcgtttcttgagggtccgagaccagaggggaaacgtactcctgtctcctgaagaggtt
agtgaaacacagggaacagaaaagctgactataacatattcatcgtccatgatgtgggagatcaatggtccggaatcagtgctagttaacacatatcaatggatcattagaa
attgggaaactgtaaagattcaatggtcccaagatcctacaatgctatacaataagatggaatttgaaccctttcaatccctagtgcctaaagctgccagaggccaatatagt
gggttcgtgagggttctattccaacagatgcgtgacgtactgggaacatttgacactgtccaaataataaagctattaccatttgcagcagccccgccggagcagagtaga
atgcagttctcttctctaactgtgaatgtgaggggttcaggaatgagaatagttgtgagaggcaactcccctgtgttcaactacaacaaggcaaccaagaggcttacagtgc
ttggaaaggatgcaggtgcattgatggaagacccagatgagggaacagcaggagtggaatctgcggtattgaggggatttctgattctgggcaaagaagacaaaagat
atgggccagcattgagcatcaacgaattgagcaatcttgcgaaaggagagaaggctaatgtattgatagggcaaggagacgttgtgttggtgatgaaacggaaacggg
actctagcatacttactgacagtcagacagcgaccaaaaggattcggatggccatcaatt
**SEQ ID NO: 31 (NC-IAV-03 antigen amino acid sequence: IAV H3N2 4801 HA head; reference sequence of H3N2 4801 HA (EPI675797 - A/Hong Kong/4801/2014))**

IDALLGDPQCDGFQNKKWDLFVERSKAYSNCYPYDVPDYASLRSLVASSGTLEFNNESFNWTGVTQNGTS
SACIRRSSSSFFSRLNWLTHLNYKYPALNVTMPNNEQFDKLYIWGVHHPGTDKDQIFLYAQSSGRITVSTKR
SQQAVIPNIGSRPRIRDIPSRISIYWTIVKPGDILLINSTGNLIAPRGYFKIRSGKSSIMRSDAPIGKCKSECITPN
GSIPNDKPF

### NUMBERED EMBODIMENTS

1. A vaccine composition comprising nano-complexes comprising a scaffold molecule comprising a viral RNA and an adaptor-antigen polypeptide comprising a viral antigen fused to a viral nucleoprotein capable of binding to the viral RNA.
2. The vaccine composition of embodiment 1, wherein the molar ratio of the adaptor-antigen polypeptide to the scaffold molecule is more than about 5:1.
3. The vaccine composition of embodiment 1 or 2, wherein the viral antigen is an antigen polypeptide.
4. The vaccine composition of embodiment 1 or 2, wherein the viral antigen is an immunogenic protein.
5. The vaccine composition of any one of embodiments 1-4, wherein the viral antigen is fused to the C-terminus of the viral nucleoprotein.
6. The vaccine composition of any one of embodiments 1-4, wherein the viral antigen is fused to the N-terminus of the viral nucleoprotein.
7. The vaccine composition of any one of embodiments 1-6, wherein the viral RNA comprises an untranslated region of a viral genome.
8. The vaccine composition of clam 7, wherein the viral RNA comprises a 5' untranslated region or a 3' untranslated region of a viral gene of the viral genome.
9. The vaccine composition of any one of embodiments 1-8, wherein the viral RNA is about 10 to about 500 nucleotides long.
10. The vaccine composition of embodiment 9, wherein the viral RNA is about 200 to about 500 nucleotides long.
11. The vaccine composition of any one of embodiments 1-10, wherein the viral RNA is derived from SARS-CoV-2.
12. The vaccine composition of embodiment 11, wherein the viral RNA comprises nucleotides 1-266 of a viral gene of the SARS-CoV-2 genome set forth in SEQ ID NO:25.
13. The vaccine composition of any one of embodiments 1-10, wherein the viral RNA is derived from an influenza A virus (IAV).
14. The vaccine composition of embodiment 13, wherein the viral RNA comprises nucleotides 1-326 of a viral gene of the IAV genome set forth in SEQ ID NO:30.
15. The vaccine composition of any one of embodiments 1-14, wherein the viral nucleoprotein is derived from SARS-CoV-2.
16. The vaccine composition of embodiment 15, wherein the viral nucleoprotein derived from SARS-CoV-2 comprises the amino acid sequence of SEQ ID NO:12.
17. The vaccine composition of any one of embodiments 1-14, wherein the viral nucleoprotein is derived from an IAV.
18. The vaccine composition of embodiment 17, wherein the viral nucleoprotein is derived from an IAV subtype selected from the group consisting of H1N1, H5N1, H3N2, and H7N9.
19. The vaccine composition of embodiment 17 or 18, wherein the viral nucleoprotein derived from IAV H5N1 comprises the amino acid sequence of SEQ ID NO:13.
20. The vaccine composition of any one of embodiments 1-19, wherein the viral nucleoprotein and the viral antigen are derived from the same virus.
21. The vaccine composition of any one of embodiments 1-19, wherein the viral nucleoprotein and the viral antigen are derived from different viruses.
22. The vaccine composition of any one of embodiments 1-21, wherein the viral antigen is a SARS-Cov-2 protein or a fragment or variant thereof.
23. The vaccine composition of embodiment 22, wherein the viral antigen is a SARS-CoV-2 spike protein or a fragment or variant thereof.
24. The vaccine composition of embodiment 23, wherein the viral antigen comprises a receptor binding domain (RBD).
25. The vaccine composition of embodiment 23 or 24, wherein the SARS-CoV-2 viral antigen comprises the amino acid sequence of SEQ ID NO:14 or 15.
26. The vaccine composition of any one of embodiments 1-21, wherein the viral antigen is an influenza virus protein or a fragment or variant thereof.
27. The vaccine composition of embodiment 26, wherein the viral antigen is an influenza HA protein or a fragment or variant thereof.
28. The vaccine composition of embodiment 26 or 27, wherein the influenza virus protein or fragment or variant thereof is derived from IAV or influenza B virus (IBV).
29. The vaccine composition of embodiment 28, wherein the influenza virus protein or fragment or variant thereof is derived from an IAV subtype selected from the group consisting of H1N1, H5N1, H3N2, and H7N9.
30. The vaccine composition of embodiment 29, wherein the IAV viral antigen comprises an amino acid sequence selected from the group consisting of SEQ ID NOs: 16-18 and 31.
31. The vaccine composition of embodiment 28, wherein the influenza virus protein or a fragment or variant thereof is derived from an IBV subtype of Yamagata or Victoria.
32. The vaccine composition of embodiment 31, wherein the IBV viral antigen comprises the amino acid sequence of SEQ ID NO:19 or 20.
33. The vaccine composition of any one of embodiments 1-21, wherein the viral antigen is a respiratory syncytial virus ("RSV") protein or a fragment or variant thereof.
34. The vaccine composition of embodiment 33, wherein the viral antigen is an RSV F protein or a fragment or variant thereof.
35. The vaccine composition of embodiment 34, wherein the RSV F protein or fragment or variant thereof is derived from RSV A subtype or RSV B subtype.
36. The vaccine composition of embodiment 34 or 35, wherein the RSV viral antigen comprises the amino acid sequence of SEQ ID NO:21 or 22.
37. The vaccine composition of any one of embodiments 1-21, wherein the viral antigen is a hepatitis C virus ("HCV") protein or a fragment or variant thereof.
38. The vaccine composition of embodiment 37, wherein the viral antigen is an HCV E2 protein or a fragment or variant thereof.
39. The vaccine composition of embodiment 38, wherein the HCV E2 protein or fragment or variant thereof is derived from HCV genotype 1a or HCV genotype 2a.
40. The vaccine composition of embodiment 38 or 39, wherein the HCV viral antigen comprises the amino acid sequence of SEQ ID NO:23 or 24.
41. The vaccine composition of any one of embodiments 1-40, wherein the adaptor-antigen polypeptide comprises an amino acid sequence selected from the group consisting of SEQ ID NOs:1-11 and 26-29.
42. The vaccine composition of any one of embodiments 1-41, wherein the nano-complexes in the vaccine composition have an average particle size of about 100nm to about 130nm.
43. The vaccine composition of any one of embodiments 1-42, wherein the nano-complexes in the vaccine composition have a zeta-potential of about -10mV to about -20mV.
44. A method of preventing a viral infection in an individual, comprising administering to the individual an effective amount of the vaccine composition of any one of embodiments 1-43.
45. The method of embodiment 44, wherein the vaccine composition is administered by intranasal or intradermal administration.
46. The method of embodiment 44 or 45, wherein the vaccine composition is administered with an initial dose, followed by one or two booster doses.
47. The method of embodiment 46, wherein the initial dose and the one or two booster doses are administered from about 7 days to about 21 days apart.
48. The method of embodiment 47, wherein the initial dose and the one or two booster doses are administered about 14 days apart.
49. The method of any one of embodiments 46-48, wherein the initial dose and a first booster dose, and the first booster dose and a second booster dose are administered about 7 to about 21 days apart, optionally about 14 days apart.
50. The method of any one of embodiments 44-49, wherein the vaccine composition provides heterosubtypic protection against two or more virus subtypes that are part of the same virus species.
51. The method of any one of embodiments 44-50, wherein the method prevents viral infection by two or more viruses from different virus genera.
52. The method of embodiment 51, wherein:
   (i) the two or more virus species are from the same virus family; or
   (ii) the two or more virus species are from different virus families.
53. The method of embodiment 51 or 52, wherein the method prevents viral infection by three or more virus species, wherein:
   (i) the three or more virus species are from the same virus family;
   (ii) the three or more virus species are from different virus families; or
   (iii) the three or more virus species comprise: (a) two or more virus subtypes or subspecies that are from the same virus species, and (b) one or more virus species that is from a different virus family than the viruses of (a).

## Claims

1. A vaccine composition comprising nano-complexes, wherein the nano-complexes comprise: (a) a scaffold molecule comprising a viral RNA, and (b) an adaptor-antigen polypeptide comprising a viral antigen fused to a viral nucleoprotein capable of binding to the viral RNA.

2. The vaccine composition of claim 1, wherein the molar ratio of the adaptor-antigen polypeptide to the scaffold molecule is more than about 5:1.

3. The vaccine composition of claim 1 or 2, wherein the viral antigen is an antigen polypeptide or an immunogenic protein; optionally wherein the viral antigen is fused to the C-terminus or to the N-terminus of the viral nucleoprotein.

4. The vaccine composition of any one of claims 1-3, wherein the viral RNA comprises an untranslated region of a viral genome; optionally wherein the viral RNA comprises a 5' untranslated region or a 3' untranslated region of a viral gene of the viral genome; further optionally wherein the viral RNA is about 10 to about 500 nucleotides long.

5. The vaccine composition of any one of claims 1-4, wherein:
(a) the viral RNA is derived from SARS-CoV-2, optionally wherein the viral RNA comprises nucleotides 1-266 of a viral gene of the SARS-CoV-2 genome set forth in SEQ ID NO:25; or
(b) the viral RNA is derived from an influenza A virus (IAV), optionally wherein the viral RNA comprises nucleotides 1-326 of a viral gene of the IAV genome set forth in SEQ ID NO:30.

6. The vaccine composition of any one of claims 1-5, wherein:
(a) the viral nucleoprotein is derived from SARS-CoV-2, optionally wherein the viral nucleoprotein derived from SARS-CoV-2 comprises the amino acid sequence of SEQ ID NO: 12; or
(b) the viral nucleoprotein is derived from an IAV, optionally wherein the viral nucleoprotein is derived from an IAV subtype selected from the group consisting of H1N1, H5N1, H3N2, and H7N9, further optionally wherein the viral nucleoprotein is derived from IAV H5N1 and comprises the amino acid sequence of SEQ ID NO: 13.

7. The vaccine composition of any one of claims 1-6, wherein the viral nucleoprotein and the viral antigen are derived from the same virus or wherein the viral nucleoprotein and the viral antigen are derived from different viruses.

8. The vaccine composition of any one of claims 1-7, wherein:
(a) the viral antigen is a SARS-CoV-2 protein or a fragment or variant thereof, optionally wherein the viral antigen is a SARS-CoV-2 spike protein or a fragment or variant thereof, further optionally wherein the viral antigen comprises a receptor binding domain (RBD), further optionally wherein the SARS-CoV-2 viral antigen comprises the amino acid sequence of SEQ ID NO: 14 or 15;
(b) the viral antigen is an influenza virus protein or a fragment or variant thereof, optionally wherein the viral antigen is an influenza HA protein or a fragment or variant thereof, further optionally wherein the influenza virus protein or fragment or variant thereof is derived from IAV or influenza B virus (IBV), further optionally wherein:
(i) the influenza virus protein or fragment or variant thereof is derived from an IAV subtype selected from the group consisting of H1N1, H5N1, H3N2, and H7N9, optionally wherein the IAV viral antigen comprises an amino acid sequence selected from the group consisting of SEQ ID NOs: 16-18 and 31, or
(ii) the influenza virus protein or a fragment or variant thereof is derived from an IBV subtype of Yamagata or Victoria, optionally wherein the IBV viral antigen comprises the amino acid sequence of SEQ ID NO: 19 or 20;
(c) the viral antigen is a respiratory syncytial virus ("RSV") protein or a fragment or variant thereof, optionally wherein the viral antigen is an RSV F protein or a fragment or variant thereof, further optionally wherein the RSV F protein or fragment or variant thereof is derived from RSV A subtype or RSV B subtype, further optionally wherein the RSV viral antigen comprises the amino acid sequence of SEQ ID NO:21 or 22; or
(d) the viral antigen is a hepatitis C virus ("HCV") protein or a fragment or variant thereof, optionally wherein the viral antigen is an HCV E2 protein or a fragment or variant thereof, further optionally wherein the HCV E2 protein or fragment or variant thereof is derived from HCV genotype 1a or HCV genotype 2a, further optionally wherein the HCV viral antigen comprises the amino acid sequence of SEQ ID NO:23 or 24.

9. The vaccine composition of any one of claims 1-8, wherein the adaptor-antigen polypeptide comprises an amino acid sequence selected from the group consisting of SEQ ID NOs:1-11 and 26-29.

10. The vaccine composition of any one of claims 1-9, wherein the nano-complexes in the vaccine composition (a) have an average particle size of about 100nm to about 130nm and/or (b) have a zeta-potential of about -10mV to about -20mV.

11. The vaccine composition of any one of claims 1-10 for use in a method of preventing a viral infection in an individual, comprising administering to the individual an effective amount of the vaccine composition; optionally wherein the vaccine composition is administered by intranasal or intradermal administration; further optionally wherein the vaccine composition is administered with an initial dose, followed by one or two booster doses; further optionally wherein the initial dose and the one or two booster doses are administered (a) from about 7 days to about 21 days apart, or (b) about 14 days apart.

12. The vaccine composition for use according to claim 11, wherein:
(A) the vaccine composition provides heterosubtypic protection against two or more virus subtypes that are part of the same virus species;
(B) the vaccine composition prevents viral infection by two or more viruses from different virus genera, optionally wherein:
(i) the two or more virus species are from the same virus family, or
(ii) the two or more virus species are from different virus families; and/or
(C) the vaccine composition prevents viral infection by three or more virus species, wherein:
(i) the three or more virus species are from the same virus family;
(ii) the three or more virus species are from different virus families; or
(iii) the three or more virus species comprise:
(a) two or more virus subtypes or subspecies that are from the same virus species, and
(b) one or more virus species that is from a different virus family than the viruses of (a).

13. An isolated nucleic acid or a vector encoding the adaptor-antigen polypeptide and/or the scaffold molecule of the vaccine composition of any one of claims 1-10.

14. A host cell comprising the nucleic acid or the vector of claim 13.

15. A method of making a vaccine composition, comprising:
(i) culturing a host cell comprising the isolated nucleic acid or vector of claim 13, or the host cell of claim 14, under a condition suitable for the expression of the adaptor-antigen polypeptide and/or the transcription of the scaffold molecule;
(ii) obtaining the expressed adaptor-antigen polypeptide and/or the transcribed scaffold molecule; and
(iii) combining and incubating the adaptor-antigen polypeptide and the scaffold molecule in a buffer to form the vaccine composition.
